# EUROPEAN PATENT APPLICATION

(11) **EP 3 735 992 A1**
(43) Date of publication of application: **11.11.2020**
(21) Application number: 20155134.8
(22) Date of filing: 29.07.2016
(51) Int. Cl.: A61L 2/10, A61L 9/20, B01J 19/08, F21S 2/00, G01N 21/33

(54) **UV DEVICES, SYSTEMS, AND METHODS OF MAKING AND USE**

(30) Priority: 29.07.2015 US 201514813057
(62) Divisional of application: 16831460.7
(71) Applicant: BlueMorph, LLC, Oakland CA 94605 (US)
(72) Inventor: Farren, Alexander, Oakland,, CA California 94605 (US); Bareket, Noah, Saratoga,, CA California 95070 (US); Beard, Thomas Edgar, Healdsburg,, CA California 95448 (US)
(74) Representative: Schmidt, Christian

(57) **Abstract**

Provided herein are portable ultraviolet (UV) devices, systems, and methods of use and manufacturing same. Methods of use include methods for UV disinfection and sterilization, more specifically, methods for UV disinfection and sterilization of a container, a room, a space or a defined environment. The portable UV devices, systems and methods are particularly useful for the UV disinfection and sterilization of a container, a room, a space or defined environment used in the food, beverage and dairy industry and in the process of fermentation for an alcoholic beverage. Provided are also portable UV devices, systems, and methods for inhibiting the growth of one or more species of microorganisms present in a container, a room, a space or a defined environment, preferably for inhibiting the growth of one or more species of microorganisms present on an interior surface of a container, a room, a space or a defined environment.

## Description

### CROSS-REFERENCE TO RELATED APPLICATIONS

This application is a divisional application of European patent application 16831460.7, the disclosure of which as originally filed is incorporated herein by reference in its entirety, including the description, the claims and the drawings, for all purposes.

### FIELD OF INVENTION

The present invention relates generally to compositions, systems and methods for ultraviolet (UV) disinfection, and more specifically, to compositions, systems and methods for UV disinfection of a container, a room, a space or a defined environment. More specifically, the present invention relates to portable UV devices and uses thereof in methods of sterilization and sanitization of an interior surface present in a container, room, space or defined environment. The present invention also relates to methods of manufacturing portable UV devices.

### BACKGROUND OF THE INVENTION

It has been well established that ultraviolet (UV) light has germicidal properties. Specifically, the mechanism by which UV light kills microorganisms is by damaging the genetic material, the deoxyribonucleic acid (DNA), of the microorganisms. Wavelengths between 200-300 nm have been shown to initiate a photoreaction between adjacent pyrimidines. Pyrimidine bases, such as cytosine and thymine, have conjugated double bonds and as such absorb UV light. The photoreaction between adjacent thymine or cytosine bases proceeds at an exceedingly rapid rate (on the order of picoseconds). There are two possible products. The most common is the formation of a cyclobutane ring between the two pyrimidines (Fu et al., 1997, Applied and Environ Microbiol 63(4):1551-1556). The other photoproduct is a (6-4) pyrimidone. The formation of these dimers leads to "kinks" within the structure of the DNA inhibiting the formation of proper transcriptional and replicational templates. Cytosine cyclobutane photodimers are susceptible to deamination and can therefore induce point mutations, specifically the CC (two adjacent cytosines) are converted into TT (two adjacent thymines) via the SOS Response system in both eukaryotic and prokaryotic organisms (Fu et al., 2008, FEMS Microbiol Rev 32(6):908-26; Eller and Gilchrest, 2000, Pigment Cell Res 13 Suppl 8:94-7). The inactivation of specific genes via point mutations is one of the mechanisms of how UV-induced genetic damage can lead to cell death or to the inhibition of cell replication. The inability to form proper replicational and transcriptional templates coupled with the increased number of point mutations leads to the deactivation and inability to reproduce of microorganisms.

DNA, specifically has a maximum absorbency of UV light at 253.7 nm. It has been determined that approximately 26,400 microwatt-seconds/cm² are needed to deactivate 100% of the most resistant bacteria (Osburne et al., 2010, Environ Microbiol; doi: 10.11 11/j.1462-2920.2010.02203.x).

UV light is separated into 3 distinct categories: UV-A (315-400 nm), UV-B (280-315 nm), and UV-C (200-280 nm). Since DNA optimally absorbs UV light at 253.7 nm, it is UV-C lamps that are used in most prior art germicidal devices. UV devices are used, *e.g.,* to inactivate microorganisms in laboratory settings. UV radiation can also be used for disinfection in hospitals, nurseries, operating rooms, cafeterias and to sterilize vaccines, serums, toxins, municipal waste, and drinking waters.

Current steel vessel and container sanitation protocols involve the use of a pressure wash using a hot water cycle to remove pigments, colloidal deposits, and tartrates following wine fermentations. After the hot water cycle, typically the vessels are washed with a 200 mg/L solution of hypochlorite as a sanitation cycle. This is usually followed by a rinse with citric acid. (Boulton et al., Principles and Practices of Winemaking, page 210, Springer, 1st Edition, January 15, 1996).

Sodium hypochlorite (NaOCI) is often used for disinfecting hospital wastewater in order to prevent the spread of pathogenic microorganisms, causal agents of nosocomial infectious diseases. Chlorine disinfectants in wastewater react with organic matters, giving rise to organic chlorine compounds such as AOX (halogenated organic compounds adsorbable on activated carbon), which are toxic for aquatic organisms and are persistent environmental contaminants (Bohrerova et al., 2008, Water Research 42(12):2975-2982). Other protocols follow the removal of pigments, colloidal deposits, and tartrates with a wash with a caustic solution containing sodium hydroxide (typically 3%) and further followed by a final wash with a citric acid solution (typically 3%) to neutralize any remaining sodium hydroxide. There are several disadvantages to using sodium hydroxide and citric acid for sterilization. The primary disadvantage is the necessary use of large amounts of water as a solvent for both solutions. Any potential water saving measure is of great value both economically and environmentally. Further, the reduction in use of extremely caustic sodium hydroxide would be an added environmental benefit.

Other methods currently used for sterilizing fermentation vessels (made from metals and/or wood) include the use of ozone. Prior to 1997, ozone could only be used for sanitation and purification of bottled drinking water in the United States, and it is widely used around the world for this purpose today. In May 1997, an expert panel assembled by the Electric Power Research Institute (EPRI) declared ozone to be Generally Recognized as Safe (GRAS) for use in food processing in the United States. Since then, wineries have embraced the use of ozone. Its use has been generally accepted and documented to be effective for barrel cleaning and sanitation, tank cleaning and sanitation, clean-in-place systems, and for general surface sanitation. Results have shown the same degree of sanitization as that achieved using caustic for a fraction of the cost and wasted water.

However, in the wine industry, ozone systems tend to be mobile (a single unit can be moved to different vessels), with multiple operators in multiple locations. This makes it important that safety features and ozone management systems be in place and that the system itself be reliable and easy to operate.

Natural levels of ozone range from 0.01 ppm to 0.15 ppm and can reach higher concentrations in urban areas. Ozone is an unstable gas and readily reacts with organic substances. It sanitizes by interacting with microbial membranes and denaturating metabolic enzymes.

Ozone is generated by irradiation of an air stream with ultraviolet (UV) light at a wavelength of 185 nm or by passing dry air or oxygen through a corona discharge (CD technology) generator. For low ozone concentrations (ca. 0.14% by weight, or 0.5 grams per hour), the less expensive UV equipment is sufficient. For more demanding situations where higher ozone concentrations (1.0% to 14% by weight) are required, CD systems are used.

The wine industry is using both CD technology and UV (different from the one described herein. Several manufacturers chose to install air-cooled or water-cooled CD generators in their systems. It is really a question of how much ozone at a certain gallons per minute (gpm) is desired for an application. For clean in place (CIP), 20 gpm may be desired, necessitating a larger system, while only 10 gpm at a lower concentration may provide satisfactory barrel washing.

The Occupational Safety and Health Administration (OSHA) has set limits for ozone exposure in the workplace. These limits are for continuous eight-hour exposure of no more than 0.1 ppm, and a short-term exposure limit (STEL) of 15 minutes at 0.3 ppm, not to be exceeded more than twice per eight-hour work day. Consequently, ozone requires monitoring in the workplace if used for environmental or equipment sanitation using, e.g., ozone.

Ozone is known to have adverse physiological effects on humans (Directorate-General of Labour, the Netherlands 1992, 4(92), 62). Technically, there is no minimum threshold for ozone toxicity. Even low concentrations of ozone produce transient irritation of the lungs as well as headaches. Higher concentrations induce severe eye and upper respiratory tract irritation. Chronic exposure to ozone leads to respiratory tract disease and has been associated with reported increases in tumor growth rates. Exposure to ozone levels greater than the maximum thresholds specified by the American Conference of Governmental Industrial Hygienists (ACGIH)/Occupational Safety and Health Administration (OSHA) results in nausea, chest pain, coughing, fatigue and reduced visual acuity. Thus, while ozone provides an efficient means of sterilization, it also poses an occupational hazard to those involved in the sterilization process.

Another bactericidal chemical frequently used to sterilize fermentation vessels is chlorinated trisodium phosphate (TSP). It has been well established that chlorinated TSP is an effective germicidal agent. TSP, however, is also a severe irritant, capable of inducing contact dermatitis in addition to irritating the respiratory tract (Health Hazard Evaluation Report No. HETA-82-281-1503; HETA-82-281-1503). Also, certain microorganisms, such as *Cryptosporidium*, have developed resistance to reactive chlorine compounds. Further, evidence is mounting that organic chemical byproducts of chemical disinfection, especially byproducts of chlorination, are carcinogens and/or toxins for humans. Thus, expensive filtration devices may be required to remove the chemicals. Further, systems based on filtration require frequent replacement and/or cleaning of the filters. In addition, use of chlorinated TSP requires large quantities of water as a solvent and to extensively rinse the container following chemical sterilization. Also, chlorinated compounds are notorious for causing wine fouling. Thus, chemical disinfection is not a viable alternative when chemical purity of a fluid or alcoholic beverage in a fermentation vessel is desired or required.

Ozone sterilization was originally used to purify blood in the late 1800s. In the 1900s, ozonated water was in use for the treatment of multiple types of disease. In the first World War, ozone was used to treat wounds, gangrene and the effects of poisonous gas. Thus, throughout the time period, toxic and/or carcinogenic chemicals have been used in the sterilization of containers used for fermenting alcoholic beverages.

Using the chemical disinfection or ozone disinfection methods, there is also no established protocol for verifying the level of sterilization achieved by using those methods.

Sanitization of food-containing equipment or food-containing containers is a growing concern in the world. An increasing number of people fall ill each year by being exposed to contaminated food or food kept in contaminated containers.

Thus, there is a need in the art for non-toxic and non-carcinogenic methods, systems, and compositions, such as improved UV devices, useful for the sterilization of an interior surface present in container, room, space or defined environment. The compositions, systems, and methods provided herein meet these and other needs in the art.

### BRIEF SUMMARY OF THE INVENTION

Provided herein are UV devices, systems comprising a UV device, methods useful for the ultraviolet (UV) sterilization of containers and for the sanitization of rooms, spaces and defined environments using a UV device, and methods for manufacturing a UV device.

The present invention provides a UV device. In some embodiments of a UV device of the present invention, the UV device is a UV device for UV sterilization of an interior surface of a container, a room, a space or a defined environment. In some embodiments, the UV device comprises a first germicidal UV light source, a frame to which the first germicidal UV light source is operatively attached, a control box for controlling a sterilization cycle, a cable operatively connecting the control box and the first germicidal UV light source; and a means for operatively attaching the UV device to the container, room, space or defined environment.

In some embodiments, the UV device is configured so that the first germicidal UV light source can be movably and inwardly inserted through an opening of the container, room, space or defined environment into the container, room, space or defined environment when the UV device is operatively attached to the container, room, space or defined environment.

In some embodiments, the UV device uses an algorithm for determining the sterilization cycle.

In some embodiments, the control box comprises a circuit board and at least three components selected from the group consisting of (1) a power supply, (2) electronics for activating/deactivating the first germicidal UV light source, (3) a timer for controlling the duration of the sterilization cycle, (4) a memory for storing a pre-determined sterilization cycle, (5) a safety switch, (6) an on/off/reset button, (7) a status indicator light, (8) an alarm light, and (9) a user interface selected from the group consisting of a touchscreen and a keyboard. In some embodiments, the control box comprises a circuit board and at least four components selected from the group consisting of (1) through (9). In some embodiments, the control box comprises a circuit board and at least five components selected from the group consisting of (1) through (9). In some embodiments, the control box comprises a circuit board and at least six components selected from the group consisting of (1) through (9).

The circuit board comprises at least three functionalities selected from the group consisting of (A) comprising a radiofrequency identifier reader, (B) communicating with a radiofrequency identifier, (C) controlling a movement of the first germicidal UV light source within the container, room, space, or defined environment, (D) controlling a rate of descent or ascent of the first germicidal UV light source within the container, room, space, or defined environment, (E) controlling a positioning of first germicidal UV light source within the container, room, space, or defined environment, (F) controlling an on/off status of a motor, wherein the motor controls the positioning of the first germicidal UV light source within the container, room, space, or defined environment, (G) controlling an on/off status of the first germicidal UV light source based on measuring whether a pre-determined UV intensity has been attained, (H) controlling extension of the first germicidal UV light source from a housing, (I) controlling retraction of the first germicidal UV light source into the housing, (J) responding to a jammed position status of the first germicidal UV light source, (K) controlling an optical sensor which initiates a timer upon placing the first germicidal UV source into the container, room space, or defined environment, (L) controlling a speaker emitting an audible signal at the beginning or completion of the sterilization cycle, (M) controlling a plurality of LED lights indicting a status of the sterilization cycle, (N) relaying UV light intensity via a UV sensor to the container, room, space, or defined environment, (O) uploading and relaying information from the radiofrequency identifier, (P) generating a report on time of the sterilization cycle, (Q) generating a report on duration of the sterilization cycle, (R) generating a report on UV light intensity attained during the sterilization cycle, (S) emailing, phoning or texting the report on time of the sterilization cycle, (T) emailing, phoning or texting the report on duration of the sterilization cycle, (U) emailing, phoning or texting the report on UV light intensity attained during the sterilization cycle, (V) emailing, phoning or texting an alert to an individual that the sterilization cycle is initiated, interrupted or complete, (W) emailing, phoning or texting an alert that a UV light source requires replacement, (X) logging date, time or individual who used the UV device, and (Y) logging information of the container, room, space, or defined environment in which the UV device will be or has been used.

In some embodiments, the circuit board comprises at least four functionalities selected from (A) through (Y). In some embodiments, the circuit board comprises at least five functionalities selected from (A) through (Y). In some embodiments, the circuit board comprises at least six functionalities selected from (A) through (Y). In some embodiments, the circuit board comprises at least seven functionalities selected from (A) through (Y). In some embodiments, the circuit board comprises at least eight functionalities selected from (A) through (Y). In some embodiments, the circuit board comprises at least nine functionalities selected from (A) through (Y). In some embodiments, the circuit board comprises at least ten functionalities selected from (A) through (Y).

In some embodiments, the UV device is a portable UV device.

A UV device of the present invention is configured to include additional parts and components. In some embodiments, the first germicidal UV light source resides in a housing. A variety of housings can be used in the UV devices described herein. In some embodiments of a UV device of the present invention, the housing permits UV light to pass through. A housing that permits UV light to pass through, can be made of a variety of materials. In some embodiments of a UV device of the present invention, a housing is made of UV fused silica, CaF₂, MgF₂, BaF₂, quartz, sapphire, teflon, polydimethylsiloxane, TPX® or polymethylpentene (PMP). A preferred material is teflon.

A UV device of the present invention is configured to include additional parts and components. In some embodiments, a UV device further comprises a rope, a cable or a rigid rod for moving the first UV light source to a position within the container, room, space or defined environment.

There is no limitation for the design of the frame to which the first germicidal UV light source is operatively attached. In some embodiments of a UV device of the present invention, the frame comprises a plurality of openings.

A UV device of the present invention is configured to include additional parts and components. In some embodiments, a UV device further comprises a second germicidal UV light source.

In some embodiments wherein the UV device comprises a second germicidal UV light source, the frame comprises (i) a lower frame comprising a first lower frame end and a second lower frame end; (ii) an upper frame comprising a first upper frame end and a second upper frame end, and (iii) a first hinge movably and operatively connecting the lower frame to the upper frame and is configured so that the upper frame can be moved into an angular position with respect to the position of the lower frame. In such embodiment, the first germicidal UV light source is operatively attached to the upper frame and the second germicidal UV light source is operatively attached to the lower frame. When not in use, the upper frame may be positioned on top of the lower frame.

In some embodiments of a UV device of the present invention, the UV device further comprises a means for operatively attaching the UV device to an opening of a container, to a fixture in a container, room, space or defined environment, wherein when the UV device is attached, the means for attaching permits moving the first germicidal UV light source to a position within the container, room, space or defined environment. A variety of such means can be used for that purpose. In some embodiments, this means is a mounting bracket.

In some embodiments of a UV device of the present invention, the UV device further comprises a second hinge movably and operatively connecting the lower frame to the means for attaching the UV device to the opening or fixture of the container, room space or defined environment.

In some embodiments of a UV device of the present invention, the UV device further comprises a means for controlling or facilitating movement of the upper frame to an angular position with respect to the position of the lower frame. In some embodiments, this means permits the first germicidal UV light source be positioned at an angle ranging from about 0 degree to about 90 degrees with respect to the position of the second germicidal UV light source.

A variety of means can be used for controlling or facilitating movement of the upper frame to an angular position with respect to the position of the lower frame. In some embodiments, this means is selected from the group consisting of a pneumatic cylinder, a motor, a winch, an upper frame fixture clip, an extension spring, a rope and a cable.

In some embodiments of a UV device, the means for controlling or facilitating movement of the upper frame to an angular position with respect to the position of the lower frame is a rope or cable. The rope or cable may be operatively connected to a first rope or cable anchoring point at the upper frame and operatively connected to a second rope or cable anchoring point located on either the lower frame or located on a mounting bracket movably attached to the lower frame. Upon release of the rope or cable from the second rope or cable anchoring point, the upper frame can be moved from a horizontal position to an angular position with respect to the position of the lower frame. In some embodiments, the second rope or cable anchoring point is a first rope post or a second rope post attached to the mounting bracket.

In some embodiments of a UV device, the means for controlling or facilitating movement of the upper frame to an angular position with respect to the position of the lower frame is an upper frame fixture clip. The upper frame fixture clip is configured to restrict movement of the upper frame. Upon release from the upper frame fixture clip, the upper frame can be moved from a horizontal position to an angular position with respect to the position of the lower frame.

In some embodiments of a UV device, the means for controlling or facilitating movement of the upper frame to an angular position with respect to the position of the lower frame is an extension spring.. The extension spring may comprise a first hook attached to a first anchoring post and a second hook attached to a second anchoring post. In some embodiments of a UV device of the present invention, the second anchoring post is adapted to function as a carrying handle.

In some embodiments of a UV device, the means for controlling or facilitating movement of the upper frame to an angular position with respect to the position of the lower frame is a motor.

In some embodiments of a UV device, the means for controlling or facilitating movement of the upper frame to an angular position with respect to the position of the lower frame is a winch.

In some embodiments of a UV device, the means for controlling or facilitating movement of the upper frame to an angular position with respect to the position of the lower frame is a pneumatic cylinder.

In some embodiments of a UV device of the present invention, the UV device further comprises at least one stop post. The at least one stop post is configured to prevent movement of a first germicidal UV light source beyond an approximately perpendicular position with respect to the position of a second germicidal UV light source.

In some embodiments of a UV device of the present invention, the first upper frame end and the second upper frame end each comprise at least one opening configured to receive a UV lamp socket and wherein a first germicidal UV light source is operatively attached to the UV lamp socket.

In some embodiments of a UV device of the present invention, the first lower frame end and the second lower frame end each comprise at least one opening configured to receive a UV lamp socket and wherein a second germicidal UV light source is attached to the UV lamp socket.

In some embodiments of a UV device of the present invention, the first upper frame end and the second upper frame end are connected by a plurality of rods. In some embodiments, the upper frame further comprises at least one cross connector and the plurality of rods penetrates the at least one cross connector.

In some embodiments of a UV device of the present invention, the UV device comprises a UV sensor. The UV sensor may be operatively attached to the frame. In some embodiments, the UV sensor is operatively attached to either the lower or upper frame.

In some embodiments of a UV device of the present invention, the UV device comprises more than one first germicidal UV light source. In some embodiments, the at least one first germicidal UV light source is a member of a plurality of first germicidal UV light sources selected from the group consisting of two first germicidal UV light sources, three first germicidal UV light sources, four first germicidal UV light sources, five first germicidal UV light sources, six first germicidal UV light sources, seven first germicidal UV light sources, eight first germicidal UV light sources, nine first germicidal UV light sources, and ten first germicidal UV light sources. Members of the plurality of first germicidal UV light sources can be the same or different germicidal UV light sources.

In some embodiments of a UV device of the present invention, the UV device comprises more than one second germicidal UV light source. In some embodiments, the at least one second germicidal UV light source is a member of a plurality of second germicidal UV light sources selected from the group consisting of two second germicidal UV light sources, three second germicidal UV light sources, four second germicidal UV light sources, five second germicidal UV light sources, six second germicidal UV light sources, seven second germicidal UV light sources, eight second germicidal UV light sources, nine second germicidal UV light sources, and ten second germicidal UV light sources. Members of the plurality of second germicidal UV light sources can be the same or different germicidal UV light sources.

In some embodiments of a UV device of the present invention, the UV device comprises two first germicidal UV light sources connected to the upper frame and two second germicidal UV light sources connected to the lower frame. The two first germicidal UV light sources can be the same or different germicidal UV light sources. The two second germicidal UV light sources can be the same or different germicidal UV light sources. The two first germicidal UV light sources and the two second germicidal UV light sources can be the same or different germicidal UV light sources.

In some embodiments, the first or second germicidal UV light sources of a UV device of the present invention may comprise a variety of UV lamps. In some embodiments those lamps are independently selected from the group consisting of a low pressure mercury lamp, a medium pressure mercury lamp, a high pressure mercury lamp, an ultra-high pressure mercury lamp, a low pressure short arc xenon lamp, a medium pressure short arc xenon lamp, a high pressure short arc xenon lamp, an ultra-high pressure short arc xenon lamp, a low pressure long arc xenon lamp, a medium pressure long arc xenon lamp, a high pressure long arc xenon lamp, an ultra-high pressure long arc xenon lamp, a low pressure metal halide lamp, a medium pressure metal halide lamp, a high pressure metal halide lamp, an ultra-high pressure metal halide lamp, a tungsten halogen lamp, a quartz halogen lamp, a quartz iodine lamp, a sodium lamp, and an incandescent lamp. Preferred is a low pressure mercury lamp.

In some embodiments of a UV device of the present invention, the first germicidal UV light source or the second germicidal UV light source is a UV-C light source.

In some embodiments of the present invention, a UV light source is connected to a control box. In some embodiments, when the first germicidal UV light source is inserted into a container, room, space or defined environment, the control box resides outside of the container, room, space or defined environment.

In some embodiments of a UV device of the present invention, a touchscreen interface or keyboard is configured so that a user can provide an input for a functionality selected from the group consisting of (A) activating the UV device, (B) deactivating the UV device, (C) providing time input for completing a UV sterilization of a container, room, space or a defined environment, (D) providing time elapsed for UV sterilization of the container, room, space or defined environment, (E) setting a desired UV intensity level, (F) adjusting a UV intensity level, and (G) logging in a code for a user.

In some embodiments of a UV device of the present invention, an algorithm determines a sterilization cycle based on at least two parameters selected from the group consisting of (i) species of microorganism present on the surface of the container, room, space or defined environment to be sterilized, (ii) size of the container, room, space or defined environment, (iii) shape of the container, room, space or defined environment,(iv) the material of the surface of the container, room, space or defined environment to be sterilized, (v) extinction depth of the first germicidal UV light source at 254 nm, (vi) distance of the first germicidal UV light source from the surface of the container, room, space or defined environment to be sterilized (vii) distribution and positioning of the first germicidal UV light source, (viii) configuration of the first germicidal UV light source, and (ix) intensity of the first germicidal UV light source.

In some embodiments of a UV device, when the first germicidal UV light source is inserted into the container, room, space or defined environment, the first germicidal UV light source can be moved from a first position to a second position within the container, room, space or defined environment.

The present invention also provides systems comprising a UV device. In some embodiments of a system, the system comprises (a) a UV device of the present invention and (b) a container, a room, a space or a defined environment. Any UV device described herein can be used in a system, in particular those described in paragraphs [0021] through [0056].

A system of the present invention may comprise a variety of containers. In some embodiments of a system of the present invention, a container is selected from the group consisting of: (A) a container for fermenting an alcoholic beverage, (B) a container for storing or transporting a dairy product, a liquid dairy, a liquid dairy composition or a dry dairy composition, (C) a container for water, milk, coffee, tea, juice, or a carbonated beverage, and (D) a container for a biological fluid.

A system of the present invention may comprise a variety of rooms, spaces or defined environments. In some embodiments, a room, space or defined environment is selected from the group consisting of a commercial kitchen, a medical facility, an acute care area, an operating room, a medical equipment storage cabinet, a clean room, a bathroom, a waiting room, a food production area, a food processing area, a nursery home, a car, a bus, a trailer, a rail car, an airplane, a ship, a boat, a grocery store display case, a deli counter, a fish display case, a poultry display case, a floral display case, a refrigerated display case, a non-refrigerated display case, and a conveyor belt.

The container, room, space or defined environments of a system of the present invention may have various interior surfaces. In some embodiments of a system of the present invention, a container, room, space or defined environment comprises an interior surface comprising wood, plastic, concrete, a polymer, etched aluminum, foil aluminum, polished aluminum, chromium, glass, nickel, silver, stainless steel, tri-plated steel, water paint, white cotton, white oil paint, white paper, white porcelain, white wall plaster or a fabric.

In some embodiments of a system of the present invention, a system comprises (a) a UV device of the present invention and (b) a case. When not in use, the UV device or parts thereof may reside in the case. In some embodiments, the case is attached to the control box.

In some embodiments of a system of the present invention, the system further comprises a transportation rack. The transportation rack is configured to accommodate the UV device and the case for transportation.

The present invention further provides methods of using a UV device of the present invention, preferably using a UV device of the present invention in a method for UV sterilization of an interior surface of a container, an interior surface of a room, an interior surface of a space, or an interior surface of a defined environment. Any UV device described herein can be used in such method, in particular those described in paragraphs [0021] through [0056]. In some embodiments, a method for UV sterilization of an interior surface of a container, room, space or defined environment comprises the steps of (a) movably and inwardly inserting a first UV light source of the UV device into a container, room, space or defined environment and (b) activating the first germicidal UV light source. Thereby, the interior surface of the container, room, space or defined environment is sterilized.

Any interior surface in a container, room, space or defined environment can be sterilized using a UV device and a method of the present invention. In some embodiments of a method for UV sterilization of an interior surface of a container, a container is selected from the group consisting of (A) a container for fermenting an alcoholic beverage, (B) a container for storing or transporting a dairy product, a liquid dairy, a liquid dairy composition or a dry dairy composition, (C) a container for water, milk, coffee, tea, juice, or a carbonated beverage, and (D) a container for a biological fluid.

A method of sterilization of the present invention may comprise a variety of rooms, spaces or defined environments. In some embodiments, a room, space or defined environment is selected from the group consisting of a commercial kitchen, a medical facility, an acute care area, an operating room, a medical equipment storage cabinet, a clean room, a bathroom, a waiting room, a food production area, a food processing area, a nursery home, a car, a bus, a trailer, a rail car, an airplane, a ship, a boat, a grocery store display case, a deli counter, a fish display case, a poultry display case, a floral display case, a refrigerated display case, a non-refrigerated display case, and a conveyor belt.

An interior surface of a container, room, space or defined environment may have various interior surfaces. Methods described herein are not limited by such surfaces. In some embodiments of a method for UV sterilization of an interior surface of a container, room, space or defined environment, the container, room, space or the defined environment comprises an interior surface comprising wood, plastic, concrete, a polymer, etched aluminum, foil aluminum, polished aluminum, chromium, glass, nickel, silver, stainless steel, tri-plated steel, water paint, white cotton, white oil paint, white paper, white porcelain, white wall plaster or a fabric.

The present invention further provides methods for manufacturing a UV device. In particular, the present invention provides a method for manufacturing a UV device, in particular a UV device described in paragraphs [0021] through [0056]. In some embodiments, a method for manufacturing a UV device comprises the steps of (a) attaching a first germicidal UV light source to a frame, (b) operatively connecting a cable to a control box and to the first germicidal UV light source; and (c) attaching a means for operatively attaching the UV device to a container, room, space or defined environment.

In some embodiments of a method for manufacturing a UV device the method comprises the steps of (a) attaching a first germicidal UV light source to an upper frame, (b) attaching a second germicidal UV light source to a lower frame, (c) attaching a first hinge to the lower frame and to the upper frame thereby operatively connecting the lower frame to the upper frame so that the upper frame can be moved into a position ranging from about 0 degree to about 90 degrees with respect to the position of the lower frame. In some embodiments, a method for manufacturing a UV device further comprises the step of attaching a means for controlling or facilitating movement of the upper frame into a position ranging from about 0 degree to about 90 degrees with respect to the position of the lower frame.

Some embodiments of a UV device of the present invention, a system of the present invention, a method of use of the present invention and a method of manufacturing a UV device of the present invention are set forth below:
1. A portable ultraviolet (UV) device for applying a sterilization cycle to a surface within a container, a room, a space, or a defined environment, the UV device comprising:
   (i) a first germicidal UV light source;
   (ii) a frame to which the first germicidal UV light source is operatively attached;
   (iii) a control box for controlling a sterilization cycle;
   (iv) a cable operatively connecting the control box and the first germicidal UV light source; and
   (v) a means for operatively attaching the UV device to the container, room, space or defined environment,
   wherein the UV device is configured to movably and inwardly insert the first germicidal UV light source through an opening of the container, room, space or defined environment into the container, room, space or defined environment when the UV device is operatively attached to the container, room, space or defined environment;
   wherein the UV device uses an algorithm for determining the sterilization cycle;
   wherein the control box comprises a circuit board and at least three components selected from the group consisting of:
   (1) a power supply,
   (2) electronics for activating/deactivating the first germicidal UV light source,
   (3) a timer for controlling the duration of the sterilization cycle,
   (4) a memory for storing a pre-determined sterilization cycle,
   (5) a safety switch,
   (6) an on/off/reset button;
   (7) a status indicator light;
   (8) an alarm light; and
   (9) a user interface selected from the group consisting of a touch screen and a keyboard; and
   wherein the circuit board comprises at least three functionalities selected from the group consisting of:
   (A) comprising a radiofrequency identifier reader;
   (B) communicating with a radiofrequency identifier;
   (C) controlling a movement of the first germicidal UV light source within the container, room, space, or defined environment;
   (D) controlling a rate of descent or ascent of the first germicidal UV light source within the container, room, space, or defined environment;
   (E) controlling a positioning of first germicidal UV light source within the container, room, space, or defined environment;
   (F) controlling an on/off status of a motor, wherein the motor controls the positioning of the first germicidal UV light source within the container, room, space, or defined environment;
   (G) controlling an on/off status of the first germicidal UV light source based on measuring whether a pre-determined UV intensity has been attained;
   (H) controlling extension of the first germicidal UV light source from a housing;
   (I) controlling retraction of the first germicidal UV light source into the housing;
   (J) responding to a jammed position status of the first germicidal UV light source;
   (K) controlling an optical sensor which initiates a timer upon placing the first germicidal UV source into the container, room space, or defined environment;
   (L) controlling a speaker emitting an audible signal at the beginning or completion of the sterilization cycle;
   (M) controlling a plurality of LED lights indicting a status of the sterilization cycle;
   (N) relaying UV light intensity via a UV sensor to the container, room, space, or defined environment;
   (O) uploading and relaying information from the radiofrequency identifier;
   (P) generating a report on time of the sterilization cycle;
   (Q) generating a report on duration of the sterilization cycle:
   (R) generating a report on UV light intensity attained during the sterilization cycle;
   (S) emailing, phoning or texting the report on time of the sterilization cycle;
   (T) emailing, phoning or texting the report on duration of the sterilization cycle;
   (U) emailing, phoning or texting the report on UV light intensity attained during the sterilization cycle;
   (V) emailing, phoning or texting an alert to an individual that the sterilization cycle is initiated, interrupted or complete;
   (W) emailing, phoning or texting an alert that a UV light source requires replacement;
   (X) logging date, time or individual who used the UV device; and
   (Y) logging information of the container, room, space, or defined environment in which the UV device will be or has been used.
2. The UV device according to embodiment 1, wherein the UV device is a portable UV device.
3. The UV device according to any one of embodiments 1 to 2, wherein the first germicidal UV light source resides in a housing.
4. The UV device according to embodiment 3, wherein the housing permits UV light to pass through.
5. The UV device according to embodiment 3, wherein the housing is made of UV fused silica, CaF₂, MgF₂, BaF₂, quartz, sapphire, teflon, polydimethylsiloxane, TPX® or polymethylpentene (PMP).
6. The UV device according to any one of embodiments 1 to 5, further comprising a rope, a cable or a rigid rod for moving the first UV light source to a position within the container, room, space or defined environment.
7. The UV device according to any one of embodiments 1 to 6, wherein the frame comprises a plurality of openings.
8. The UV device according to any one of embodiments 1 to 7, further comprising a second germicidal UV light source and wherein the frame comprises
   (a) a lower frame comprising a first lower frame end and a second lower frame end;
   (b) an upper frame comprising a first upper frame end and a second upper frame end;
   (c) a first hinge movably and operatively connecting the lower frame to the upper frame and configured to move the upper frame into an angular position with respect to the position of the lower frame;
   wherein the first germicidal UV light source is operatively attached to the upper frame; wherein the second germicidal UV light source is operatively attached to the lower frame; and
   wherein, when not in use, the upper frame is positioned on top of the lower frame.
9. The UV device according to any one of embodiments 1 to 8, wherein (v) is configured to operatively attach the portable UV device to an opening of the container, to a fixture in or at the container, room, space or defined environment and to permit the moving of the first germicidal UV light source to a position within the container, room, space or defined environment.
10. The UV device according to any one of embodiments 1 to 9, wherein (v) is a mounting bracket.
11. The UV device according to embodiment 8, further comprising a second hinge movably and operatively connecting the lower frame to (v).
12. The UV device according to any one of embodiments 8 and 11, further comprising a means for controlling or facilitating movement of the upper frame to an angular position with respect to the position of the lower frame.
13. The UV device according to any one of embodiments 8, 11 and 12, wherein the means for controlling or facilitating movement of the upper frame to an angular position with respect to the position of the lower frame permits the first germicidal UV light source be positioned at an angle ranging from about 0 degree to about 90 degrees with respect to the position of the second germicidal UV light source.
14. The UV device according to any one of embodiments 12 to 13, wherein the means for controlling or facilitating movement of the upper frame to an angular position with respect to the position of the lower frame is selected from the group consisting of a pneumatic cylinder, a motor, a winch, an upper frame fixture clip, an extension spring, a rope and a cable.
15. The UV device according to embodiment 14, wherein the means for controlling or facilitating movement of the upper frame to an angular position with respect to the position of the lower frame is a rope or a cable and wherein the rope or cable is operatively connected to a first rope or cable anchoring point at the upper frame and operatively connected to a second rope or cable anchoring point located on either the lower frame or located on a mounting bracket movably attached to the lower frame and wherein, upon release of the rope or cable from the second rope or cable anchoring point, the upper frame moves from a horizontal position to an angular position with respect to the position of the lower frame.
16. The UV device according to embodiment 15, wherein the second rope or cable anchoring point is a first rope post or a second rope post attached to the mounting bracket.
17. The UV device according to embodiment 14, wherein the means for controlling or facilitating movement of the upper frame to an angular position with respect to the position of the lower frame is an upper frame fixture clip, wherein the upper frame fixture clip is configured to restrict movement of the upper frame, and wherein, upon release from the upper frame fixture clip, the upper frame moves from a horizontal position to an angular position with respect to the position of the lower frame.
18. The UV device according to embodiment 14, wherein the means for controlling or facilitating movement of the upper frame to an angular position with respect to the position of the lower frame is an extension spring comprising a first hook attached to a first anchoring post and a second hook attached to a second anchoring post.
19. The UV device according to embodiment 14, wherein the means for controlling or facilitating movement of the upper frame to an angular position with respect to the position of the lower frame is a motor.
20. The UV device according to embodiment 14, wherein the means for controlling or facilitating movement of the upper frame to an angular position with respect to the position of the lower frame is a winch.
21. The UV device according to any one of embodiments 8 to 20, further comprising at least one stop post, wherein the at least one stop post is configured to prevent movement of the first germicidal UV light source beyond an approximately perpendicular position with respect to the position of the second germicidal UV light source.
22. The UV device according to embodiment 18, wherein the second anchoring post is configured to function as a carrying handle.
23. The UV device according to any one of embodiments 8 to 22, wherein the first upper frame end and the second upper frame end each comprise at least one opening configured to receive a UV lamp socket and wherein the first germicidal UV light source is operatively attached to the UV lamp socket.
24. The UV device according to any one of embodiments 8 to 23, wherein the first lower frame end and the second lower frame end each comprise at least one opening configured to receive a UV lamp socket and wherein the second germicidal UV light source is operatively attached to the UV lamp socket.
25. The UV device according to any one of embodiments 8 to 24, wherein the first upper frame end and the second upper frame end are operatively connected by a plurality of rods.
26. The UV device according to embodiment 25, wherein the upper frame further comprises at least one cross connector and wherein the plurality of rods penetrates the at least one cross connector.
27. The UV device according to any one of embodiments 1 to 26, further comprising a UV sensor.
28. The UV device according to any one of embodiments 1 to 27, wherein the first germicidal UV light source is a member of a plurality of first germicidal UV light sources selected from the group consisting of two first germicidal UV light sources, three first germicidal UV light sources, four first germicidal UV light sources, five first germicidal UV light sources, six first germicidal UV light sources, seven first germicidal UV light sources, eight first germicidal UV light sources, nine first germicidal UV light sources, and ten first germicidal UV light sources, and wherein members of the plurality of first germicidal UV light sources are the same or different germicidal UV light sources.
29. The UV device according to any one of embodiments 8 to 28, wherein the second germicidal UV light source is a member of a plurality of second germicidal UV light sources selected from the group consisting of two second germicidal UV light sources, three second germicidal UV light sources, four second germicidal UV light sources, five second germicidal UV light sources, six second germicidal UV light sources, seven second germicidal UV light sources, eight second germicidal UV light sources, nine second germicidal UV light sources, and ten second germicidal UV light sources, and wherein members of the plurality of second germicidal UV light sources can be the same or different germicidal UV light sources.
30. The UV device according to any one of embodiments 8 to 29, wherein the UV device comprises two first germicidal UV light sources operatively connected to the upper frame and two second germicidal UV light sources operatively connected to the lower frame and wherein the two first germicidal UV light sources and the two second germicidal UV light sources are the same or different germicidal UV light sources.
31. The UV device according to any one of embodiments 1 to 30, wherein the first germicidal UV light source comprises a lamp selected from the group consisting of a low pressure mercury lamp, a medium pressure mercury lamp, a high pressure mercury lamp, an ultra-high pressure mercury lamp, a low pressure short arc xenon lamp, a medium pressure short arc xenon lamp, a high pressure short arc xenon lamp, an ultra-high pressure short arc xenon lamp, a low pressure long arc xenon lamp, a medium pressure long arc xenon lamp, a high pressure long arc xenon lamp, an ultra-high pressure long arc xenon lamp, a low pressure metal halide lamp, a medium pressure metal halide lamp, a high pressure metal halide lamp, an ultra-high pressure metal halide lamp, a tungsten halogen lamp, a quartz halogen lamp, a quartz iodine lamp, a sodium lamp, and an incandescent lamp.
32. The UV device according to any one of embodiments 1 to 31, wherein when the first germicidal UV light source is inserted into the container, room, space or defined environment, the control box resides outside of the container, room, space or defined environment.
33. The UV device according to any one of embodiments 1 to 32, wherein the touchscreen is configured to provide an input for a functionality selected from the group consisting of:
   (A) activating the UV device;
   (B) deactivating the UV device;
   (C) providing time input for completing a UV sterilization of a container, room, space or defined environment;
   (D) providing time elapsed for UV sterilization of the container, room, space or defined environment;
   (E) setting a desired UV intensity level;
   (F) adjusting a UV intensity level; and
   (G) logging in a code for a user.
34. The UV device according to any one of embodiments 1 to 33, wherein the algorithm determines the sterilization cycle based on at least two parameters selected from the group consisting of (i) species of microorganism present on the surface of the container, room, space or defined environment to be sterilized, (ii) size of the container, room, space or defined environment, (iii) shape of the container, room, space or defined environment,(iv) the material of the surface of the container, room, space or defined environment to be sterilized, (v) extinction depth of the first germicidal UV light source at 254 nm, (vi) distance of the first germicidal UV light source from the surface of the container, room, space or defined environment to be sterilized (vii) distribution and positioning of the first germicidal UV light source, (viii) configuration of the first germicidal UV light source, and (ix) intensity of the first germicidal UV light source.
35. The UV device according to any one of embodiments 1 to 34, wherein when the first germicidal UV light source is inserted into the container, room, space or defined environment, the first germicidal UV light source can be moved from a first position to a second position within the container, room, space or defined environment.
36. The UV device according to any one of embodiments 1 to 35, wherein the control box comprises components selected from the group consisting of at least four components of (1) - (9), at least five components of (1) - (9), at least six components of (1) - (9), at least seven components of (1) - (9), at least eight components of (1) - (9), and nine components of (1) - (9).
37. The UV device according to any one of embodiments 1 to 36, wherein the circuit board comprises functionalities selected from the group consisting of at least four functionalities of (A) - (Y), at least five functionalities of (A) - (Y), at least six functionalities of (A) - (Y), at least seven functionalities of (A) - (Y), at least eight functionalities of (A) - (Y), at least nine functionalities of (A) - (Y), at least ten functionalities of (A) - (Y), at least eleven functionalities of (A) - (Y), at least twelve functionalities of (A) - (Y), at least thirteen functionalities of (A) - (Y), at least fourteen functionalities of (A) - (Y), at least fifteen functionalities of (A) - (Y), at least sixteen functionalities of (A) - (Y), at least seventeen functionalities of (A) - (Y), at least eighteen functionalities of (A) - (Y), at least nineteen functionalities of (A) - (Y), at least twenty functionalities of (A) - (Y), at least twenty-one functionalities of (A) - (Y), at least twenty-two functionalities of (A) - (Y), at least twenty-three functionalities of (A) - (Y), at least twenty-four functionalities of (A) - (Y), and twenty-five functionalities of (A) - (Y).
38. A system comprising:
   (i) the UV device according to any one of embodiments 1 to 37; and
   (ii) a container, a room, a space or a defined environment.
39. The system according to embodiment 38 wherein the container is selected from the group consisting of:
   (A) a container for fermenting an alcoholic beverage;
   (B) a container for storing or transporting a dairy product, a liquid dairy, a liquid dairy composition or a dry dairy composition;
   (C) a container for water, milk, coffee, tea, juice, or a carbonated beverage; and
   (D) a container for a biological fluid.
40. The system according to embodiment 38, wherein the room, space or defined environment is selected from the group consisting of a commercial kitchen, a medical facility, an acute care area, an operating room, a medical equipment storage cabinet, a clean room, a bathroom, a waiting room, a food production area, a food processing area, a nursery home, a car, a bus, a trailer, a rail car, an airplane, a ship, a boat, a grocery store display case, a deli counter, a fish display case, a poultry display case, a floral display case, a refrigerated display case, a non-refrigerated display case, and a conveyor belt.
41. The system according to any one of embodiments 38 to 40, wherein the container, room, space or defined environment comprises an interior surface comprising wood, plastic, concrete, a polymer, etched aluminum, foil aluminum, polished aluminum, chromium, glass, nickel, silver, stainless steel, tri-plated steel, water paint, white cotton, white oil paint, white paper, white porcelain, white wall plaster or a fabric.
42. A system comprising:
   (i) the UV device according to any one of embodiments 1 to 37; and
   (ii) a case, wherein, the UV device, when not in use, resides within the case.
43. The system according to embodiment 42, wherein the case is attached to the control box.
44. The system according to any one of embodiments 42 to 43, further comprising a transportation rack configured to accommodate the control box and the case for transportation.
45. A method for UV sterilization of an interior surface of a container, room, space or defined environment, the method comprising the steps of:
   (a) movably and inwardly inserting the first UV light source of the UV device of any one of embodiments 1 to 37 into a container, room, space or defined environment; and
   (b) activating the first germicidal UV light source; whereby the interior surface of the container, room, space or defined environment is sterilized.
46. The method according to embodiment 45, wherein the container is selected from the group consisting of:
   (A) a container for fermenting an alcoholic beverage;
   (B) a container for storing or transporting a dairy product, a liquid dairy, a liquid dairy composition or a dry dairy composition;
   (C) a container for water, milk, coffee, tea, juice, or a carbonated beverage; and
   (D) a container for a biological fluid.
47. The method according to embodiment 45, wherein the room, space or defined environment is selected from the group consisting of a commercial kitchen, a medical facility, an acute care area, an operating room, a medical equipment storage cabinet, a clean room, a bathroom, a waiting room, a food production area, a food processing area, a nursery home, a car, a bus, a trailer, a rail car, an airplane, a ship, a boat, a grocery store display case, a deli counter, a fish display case, a poultry display case, a floral display case, a refrigerated display case, a non-refrigerated display case, and a conveyor belt.
48. A method for manufacturing the UV device of any one of embodiments 1 to 37, the method comprising the steps of:
   (a) attaching the first germicidal UV light source to the frame;
   (b) operatively connecting the cable to the control box and to the first germicidal UV light source; and
   (c) attaching the means for operatively attaching the UV device to a container, room, space or defined environment.
49. A method for manufacturing the UV device of any one of embodiments 8 to 37, the method comprising the steps of:
   (a) attaching the first germicidal UV light source to the upper frame;
   (b) attaching the second germicidal UV light source to the lower frame; and
   (c) attaching the first hinge to the lower frame and to the upper frame thereby operatively connecting the lower frame to the upper frame so that the upper frame can be moved into a position ranging from about 0 degree to about 90 degrees with respect to the position of the lower frame.
50. The method according to embodiment 49, further comprising the step of:
   (d) attaching a means for controlling or facilitating movement of the upper frame into a position ranging from about 0 degree to about 90 degrees with respect to the position of the lower frame.

### BRIEF DESCRIPTION OF THE DRAWINGS

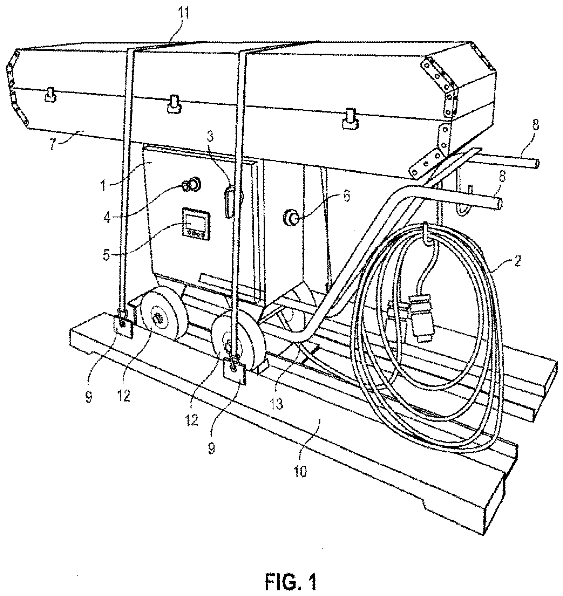

Figure 1 schematically depicts a front view of a system of the present invention comprising a case **7**, a control box **1** (as part of a UV device of the present invention) and a transportation rack **10.** Also shown are a touchscreen interface **5**, an on/off/reset switch **3**, a status indicator light/alarm light 6, an emergency shutdown button **4**, wheels **12**, and handrails **8** attached to the control box **1.** Further shown are a power cable **2** and a cable **13** which operatively connects the control box **1** with a UV device (residing in case **7**, however, not visible). Fastening brackets **9** are attached to the transportation rack **10.** Fastenings **11** hold the case **7** and control box **1** in place during transportation or when system is not in use. Details of individual parts and components are described herein.

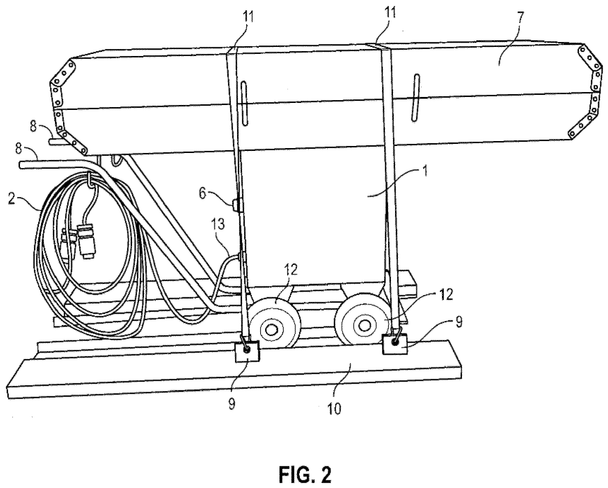

Figure 2 schematically depicts a rear view of a system of the present invention comprising a case **7** (a UV device residing therein, however, not visible), a control box **1**, and a transportation rack **10.** Parts and components are as in FIG. 1.

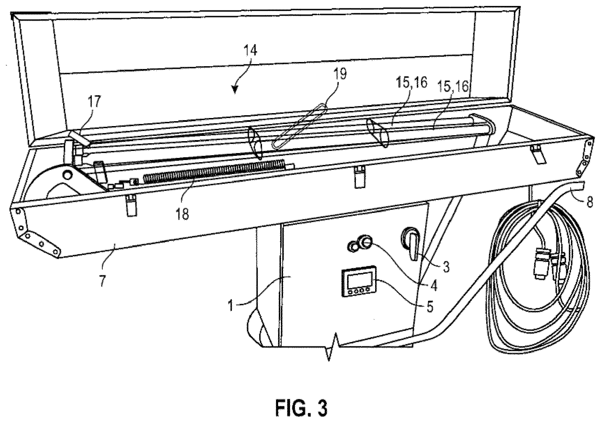

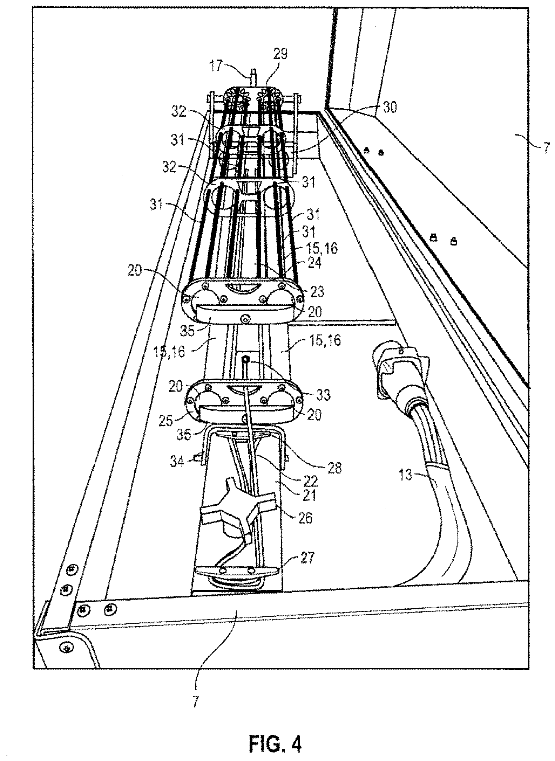

Figure 3 schematically depicts a front view of a system of the present invention comprising a UV device (Model UVT-4, **14**), case **7** (in an open configuration), and a control box **1.** Case **7** is open to show UV device Model UVT-4, **14**. Also schematically shown are extension spring **18**, a second anchoring post **19** configured to have a carrying handle at its end, a UV sensor **17**, UV light sources **16** and housings **15** surrounding the UV light sources **16**. In this exemplary embodiment of a UV device of the UVT-4 family of UV devices, the housing **15** is a see-through housing permitting the UV light to pass through; thus, housing **15** and UV light source **16** are indicated in this and the following drawings (FIGS 4-13 and 15-19) by **15,16.** Other parts and components are as in FIGS. 1 and 2. Further details of UV device Model UVT-4 are shown in the following drawings and are described herein.
Figure 4 schematically depicts a top rear view of a member of the UVT-4 family of UV devices. The following parts and components are indicated: housing **15** and UV light source/UV lamp **16** (**15,16**), mounting bracket **21**, rope or cable **22**, UV lamp sockets/adapters **20**, cable **13** connecting the UV light source with control box **1** (not visible), case **7**, lower frame **23**, first upper frame end **24**, first lower frame end **25**, bracket tightening knob **26**, first rope post **27**, second rope post **28**, second upper frame end **29**, second lower frame end **30**, UV sensor **17**, protective rods **31**, cross connector **32** on upper frame, upper frame fixture clip **33**, second hinge **34** (for movably and operatively connecting the bracket with the lower frame), T-shaped cap **35** (for maintaining bulb clamps in place). Details of individual parts and components are described herein.

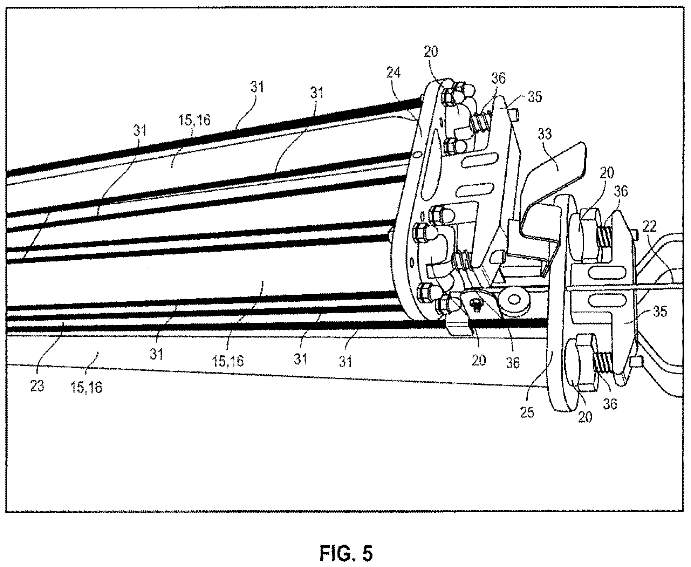

Figure 5 schematically depicts a side view of the top rear of a member of the UVT-4 family of UV devices. Parts and components are as in FIGS. 1-4. In addition, bulb clamps **36**, held in place by T-shaped cap **35,** are shown. Details of individual parts and components are described herein.

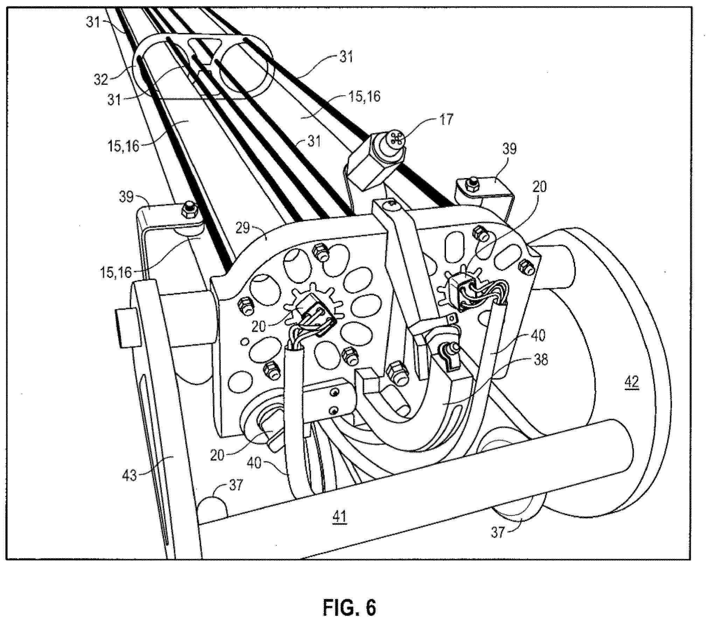

Figure 6 schematically depicts a view of the top front of a member of the UVT-4 family of UV devices. Parts and components are as in FIGS. 1-5. In addition, wheels **37** (for easy moving of UV device on a surface), first hinge **38** (movably and operatively connecting lower and upper frames), stop posts **39** (preventing extender spring from extending vertically positioned UV light source moving beyond perpendicular/vertical position), cables **40** activating UV light sources/UV lamps **16**, side plate spacer **41** (for stabilization of cross bar), first side plate **42**, and second side plate **43** are shown. Details of individual parts and components are described herein.

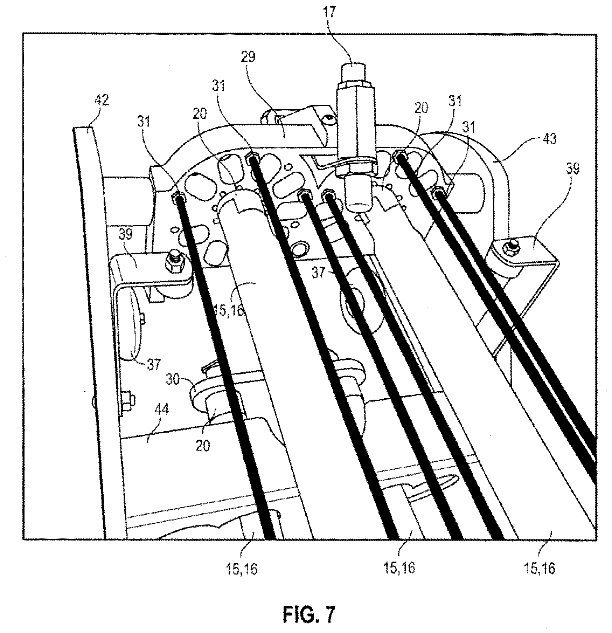

Figure 7 schematically depicts a top view of the front end of a member of the UVT-4 family of UV devices. Parts and components are as in FIGS. 1-6. In addition, a cross connector **44**, connected to the lower frame **23**, is shown. Details of individual parts and components are described herein.

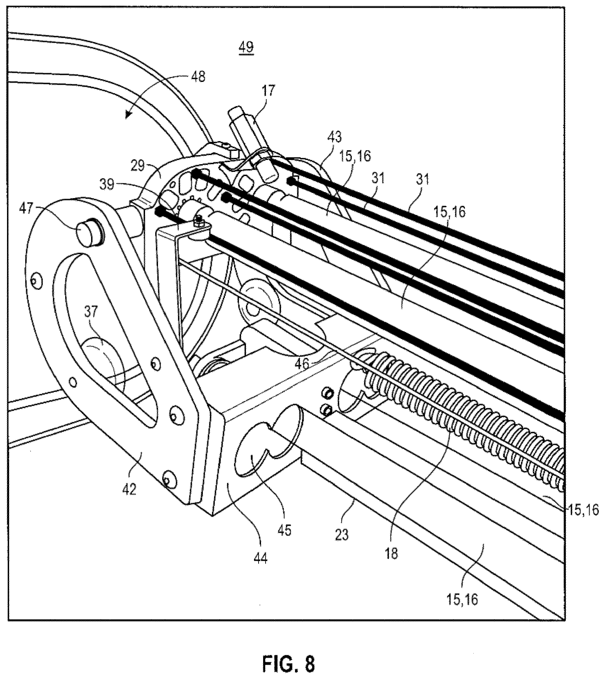

Figure 8 schematically depicts movably and inwardly inserting a member of the UVT-4 family of UV devices through an opening **48** of a container **49** into a container **49.** Parts and components are as in FIGS. 1-7. In addition, openings **45** within a cross connector **44** of the lower frame **23** are shown. Openings **45** through which not already a housing/UV light source **15**, **16** is guided through may accommodate an additional housing/UV light source. Also shown are fasteners **47** movably and operatively connecting the upper frame to the lower frame and configured to permit "swinging" of the upper frame and UV light source(s) attached thereto into an angular position with respect to the position of the lower frame **23** and the UV light source(s) attached thereto. Also shown is a first anchoring post **46** for the extension spring **18.** Details of individual parts and components are described herein.

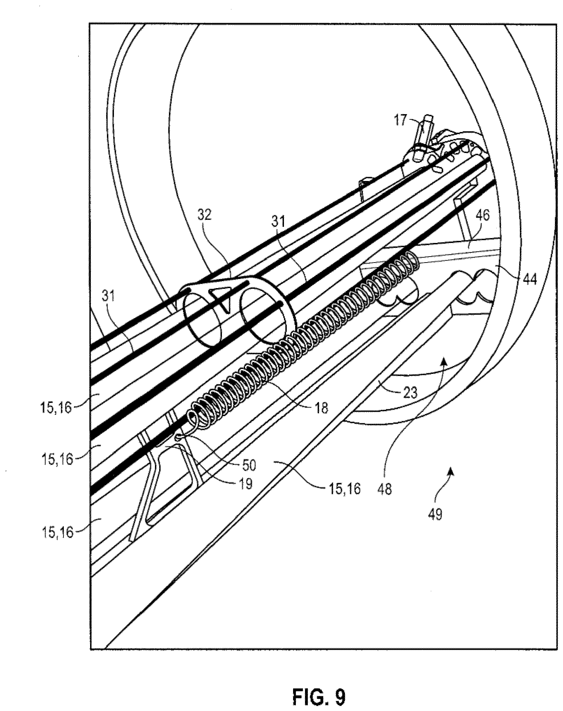

Figure 9 schematically depicts movably and inwardly inserting a member of the UVT-4 family of UV devices through an opening **48** of a container **49** into a container **49.** Parts and components are as in FIGS. 1-8. The UV device has been further inserted through the opening **48** as compared to FIG. 8. In addition, a second hook **50** of the extension spring **18** and the position of a first anchoring post **46** for extension spring **18** where the first end **59** of the extension spring **18** is connected to cable **58** (no shown, however, further described herein) are shown. Details of individual parts and components are described herein.

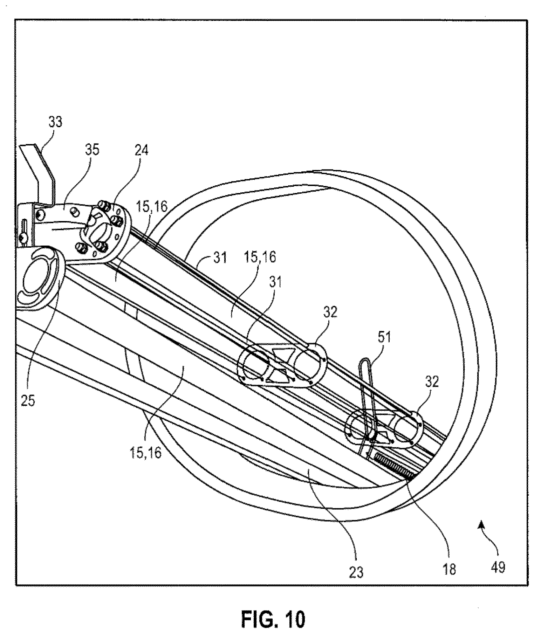

Figure 10 schematically depicts movably and inwardly inserting a member of the UVT-4 family of UV devices through an opening **48** of a container **49** into a container **49.** The UV device is shown further inserted into the container as in FIG. 9. Parts and components are as in FIGS. 1-9. In addition, carrying handle **51** is shown. Details of individual parts and components are described herein.

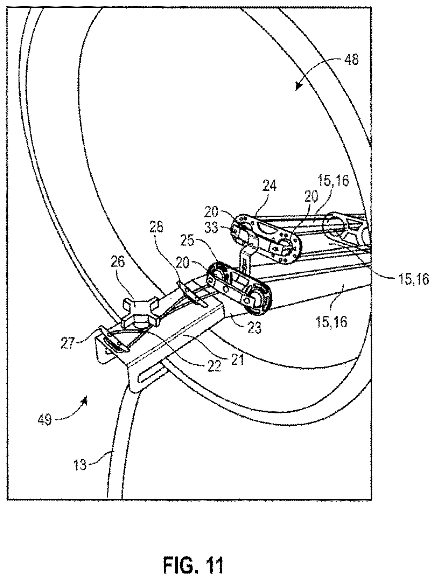

Figure 11 schematically depicts temporarily attaching a member of the UVT-4 family of UV devices at an opening **48** of a container **49.** The UV device has been further inserted through the opening **48** as compared to FIG. 10. Parts and components are as in FIGS. 1-10. Cable **13** connects the UV light source of the UV device with the control box **1** (not shown in figure). Details of individual parts and components are described herein.

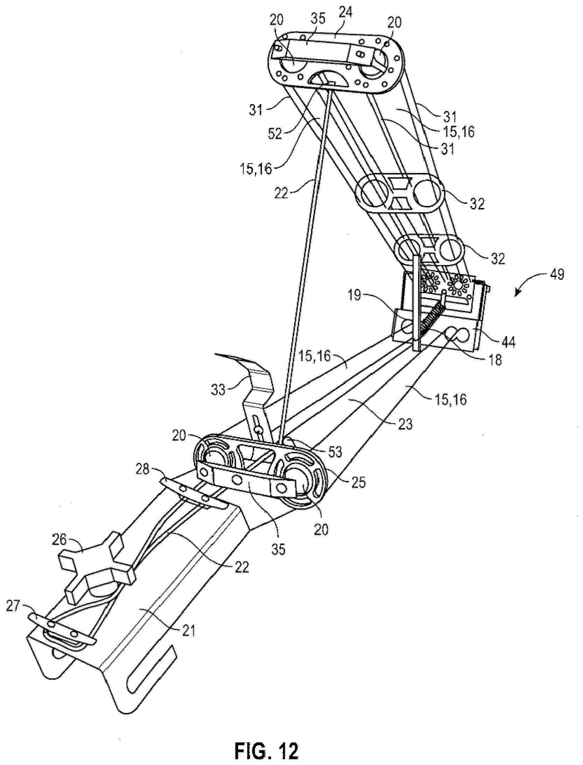

Figure 12 schematically depicts moving the upper frame of a member of the UVT-4 family of UV devices into an angular position with respect to the position of the lower frame **23.** Parts and components are as in FIGS. 1-11. In addition, a first cable guide wheel **53** for rope or cable **22**, and a rope or cable anchoring point **52** at the first upper frame end **24**, are shown. Details of individual parts and components are described herein.

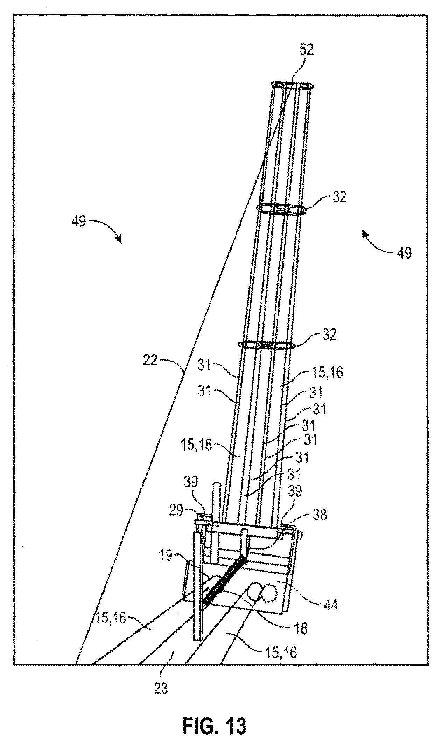

Figure 13 schematically depicts a member of the UVT-4 family of UV devices positioned on the bottom surface of a container **49.** The upper frame and the UV light sources attached thereto have been moved from a horizontal position into a perpendicular/vertical position with respect to the lower frame **23** and the UV light sources attached to the lower frame **23.** Parts and components are as in FIGS. 1-12. Details of individual parts and components are described herein.

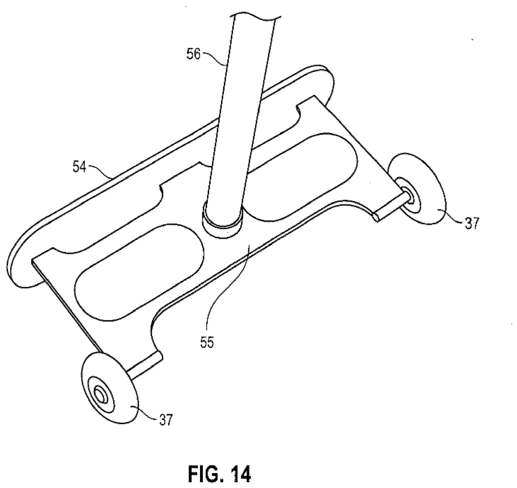

Figure 14 schematically depicts an extension tool for manually moving a UV device within a large container, large room, large space, or large defined environment without a user having to crawl into or be in that large container, large room, large sapce or large defined environment. The exemplary extension tool depicted comprises wheels **37**, a top plate **54**, a base plate **55** and an extension rod **56.** Details of individual parts and components are described herein.

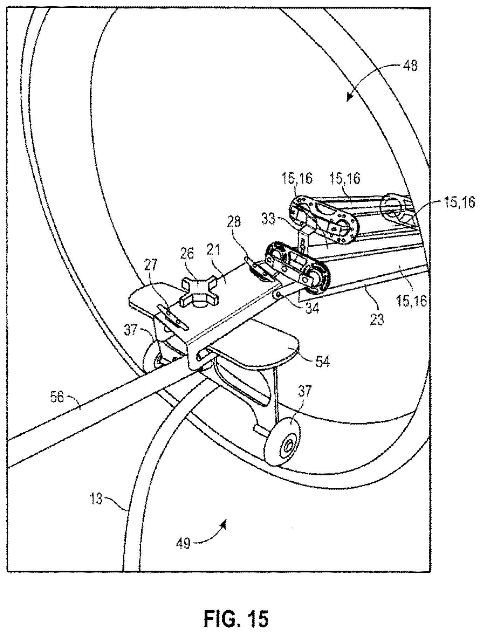

Figure 15 schematically depicts an extension tool attached to a UV device of the UVT-4 family of UV devices. As shown, the extension tool is connected to the UV device through the mounting bracket **21** and bracket tightening knob **26** fastens the mounting bracket **21** to the top plate **54** of the extension tool. Both the extension tool and the UV device are shown to be inserted movably and inwardly into a container **49** through opening **48** (on side wall of container). Parts and components are as in FIGS. 1-14. Details of individual parts and components are described herein.

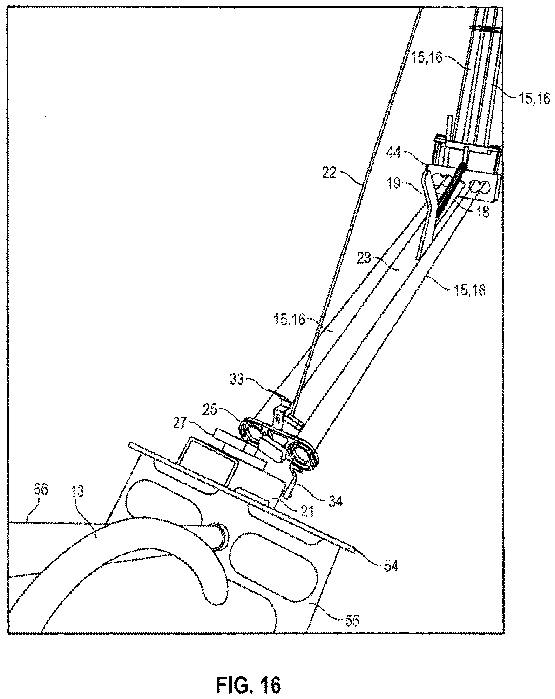

Figure 16 schematically depicts an extension tool attached to a UV device of the UVT-4 family of UV devices. Both the extension tool and the UV device are shown to be positioned on the bottom surface of a container **49** close to the opening **48** of the container **49**. A second hinge **34** movably connecting the lower frame **23** to the mounting bracket **21** is configured so that the extension tool can be moved into an angular position with respect to the lower frame of the UV device. Parts and components are as in FIGS. 1-15. Details of individual parts and components are described herein.

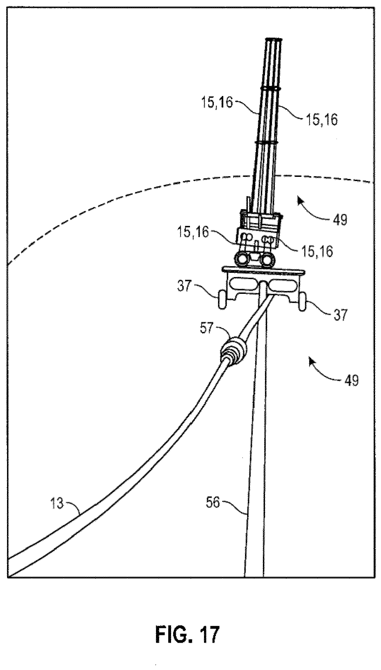

Figure 17 schematically depicts an extension tool attached to a UV device of the UVT-4 family of UV devices. Both the extension tool and the UV device are shown to be positioned on the bottom surface of a container **49.** The extension tool is used to manually move the UV device into a desired position within a container **49**, here into the middle part of the container **49.** A second hinge **34** is configured to move the extension tool from an angular position with respect to the lower frame of the UV device shown in FIG. 16 into a horizontal position (same as lower frame). Parts and components are as in FIGS. 1-16. Also shown is a joint socket **57** joining cables from the control box **1** with cables from the UV light source. Details of individual parts and components are described herein.

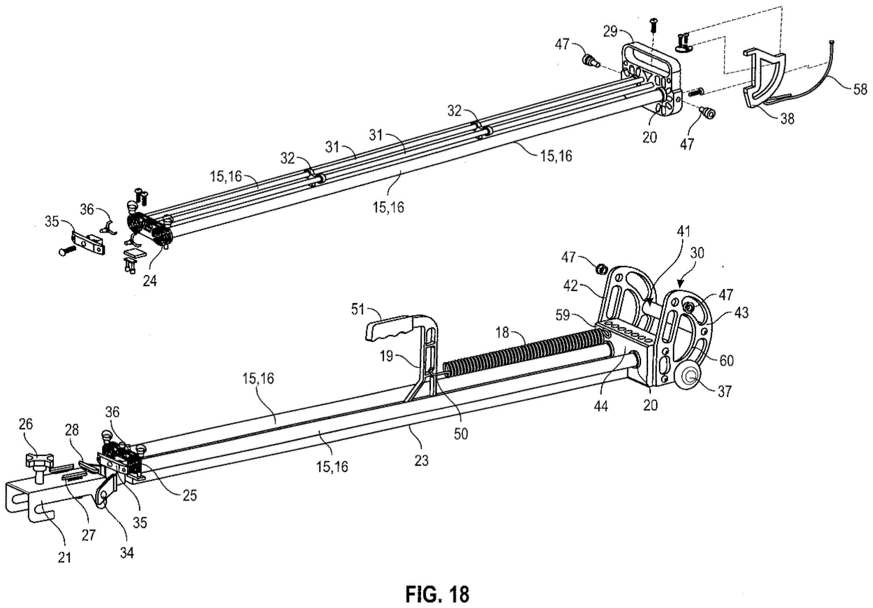

Figure 18 schematically depicts an upper frame (on top) and a lower frame (on bottom) of a UV device of the UVT-4 family of UV devices, with parts and components attached thereto or to be attached thereto. Parts and components are as in FIGS. 1-17, although some are shown with a different configuration. In addition, a T-shaped cap **35** is shown to keep bulb clamps **36** in place. With respect to the upper frame, the figure shows the position to which a first hinge **38** and cable **58** are attached. With respect to both upper and lower frame, the figure shows where fasteners **47** are used to movably connect the upper frame to the lower frame. This exemplary embodiment, in comparison to the ones shown in FIGS. 3-13 and 15-17 shows only two protective rods **31** on the upper frame (vs. four) and smaller and differently configured cross connectors **32.** Also, carrying handle **51** and second anchoring post **19** for extension spring **18**, have a different configuration. Thus, one of ordinary skill in the art will appreciate that individual parts of a UV device described herein can be configured differently and still serve the function(s) as described herein. Details of individual parts and components are described herein.

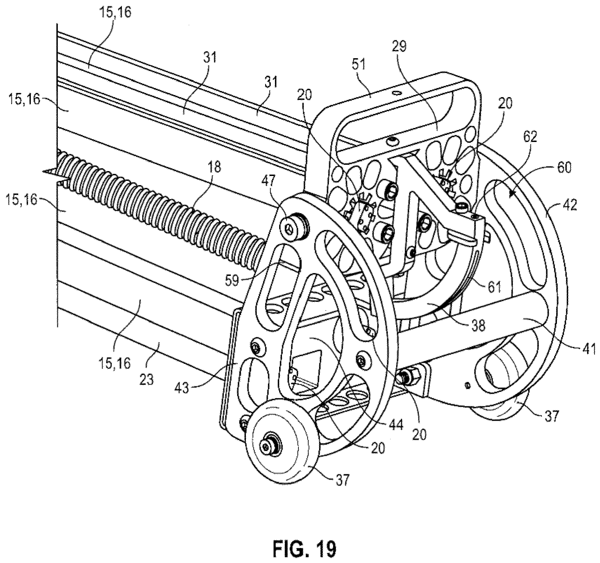

Figure 19 schematically depicts a close-up showing attachment of the first hinge **38** to the upper frame and the movably and operatively connection of the upper frame to the lower frame **23** by fasteners **47.** Cable **58** (not shown) is attached to the first hinge **38**, runs through a cable guide **61** on the first hinge **38** and is locked in position at a cable anchoring point **62.** Parts and components are as described in FIGS. 1-18. Details of individual parts and components are described herein.

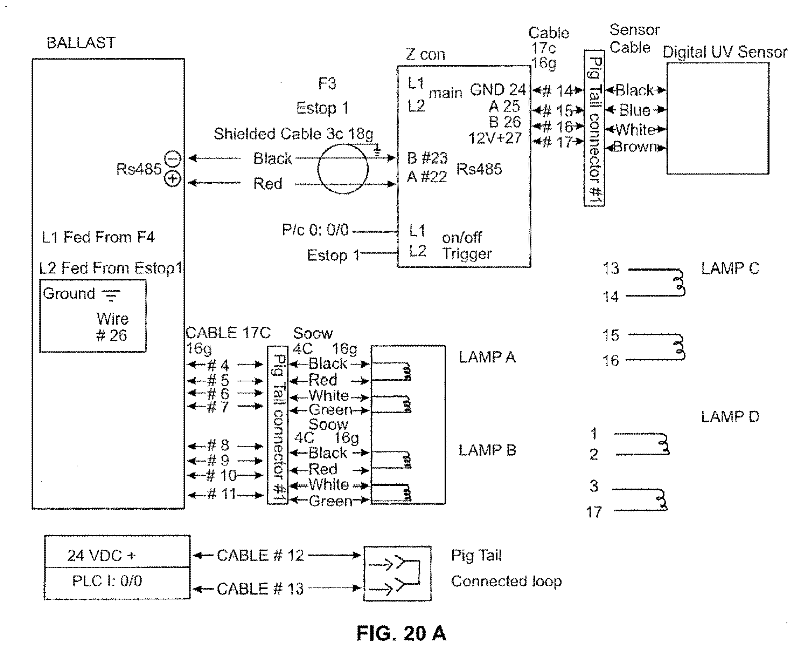

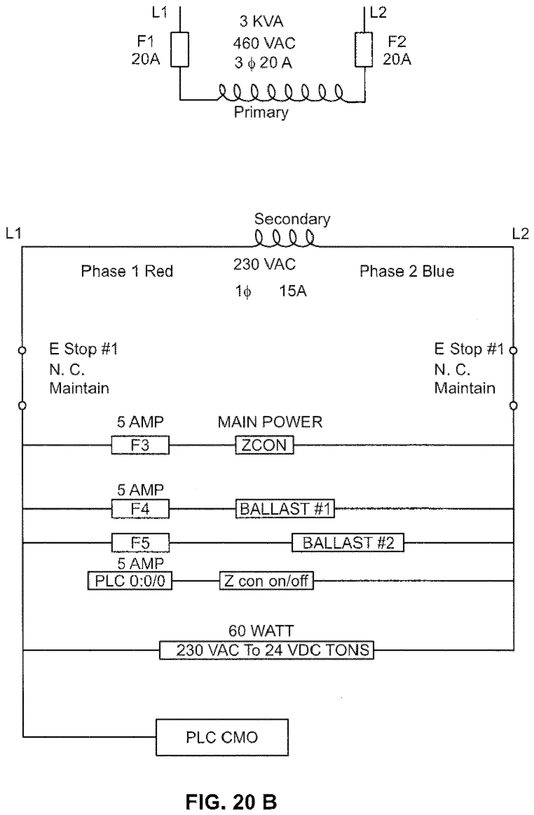

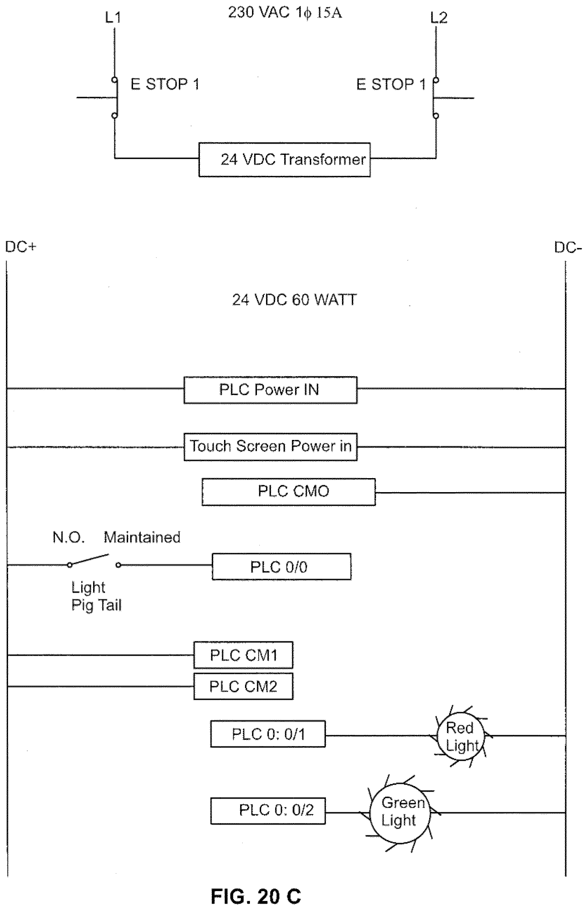

Figures 20A, 20B, and 20C schematically depict parts of an interior layout of an exemplary control box **1** for use in connection with a UV device of the present invention. The following components of an exemplary layout are shown: L1: Input Line 1; L2: Input Line 2; F1: Fuse 1; F2: Fuse 2; 3KVA, 460VAC, Primary: single phase transformer input; Secondary 230VAC: single phase transformer output; Phase 1 red: color coded wire (red); Phase 2 blue: color coded wire (blue); E. STOP #1 NC Maintain: Emergency Stop (also Estop1); F3, F4, F5: 5 Amp fuses; Zcon: integrated control system for lamps from ZED; PLC0/0.0, PLC 0: 0/1, PLC 0: 0.2: Programmable logic controller output; 230VAC to 24 VDC TONS: Power supply; PLC CMO: Programmable logic controller input; 24VDC Transformer: Transformer; PLC power in: Programmable logic controller power input; Touch Screen power in: Touch Screen power input; PLC CM1, PLC CM2: Programmable logic controller input 1 and 2; 1, 2, 3, 13, 14, 15, 16, 17: lines input to lamps (Lamps A, B, C, and D); black, blue, brown, white: color coded wires going into "pig tail connector" (corresponding to cable **13** connecting the control box with the portable UV device), ballast cord wires; RS485: communication cable; 24VDC+: lamp activation indicator/pigtail connection loop; Shielded cable 18g: shielded cable 18 gauge wire; Ground #26: ground wire; 4, 5, 6, 7, 8, 9, 10, 11, 12: lines output to lamps; black, red, white, green: color coded wires. Details of individual parts and components are described herein.

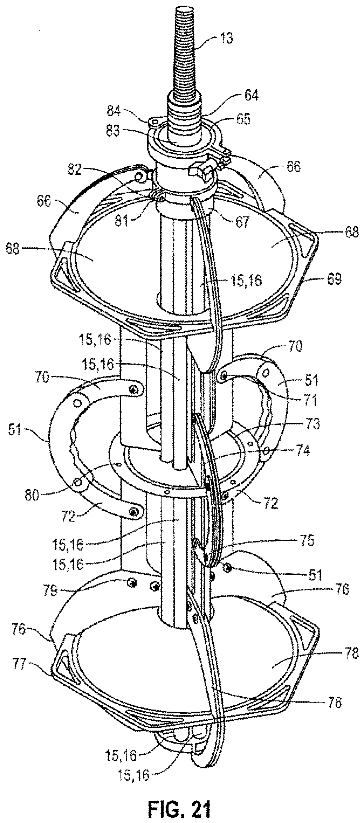

Figure 21 schematically depicts a UV device of the UV6K family of UV devices. Characteristic features of UV devices of this family are the presence of one or more reflectors and six UV light sources. The following parts are shown: Cable **13** connecting control box **1** (not visible) with UV light sources **16**, optional housing **15**, handle **51**, first ring **64**, tri-clamp solid end cap **65**, top clamp **66**, top mounting tube **67**, first or upper parabolic reflector **68**, top outer frame **69** (holding first or upper parabolic reflector in place), upper handle core **70**, button head **71**, lower handle core **72**, linear divider **73**, divider disk **74**, binding post barrel **75**, bottom clamp **76**, bottom outer frame **77** (holding second or lower parabolic reflector in place), second or lower parabolic reflector **78**, thin nut **79**, divider retaining ring **80**, UV lamp alignment plug **81**, top-star bracket **82**, second ring **83**, tri-clamp wing nut clamp **84.** Details of individual parts are described herein.

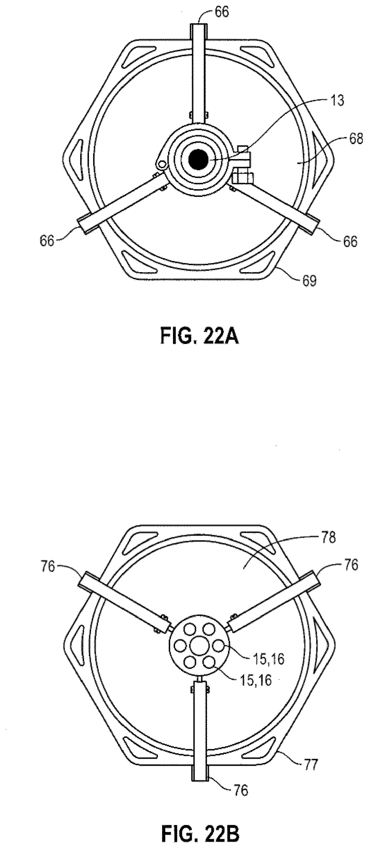

Figure 22A schematically depicts a UV device of the UV6K family of UV devices (top view). Parts are as described in FIG. 21.
Figure 22B schematically depicts a UV device of the UV6K family of UV devices (bottom view). Parts are as described in FIG. 21.

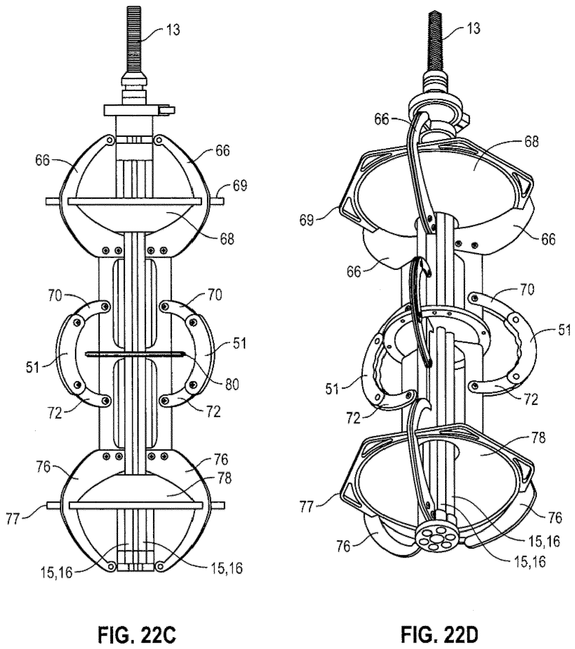

Figure 22C schematically depicts a UV device of the UV6K family of UV devices (frontal side view). Parts are as described in FIG. 21.
Figure 22D schematically depicts a UV device of the UV6K family of UV devices (side/bottom view). Parts are as described in FIG. 21.

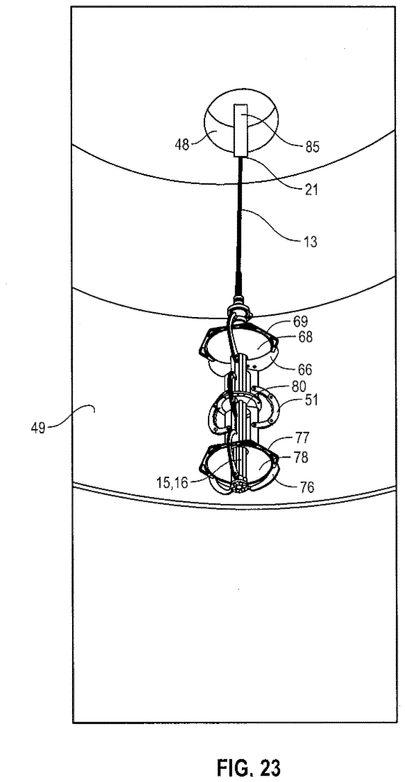

Figure 23 schematically depicts a UV device of the UV6K family of UV devices being inserted through an opening **48** located at the top of a container **49** into the container **49** and descending to a desired position within the container **49.** Parts are as described in FIG. 21. In addition, the extension **85** of a mounting bracket **21** is shown schematically.

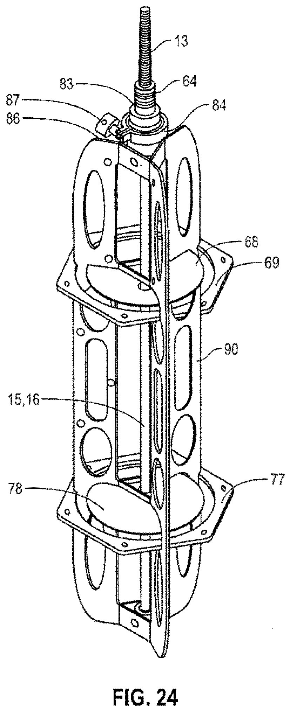

Figure 24 schematically depicts a UV device of the UV2K family of UV devices. Characteristic features of UV devices of this family are the presence of one or more reflectors and a single UV light source. The following parts are shown: Cable **13** connecting control box **1** (not visible) with UV light source **16**, optional housing **15**, first ring **64**, first or upper parabolic reflector **68**, top outer frame **69**, bottom outer frame **77**, second or lower parabolic reflector **78**, second ring **83**, first fastener **86**, second fastener **87**, vertical frame **90.** The three vertical frames **90** hold in place the upper and lower parabolic reflectors **68**, **78.** Details of individual parts are described herein.

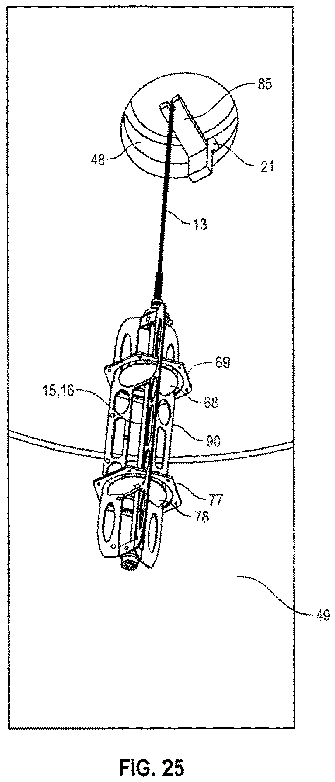

Figure 25 schematically depicts a UV device of the UV2K family of UV devices being inserted through an opening **48** located at the top of a container **49** into the container **49** and descending to a desired position within the container **49.** Parts are as described in FIG. 24. In addition, the extension **85** of a mounting bracket **21** is shown.

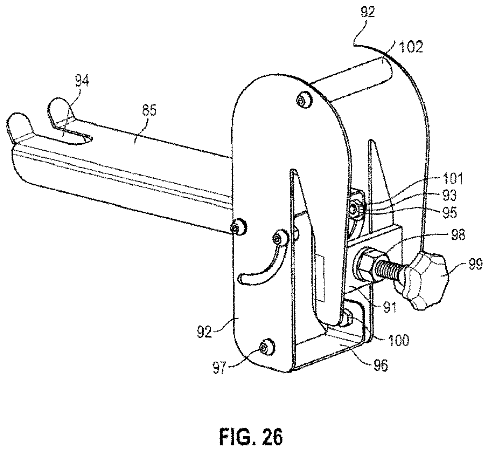

Figure 26 schematically depicts a mounting bracket used for attaching a UV device of the UV6K family and UV2K of UV devices to an opening **48** at a container **49.** The following parts are shown: cross brace-bolt mount plate **91**, gate hook side plate **92**, teflon washer **93**, main support channel **94**, button-head screw **95**, lower cross brace bracket **96**, button head **97**, flanged nut **98**, clamping know with stud **99**, acorn nut **100**, thin nylon nut **101**, Delrin spreader handle **102.**

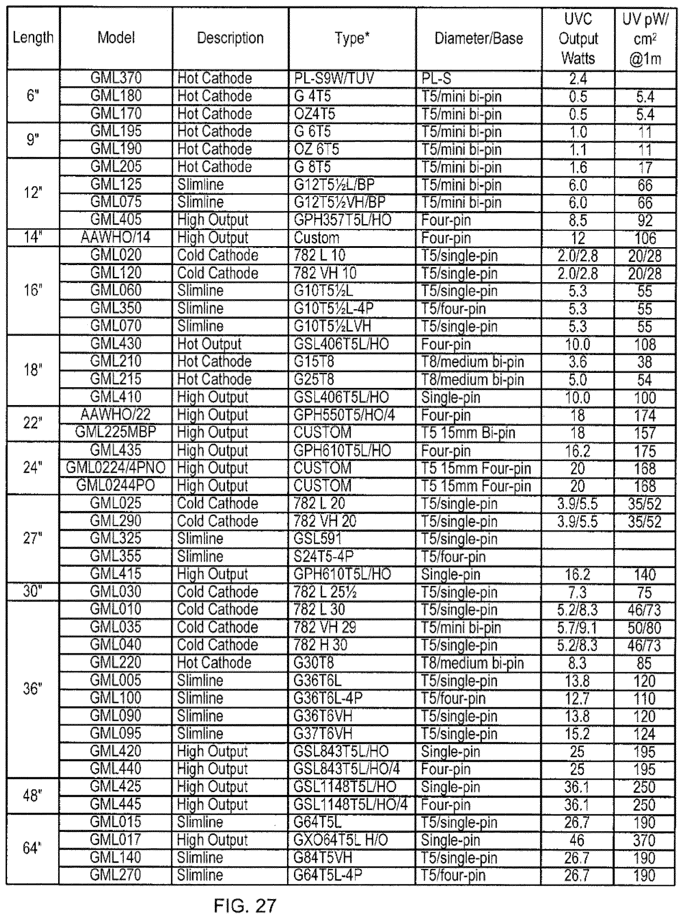

Figure 27 provides a variety of commercially available UV lamps of different length, shape, and type useful in the present invention (American Air & Water Inc., Hilton Head Island, SC 29926, USA). For each UV lamp, the UV-C output is provided in watts and the UV intensity is provided in UV µW/cm² at 1m. Length as indicated reflects nominal length with standard lamp holders adding 2" overall length. Additional lamp lengths and types are available. *, Ozone is negligible unless noted as OZ for high or VH for very high ozone production.

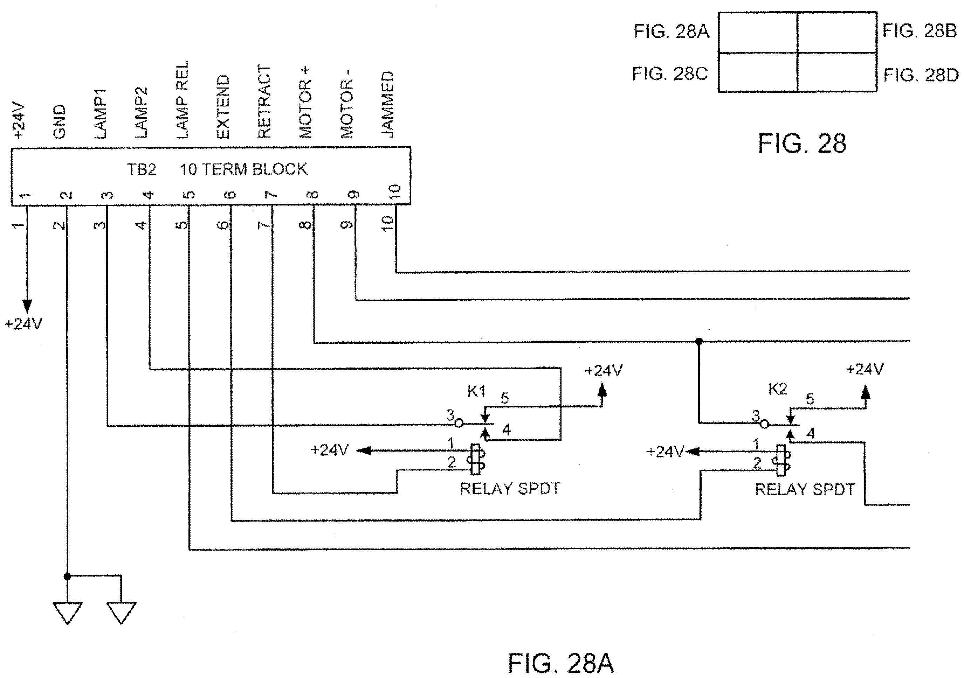

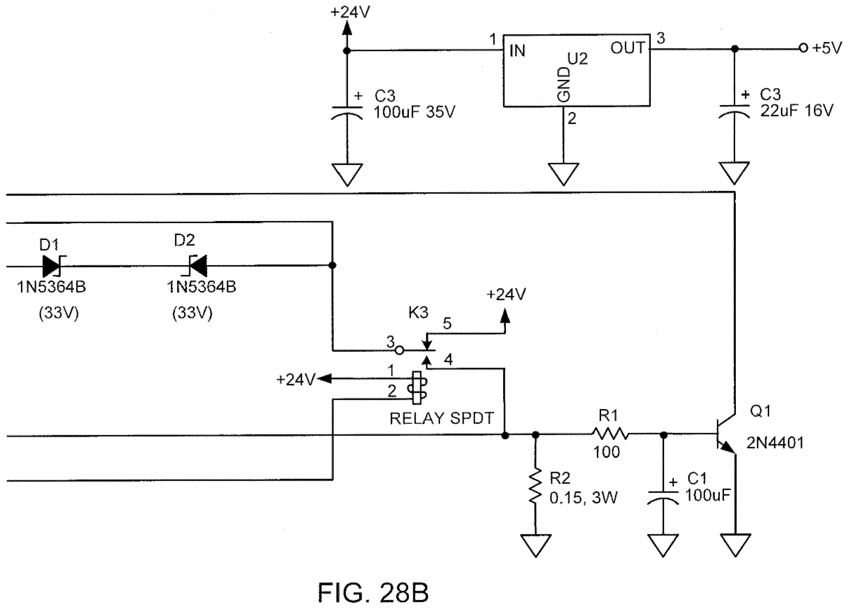

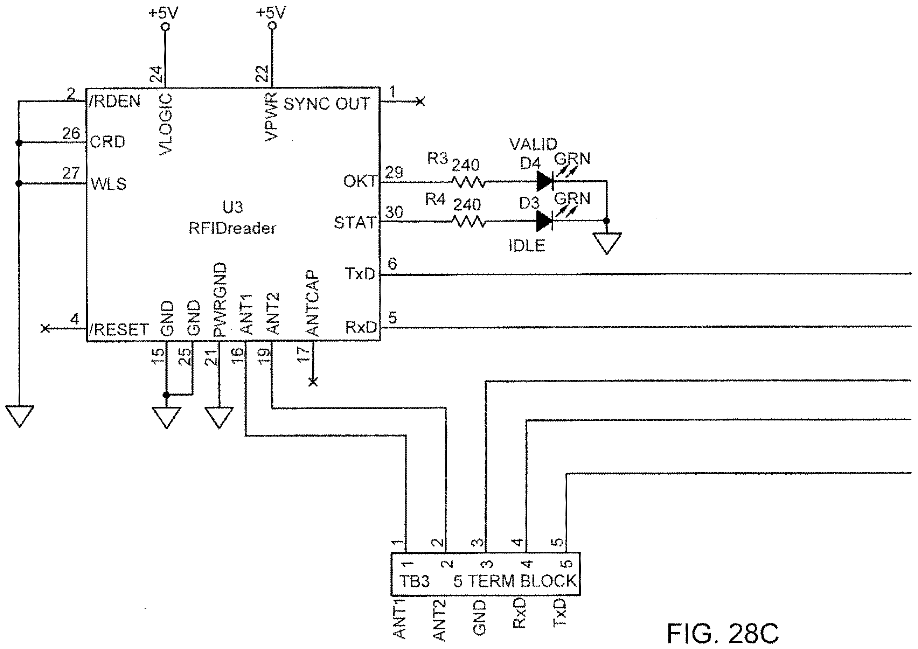

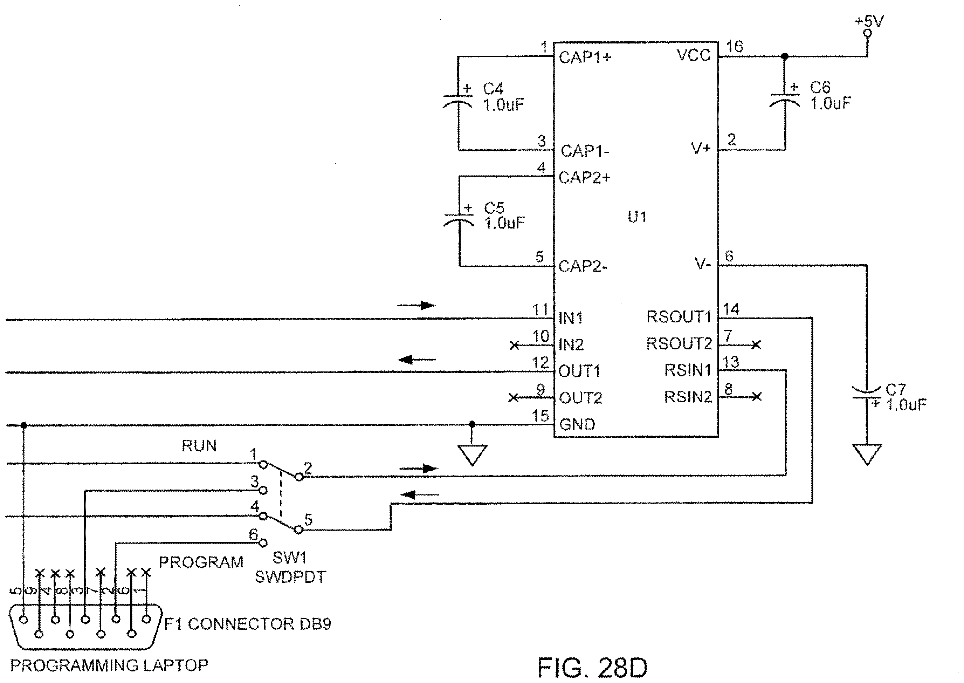

Figure 28 consists of FIG. 28A, FIG. 28B, FIG. 28C and FIG. 28D arranged as shown in the upper right corner of FIG. 28A and schematically depicts an exemplary circuit board for use in a UV device of the present invention. The circuit board, which provides for several functionalities, can be attached to a UV device and, e.g. communicates with an RFID chip that can be mounted to an interior wall of the container. Once information is retrieved from the RFID chip, the circuit board will control movement, the length of which a UV device descends into a container (i.e., the length the UV light source is moved from an opening of container, room, space or defined environment into a vertical downwards position into the container, room, space or defined environment) and the rate of descent based on dimensions of the container, room, space or defined environment as it may be stored in the RFID chip. As one of ordinary skill in the art will appreciate, the exemplary circuit board shown, comprises a TI module (part number shown) and a serial port. Also shown on the circuit board are relays to control a motor and the positioning of the UV light source. In some embodiments is also a 5VDC regulator to power the electronics. In the exemplary circuit board shown, the RFID tag part number is also shown. Other functionalities of a circuit board are described herein.

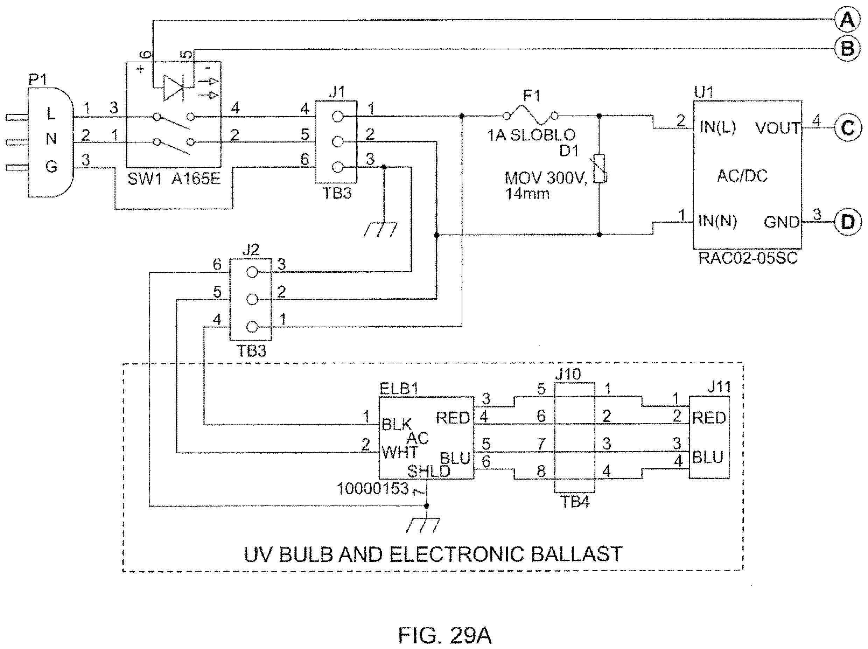

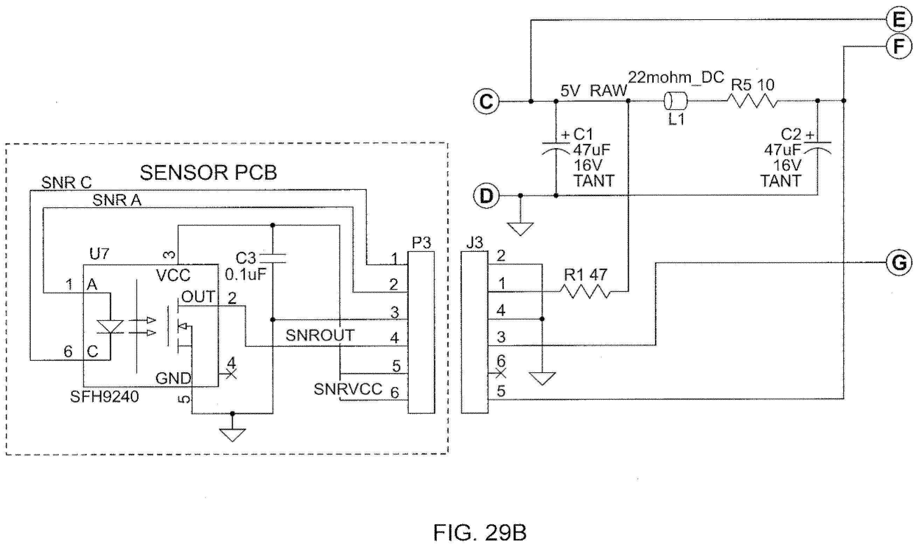

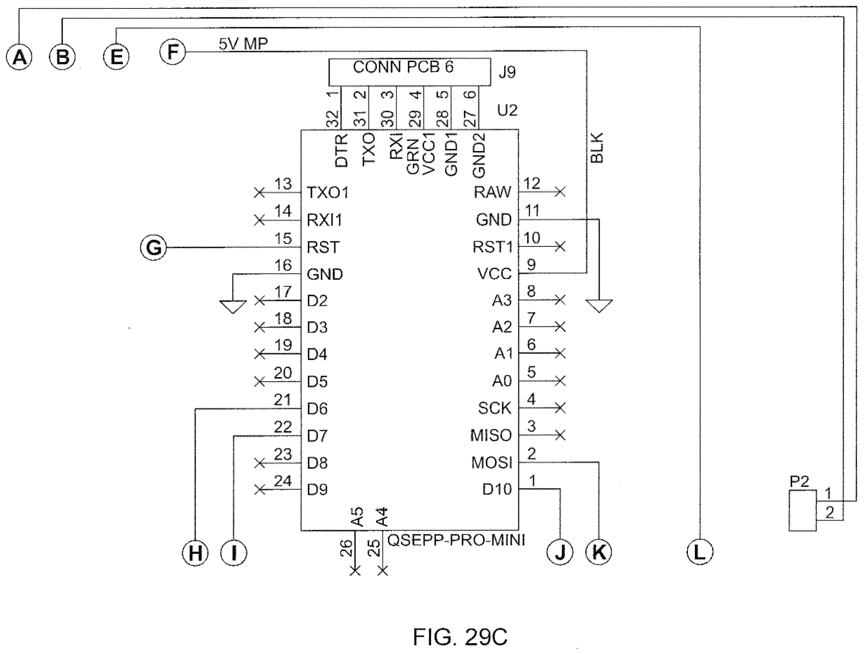

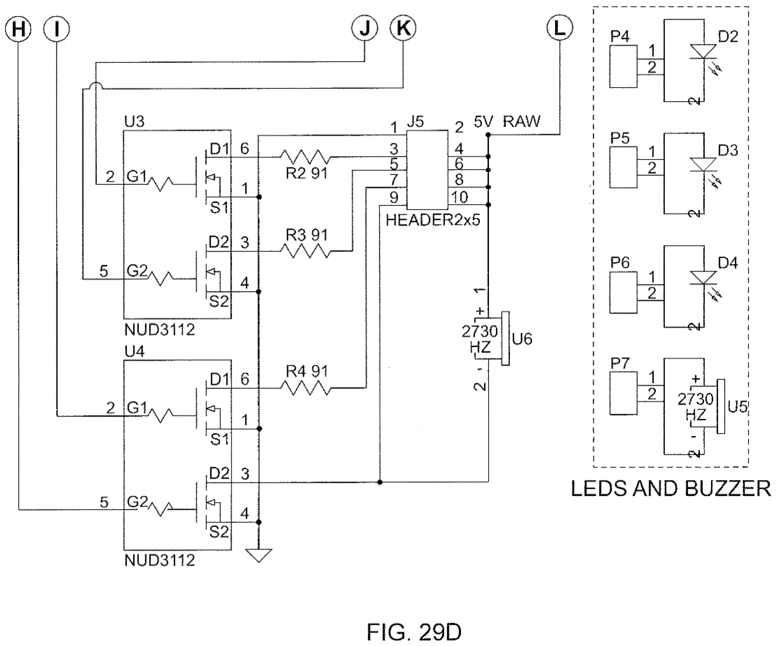

Figures 29A-D schematically depict a further embodiment of an exemplary circuit board for use in a UV device of the present invention. The circuit board depicted includes an optical sensor which starts a timer once the unit is inserted into a container, room, space or defined environment. It also controls a small speaker that emits an audible sound once a sterilization cycle has begun and upon completion of the sterilization cycle. It further controls a series of LED lights that signify a given status of the sterilization cycle, *e.g*., indicating time elapsed. The LED bulbs may blink once intermittently during the first minute, twice intermittently in the second minute, three times intermittently in the third minute and so on until the final minute of the sterilization cycle.

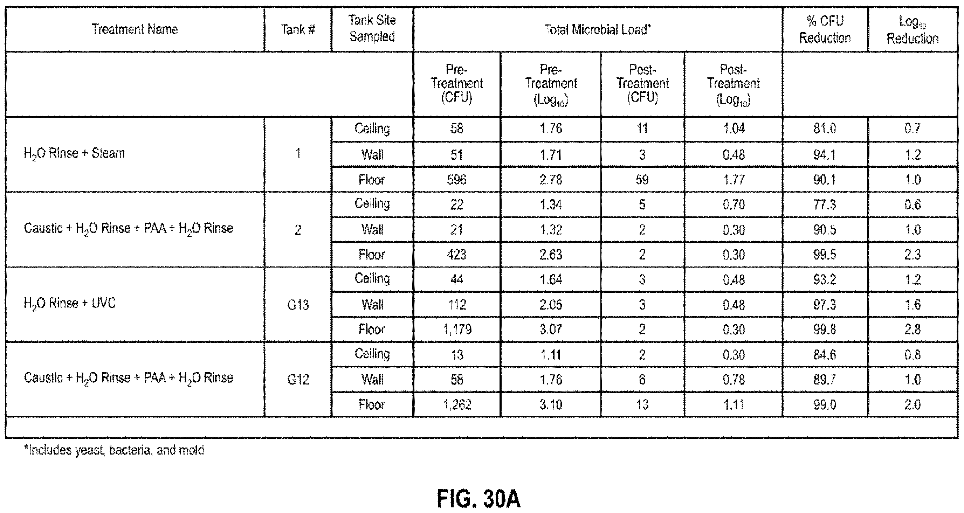

Figure 30A depicts a data set for a comparative trial testing efficacies of steam, PAA, and UVC (UVT-4 Model) sanitizing methods on reduction of total microbial loads on interior surfaces of stainless steel tanks. The data set includes a description of all four sanitation treatment methods performed on interior of stainless steel tanks; the tank number; the sites sampled on each tank (ceiling, wall, and floor); the total microbial load (includes yeast, bacteria, and mold) determined prior to each treatment; the total microbial load determined after each treatment; the percent CFU reduction in microbe populations after application of sanitizer; and the Log₁₀ reduction in microbe populations after application of sanitizer. Details are described in Example 3.

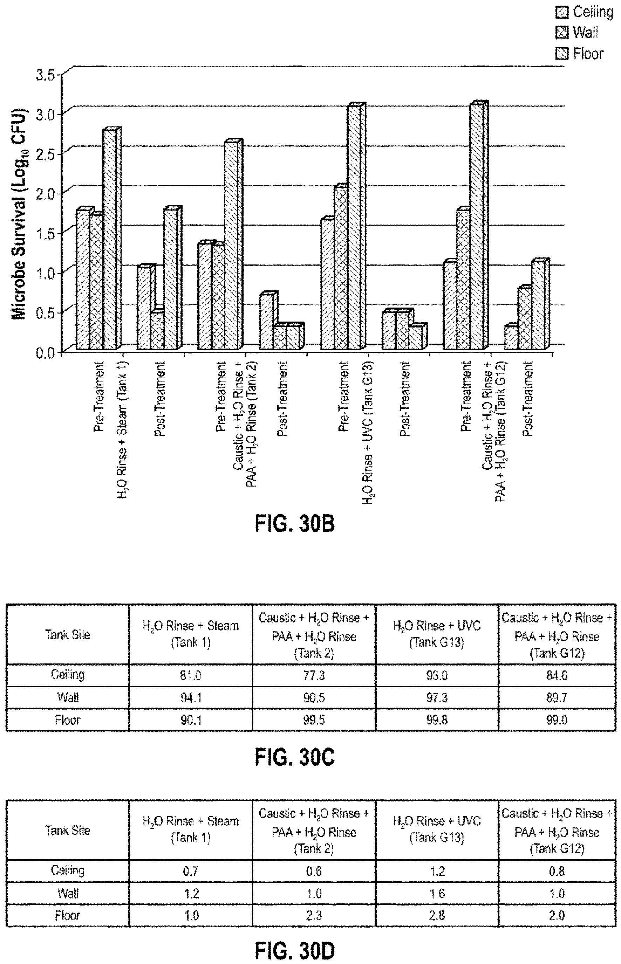

Figure 30B schematically depicts the effect of sanitizing interior of tanks with steam, PAA, and UVC on the survivability of microbial populations on ceiling, wall, and floor of each tank. Treatment with caustic and PAA was performed twice: once in comparison to steam treatment and once in comparison to UVC treatment. Microbe survival is represented as Log₁₀ CFU. Details are described in Example 3.
Figure 30C depicts a data set showing the percent CFU reduction in microbes on ceiling, wall, and floor of stainless steel tanks after application of the various sanitizer methods as indicated. Details are described in Example 3.
Figure 30D depicts a data set showing the Log₁₀ reduction in microbes on ceiling, wall, and floor of stainless steel tanks after application of the various sanitizer methods as indicated. Details are described in Example 3.

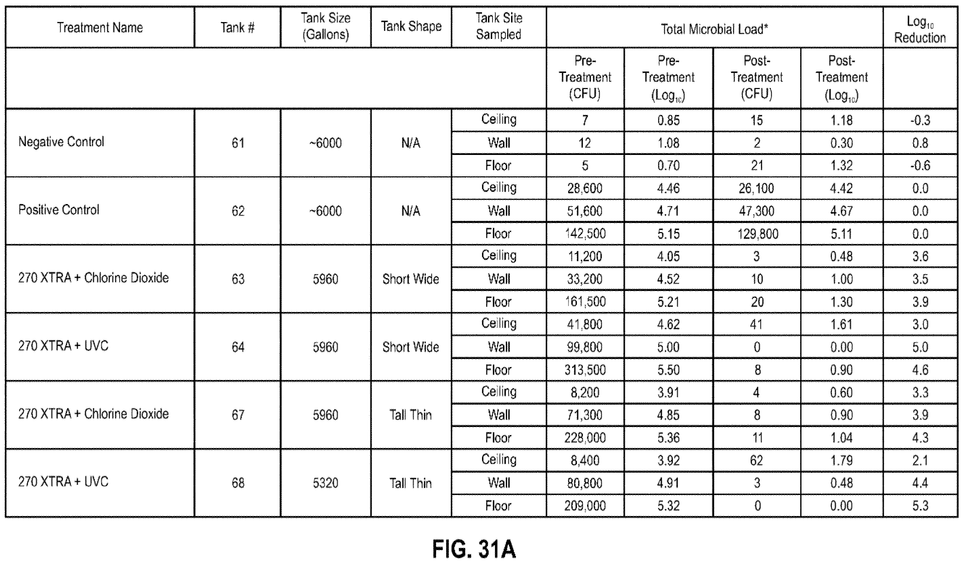

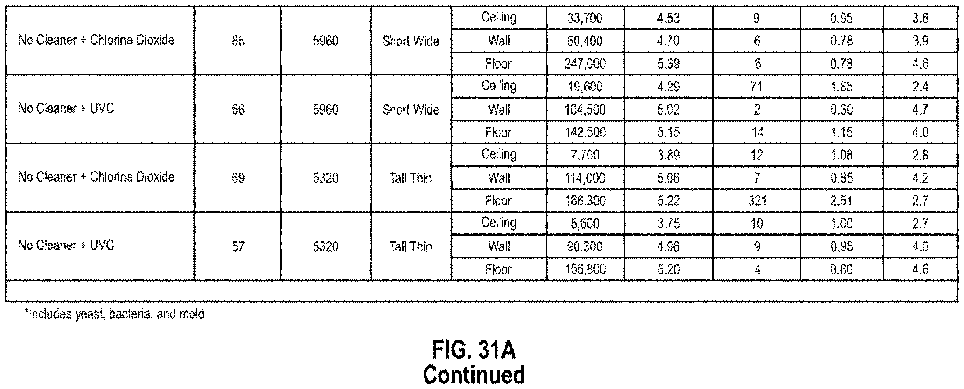

Figure 31A depicts the complete data set for a comparative study testing the efficiency of chlorine dioxide (ozone) and UVC (UVT-4 Model) sanitizing methods as detailed in Example 4. The data set includes a description of all ten treatments performed on interior of stainless steel tanks, the tank number, the tank size, and the tank shape used for each treatment, the sites sampled on each tank (ceiling, wall, and floor), the total microbial load (includes yeast, bacteria, and mold) determined prior to each treatment, the total microbial load determined after each treatment, and the Log₁₀ reduction in microbe populations after application of sanitizer.

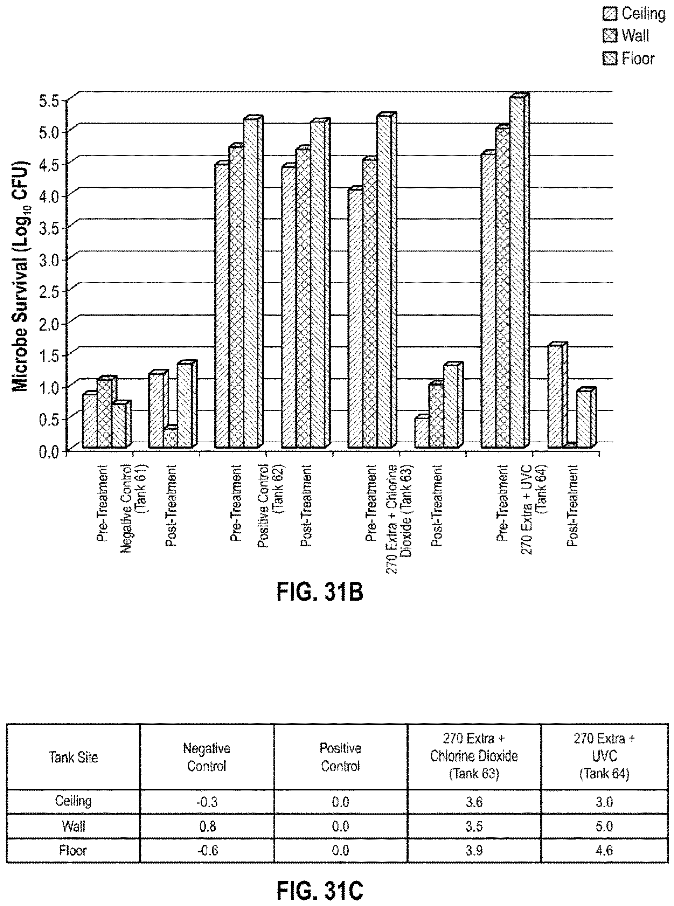

Figure 31B schematically depicts survival of microbes on contaminated short wide stainless steel tanks after cleaning and then sanitizing with either chlorine dioxide or UVC (UVT-4 Model) (tanks 63 and 64). Details are described in Example 4.
Figure 31C depicts Log₁₀ reduction of microbe populations on short wide stainless steel tanks after application of cleaner and then sanitizing with either chlorine dioxide or UVC (UVT-4 Model). Details are described in Example 4.

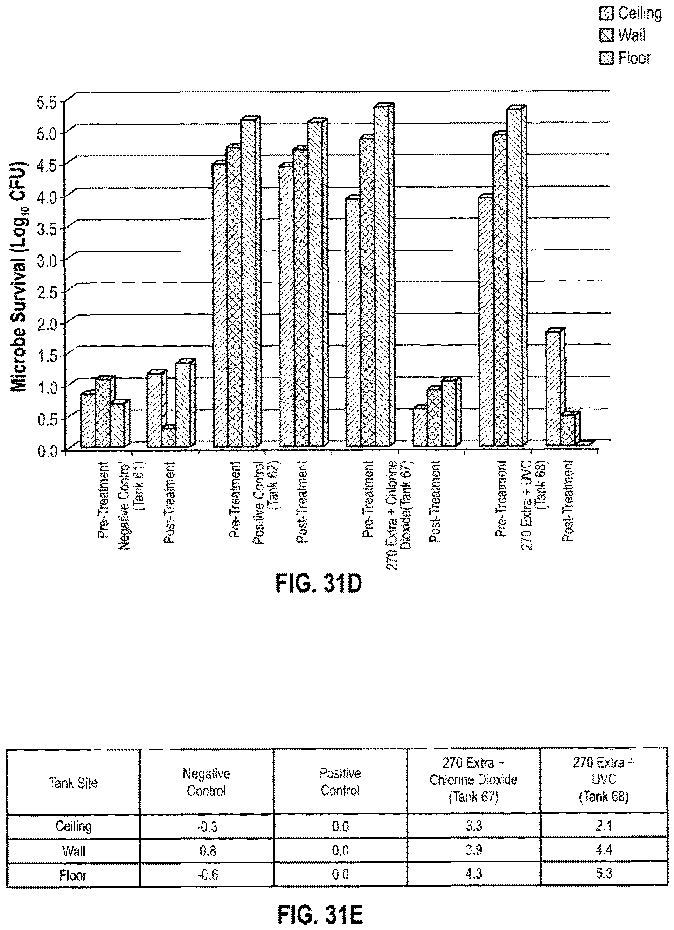

Figure 31D schematically depicts survival of microbes on contaminated tall thin stainless steel tanks after cleaning and then sanitizing with either chlorine dioxide or UVC (UVT-4 Model) (tanks 67 and 68). Details are described in Example 4.
Figure 31E depicts Log₁₀ reduction of microbe populations on tall thin stainless steel tanks after application of cleaner and then sanitizing with either chlorine dioxide or UVC (UVT-4 Model). Details are described in Example 4.

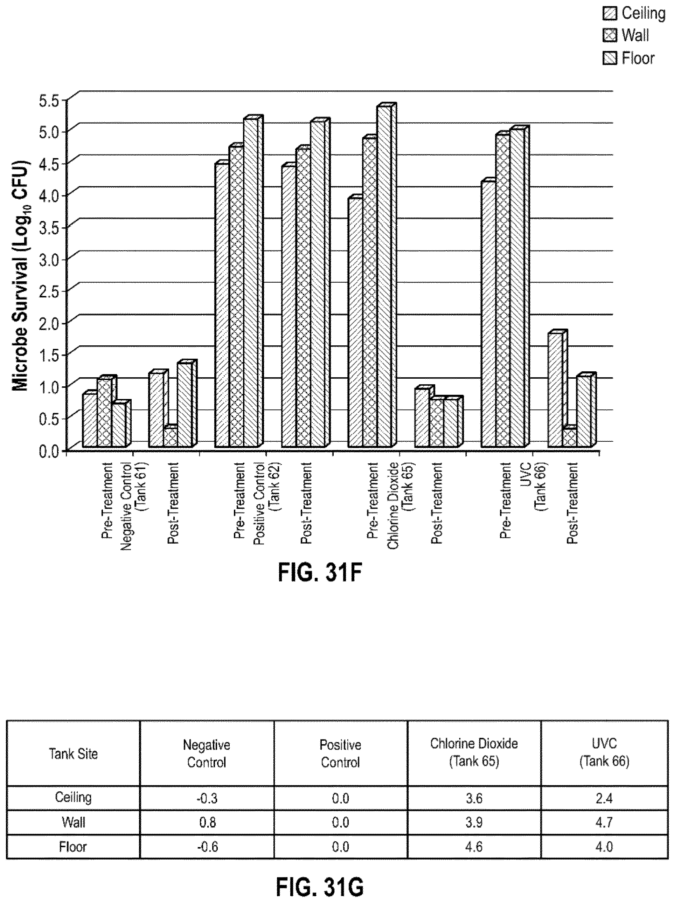

Figure 31F schematically depicts survival of microbes on contaminated short wide stainless steel tanks after water rinsing and then sanitizing with either chlorine dioxide or UVC (UVT-4 Model) (tanks 65 and 66). Details are described in Example 4.
Figure 31G depicts Log₁₀ reduction of microbe populations on short wide stainless steel tanks after application of water rinse and then sanitizing with either chlorine dioxide or UVC (UVT-4 Model). Details are described in Example 4.

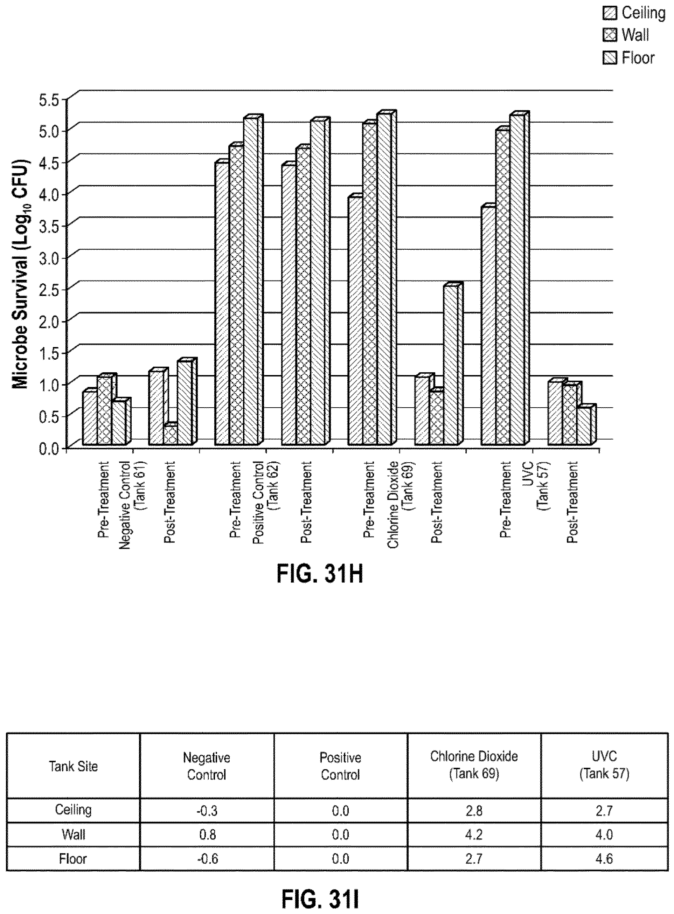

Figure 31H schematically depicts survival of microbes on contaminated tall thin stainless steel tanks after water rinsing and then sanitizing with either chlorine dioxide or UVC (UVT-4 Model) (tanks 69 and 57). Details are described in Example 4.
Figure 31I depicts Log₁₀ reduction of microbe populations on tall thin stainless steel tanks after application of water rinse and then sanitizing with either chlorine dioxide or UVC (UVT-4 Model). Details are described in Example 4.

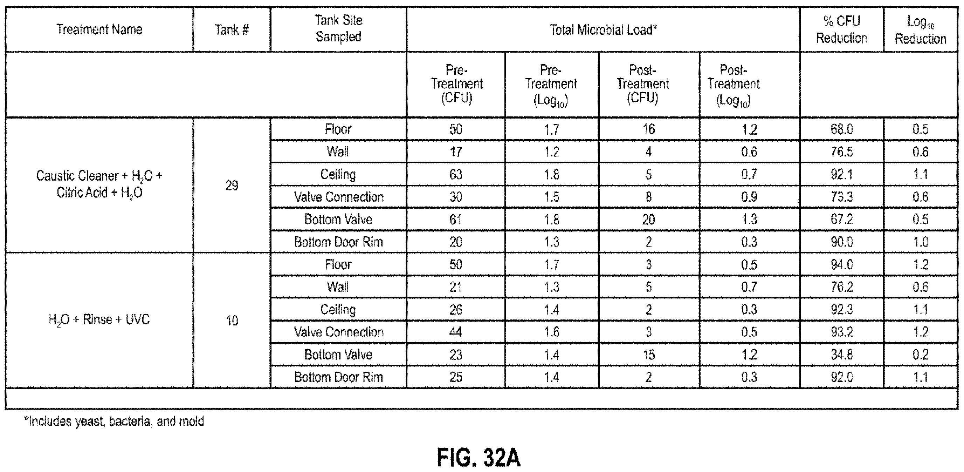

Figure 32A depicts a data set for a comparative trial testing efficacies of caustic cleaner/citric acid, and UVC (UV6K Model) sanitizing methods on reduction of total microbial loads on interior surfaces of stainless steel tanks. The data set includes a description the two sanitation treatment methods performed on interior of stainless steel tanks; the tank number; the tank sites sampled (floor, wall, ceiling, valve connection, bottom valve, bottom door rim); the total microbial load (includes yeast, bacteria, and mold) determined prior to each treatment; the total microbial load determined after each treatment; the percent CFU reduction in microbe populations after application of sanitizer; and the Log₁₀ reduction in microbe populations after application of sanitizer. Details are described in Example 5.

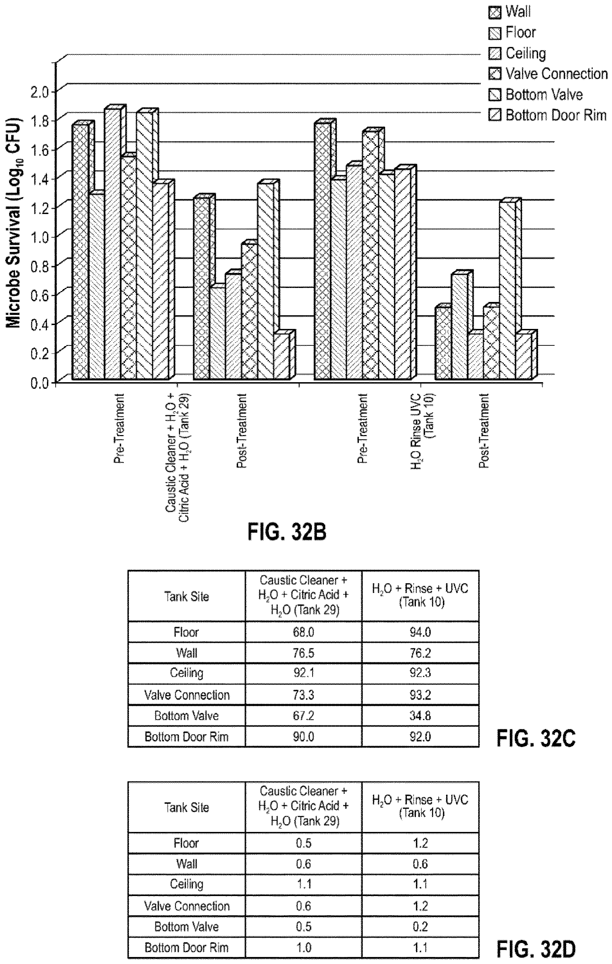

Figure 32B schematically depicts the effect of sanitizing interior of tanks with caustic cleaner/citric acid, and UVC (UV6K Model) on the survivability of microbial populations on floor, wall, ceiling, valve connection, bottom valve, and bottom door rim (columns from left to right) of each tank. Microbe survival is represented as Log₁₀ CFU. Details are described in Example 5.
Figure 32C depicts a data set showing the percent CFU reduction of microbe populations on stainless steel tanks on floor, wall, ceiling, valve connection, bottom valve, and bottom door rim of stainless steel tanks after application of the various sanitizer methods as indicated. Details are described in Example 5.
Figure 32D depicts a data set showing the Log₁₀ reduction of microbe populations on stainless steel tanks on floor, wall, ceiling, valve connection, bottom valve, and bottom door rim of stainless steel tanks after application of the various sanitizer methods as indicated. Details are described in Example 5.

### DETAILED DESCRIPTION OF THE INVENTION

### I. DEFINITIONS

Throughout the present specification and the accompanying claims the words "comprise" and "include" and variations thereof, such as "comprises," "comprising," "includes," and "including" are to be interpreted inclusively. That is, these words are intended to convey the possible inclusion of other elements or integers not specifically recited, where the context allows. No language in the specification should be construed as indicating any non-claimed element essential to the practice of the invention.

The terms "a" and "an" and "the" and similar referents used in the context of describing the invention (especially in the context of the following claims) are to be construed to cover both the singular and the plural, unless otherwise indicated herein or clearly contradicted by context.

Recitation of ranges of values herein are merely intended to serve as a shorthand method of referring individually to each separate value falling within the range, unless otherwise indicated herein, each individual value is incorporated into the specification as if it were individually recited herein. Ranges may be expressed herein as from "about" (or "approximate") one particular value, and/or to "about" (or "approximate") another particular value. When such a range is expressed, another embodiment includes from the one particular value and/or to the other particular value. Similarly, when values are expressed as approximations, by use of the antecedent "about" or "approximate" it will be understood that the particular value forms another embodiment. It will be further understood that the endpoints of each of the ranges are significant both in relation to the other endpoint, and independently of the other endpoint. It is also understood that there are a number of values disclosed herein, and that each value is also herein disclosed as "about" that particular value in addition to the value itself. For example, if the value "10" is disclosed, then "about 10" is also disclosed. It is also understood that when a value is disclosed that is "less than or equal to the value" or "greater than or equal to the value" possible ranges between these values are also disclosed, as appropriately understood by the skilled artisan. For example, if the value "10" is disclosed, the "less than or equal to 10 "as well as "greater than or equal to 10" is also disclosed.

All methods described herein can be performed in any suitable order unless otherwise indicated herein or otherwise clearly contradicted by context. The use of any and all examples, or exemplary language (*e.g*., "such as" or "*e.g*.," or "for example") provided herein is intended merely to better illuminate the invention and does not pose a limitation on the scope of the invention otherwise claimed.

Groupings of alternative elements or embodiments of the invention disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is herein deemed to contain the group as modified thus fulfilling the written description of all Markush groups used in the appended claims.

The headings used herein are for organizational purposes only and are not meant to be used to limit the scope of the description or the claims, which can be had by reference to the specification as a whole. Accordingly, the terms defined immediately below are more fully defined by reference to the specification in its entirety.

Illustrations are for the purpose of describing a preferred embodiment of the invention and are not intended to limit the invention thereto.

The abbreviations used herein have their conventional meaning within the mechanical, chemical, and biological arts.

As used herein, the term "about" refers to a range of values of plus or minus 10% of a specified value. For example, the phrase "about 200" includes plus or minus 10% of 200, or from 180 to 220, unless clearly contradicted by context.

As used herein, the term "algorithm" generally refers to a sequence of steps leading to a desired result.

As used herein, the terms "amount effective" or "effective amount" mean an amount, which produces a desired effect, such as a biological effect. In particular, an effective amount of a UV dosage is an amount, which inhibits the growth of a microorganism by at least 90% (by at least 1 log reduction), by at least 99% (by at least 2 log reduction), by at least 99.9% (by at least 3 log reduction), by at least 99.99% (by at least 4 log reduction), by at least 99.999% (at least 5 log reduction), or by at least 99.9999% (at least 6 log reduction).

As used herein, the terms "connect to," "connected to," "attach to" or "attached to," "operatively connect to," "operatively connected to," "operatively attach to, "operatively attached to," or grammatical equivalents thereof, as uswed herein, mean to fasten on, to fasten together, to affix to, to mount to, mount on, to connect to, to couple, to join, to position onto, to position into, to place onto, or to place into and permit an operation or functionality as described, e.g., without limitation UV sterilization or applying a UV sterilization cycle. "Attachment" means the act of attaching or the condition of being attached. Attachment can be direct or indirectly. For example a part A may be attached directly to part B. Alternatively, part A may be attached indirectly to part B through first attaching part A to part C and then attaching part C to part B. More than one intermediary part can be used to attach part A to part B. Attaching can be permanent, temporarily, or for a prolonged time. For example, a UV device of the present invention may be attached to a container, room, space or defined environment temporarily for the time necessary to perform a method of the invention. Alternatively, a UV device of the present invention may be attached to a container or to an object or structure in a room, a space or a defined environment for a prolonged time, *e.g*., also when a method of the present invention is not performed. Also, a UV device of the present invention may be attached permanently to a container or to an object or structure in a room, a space or a defined environment.

The terms "container," "vessel," or "tank" are used interchangeably herein.

As used herein, the terms "germicidal lamp" or "germicidal UV lamp" refer to a type of lamp, which produces ultraviolet (UV) light. Short-wave UV light disrupts DNA base pairing causing thymine-thymine dimers leading to death of bacteria and other microorganisms on exposed surfaces.

As used herein, the terms "inhibiting the growth of a microorganism," "inhibiting the growth of a population of microorganisms," "inhibiting the growth of one or more species of microorganisms" or grammatical equivalents thereof refer to inhibiting the replication of one or more microorganisms and may include destruction of the microorganism(s). Assays for determining inhibiting the growth of a microorganism are known in the art and are described herein.

As used herein, the terms "microorganism" or "microbe" comprise a diverse group of microscopic organisms, including, but not limited to, bacteria, fungi, viruses, archaea, and protists.

The terms "optional" or "optionally" as used throughout the specification means that the subsequently described event or circumstance may but need not occur, and that the description includes instances where the event or circumstance occurs and instances in which it does not. The terms also refer to a subsequently described composition that may but need not be present, and that the description includes instances where the composition is present and instances in which the composition is not present.

As used herein, the term "portable" in the context of a UV device refers to a UV device of the present invention that can be carried by a person and that can be temporarily (e.g., for the duration of a sanitization cycle) attached to a container, a room, a space, or a defined environment.

As used herein, the term "radiation" or grammatical equivalents refer to energy, which may be selectively applied, including energy having a wavelength of between 10⁻¹⁴ and 10⁴ meters including, for example, electron beam radiation, gamma radiation, x-ray radiation, light such as ultraviolet (UV) light, visible light, and infrared light, microwave radiation, and radio waves. A preferred radiation is UV light radiation. "Irradiation" refers to the application of radiation to a surface.

As used herein, the terms "sterile" or "sterilization" and grammatical equivalents thereof refer to an environment or an object, which is free or which is made free of detectable living cells, viable spores, viruses, and other microorganisms. Sometimes the process of sterilization is also referred herein to as "disinfection" or "sanitization." Those terms are used interchangeably herein.

As used herein the term "ultraviolet" and the abbreviation "UV" refer to electromagnetic radiation with wavelengths shorter than the wavelengths of visible light and longer than those of X-rays. The UV part of the light spectrum is situated beyond the visible spectrum at its violet end.

As used herein, the abbreviation "UV-A" refers to ultraviolet light in the range of 315-400 nanometers (nm).

As used herein, the abbreviation "UV-B" refers to ultraviolet light in the range of 280-315 nanometers (nm).

As used herein, the abbreviation "UV-C" refers to ultraviolet light in the range of 200-280 nanometers (nm).

As used herein, the term "UV dose" refers to an amount of UV irradiation absorbed by an exposed population of microbes, typically in units of mJ/cm² (mJ/cm² = 1,000 µW/cm² per second).

As used herein, the terms "UV intensity" or "UV irradiance" refer to the irradiance field of a UV germicidal irradiation system (such as a UV light source described herein), i.e., the total radiant energy incident on a surface from all directions. It is measured in µW/cm² at 1m. The UV intensity greatly depends on the distance from the UV emitter and the transmittance of the medium.

As used herein, the terms "ultraviolet radiation" or "UV radiation" refer to radiation having a wave-length or wavelengths between from 160 to 400 nm. If a range is specified, a narrower range of radiation is meant within the 160 to 400 nm range. The range specified, unless otherwise indicated, means radiation having a wavelength or wavelengths within this specified range.

In the following description it is to understood that terms such as "forward," "rearward," "front," "back," "right," "left," upward," "downward," "horizontal," "vertical," "longitudinal," "lateral," "angular," "first," "second" and the like are words of convenience and are not to be construed as limiting terms.

The present invention generally relates to compositions, systems and methods for ultraviolet (UV) sterilization, and more specifically, to compositions, systems and methods for UV sterilization of a container, room, space, or defined environment. In one aspect, present invention generally relates to compositions, systems and methods for ultraviolet (UV) sterilization of a container, and more particularly to compositions, systems and methods for UV sterilization of a container used in the process of fermentation for an alcoholic beverage. A system as described herein comprises a UV device and a container or a UV device and a case.

### II. UV DEVICES

The present invention describes a variety of UV devices, in particular, portable UV devices. In some embodiments of the present invention, a UV device is a UV device as depicted in FIGS. 3-13 and 15-19. In some embodiments of the present invention, a UV device is a UV device as depicted in FIGS. 21, 22A-D, and 23. In some embodiments of the present invention, a UV device is a UV device as depicted in FIGS. 24 and 25. UV devices depicted in FIGS. 3-13, 15-19, 21, 22A-D, and 23-25 are portable UV devices.

### A. UV Light Sources

A UV device of the present invention comprises a UV light source, also referred to as UV lamp. In some embodiments, UV light sources are referred to as a first UV light source, a second UV light source, etc.

In some embodiments of a UV device of the present invention, a UV light source comprises one UV lamp. In some embodiments of a UV device of the present invention, a UV light source comprises one or more UV lamps. If a UV light source comprises more than one UV lamp, e.g., two, three, four, five, six, seven, eight or more UV lamps, it is also referred to as a "UV lamp cluster," "UV cluster," "UV lamp assembly" or "UV assembly."

In some embodiments, a UV light source of the present invention comprises a halogen lamp. A halogen lamp includes, but is not limited to a tungsten halogen lamp, a quartz halogen lamp and a quartz iodine lamp. Halogen lamps are known in the art and are commercially available, *e.g*., General Electric model 16751.

In some embodiments, a UV light source of the present invention comprises a sodium lamp. A sodium lamp includes, but is not limited to a high pressure sodium lamp. Sodium lamps are known in the art and are commercially available, *e.g*., General Electric ED18, 400W, high pressure sodium lamp.

In some embodiments, a UV light source of the present invention comprises an incandescent lamp. An incandescent lamp includes, but is not limited to an electric light filament lamp. Incandescent lamps are known in the art and are commercially available, *e.g*., Philips 60-Watt Household Incandescent Light Bulb.

In some embodiments, a UV light source of the present invention comprises a light emitting diode (LED) or a solid state light emitting device, including, but not limited to a semiconductor laser. LEDs are known in the art and are commercially available, *e.g*., Model L-A3W Energy Efficient UV 110V LED Spot light from Battery Junction.

Additionally, spectral calibration lamps, electrodeless lamps, and the like can be used.

In some embodiments a UV lamp within a UV device has a polymer coating. The polymer coating will prevent small glass pieces from falling into a container in case of accidental shattering during use of a UV device in a method of the present invention.

### 1. Germicidal UV Light Sources

Preferably, a UV light source is a germicidal UV light source. In some embodiments, germicidal UV light sources are referred to as a first germicidal UV light source, a second germicidal UV light source, etc.

Ultraviolet (UV) light is classified into three wavelength ranges: UV-C, from about 200 nanometers (nm) to about 280 nm; UV-B, from about 280 nm to about 315 nm; and UV-A, from about 315 nm to about 400 nm. Generally, UV light, and in particular, UV-C light is "germicidal," i.e., it deactivates the DNA of microorganism, such as bacteria, viruses and other pathogens and thus, destroys their ability to multiply and cause disease, effectively resulting in sterilization of the microorganisms. While susceptibility to UV light varies, exposure to UV energy for about 20 to about 34 milliwatt-seconds/cm² is adequate to deactivate approximately 99 percent of the pathogens. In some embodiments of the present invention, a UV light source is a germicidal UV light source. A UV light source, also referred to herein as UV lamp, is indicated in the drawings and respective legends as **16.**

In some embodiments of a UV device of the present invention, the UV light source is a germicidal UV light source. In some embodiments of a UV device of the present invention, the UV light source is a UV-C light source. In some embodiments of a UV device of the present invention, the UV light source is a UV-B light source. In some embodiments of a UV device of the present invention, the UV light source is a UV-A light source.

### 2. Pulsed Germicidal UV Light Sources

In some embodiments of the present invention, a UV light source is configured to include a pulsed UV light source or a continuous wavelength mode UV light source. In some embodiments of the present invention, a UV light source is a pulsed UV light source. In some embodiments of the present invention, a UV light source is a continuous wavelength mode UV light source.

In some embodiments of the present invention, a germicidal UV light source is a pulsed germicidal UV light source. Pulsed UV light is composed of a wide spectrum of light ranging from the UV region to the infrared (Wang and MacGregor, 2005, Water Research 39(13):2921-25). A large portion of the spectrum lies below 400 nm and as such has germicidal properties. Pulsed UV light has proven equally if not more effective (same sterilization levels achieved more rapidly) at sterilizing surfaces when compared with traditional germicidal UV-C lights (Bohrerova et al., 2008, Water Research 42(12):2975-2982). In a pulsed UV system, UV-light is pulsed several times per second, each pulse lasting between 100 ns (nanosecond) and 2 ms (millisecond). An additional advantage of a pulsed UV light system is that it obviates the need for the toxic heavy metal mercury, which is used in traditional germicidal UV lamps. A pulsed UV system requires less power than a mercury UV lamp and as such, is more economical.

The peak intensity of a pulsed UV lamp is typically one to two orders of magnitude higher than that of a mercury UV lamp of similar wattage. These high peak energies are achieved by storing energy in the high voltage storage capacitor and releasing this energy in a very short burst through the flash lamp. Pulse widths of 10 µs (microsecond) to 300 µs are common in today's industrial flashlamp systems. Peak energy levels range from 300 kilowatts to over a megawatt. (Kent Kipling Xenon Corporation Wilmington, MA). Sterilization is achieved because the intensity of the light produced by the pulsed lamp is greater than that of conventional UV-C lamps. Further, pulsed UV achieves sterilization via the rupture and disintegration of micro-organisms caused by overheating following absorption UV photons emitted in the light pulse (Wekhof et al., "Pulsed UV Disintegration (PUVD): a new sterilization mechanism for packaging and broad medical-hospital applications." The First International Conference on Ultraviolet Technologies. June 14-16, 2001; Washington, DC, USA).

### 3. Low Pressure, Medium Pressure, High Pressure And Ultra-High Pressure Germicidal UV Light Sources

In some embodiments of the present invention, a germicidal UV light source is selected from the group consisting of a low pressure UV lamp, a medium pressure UV lamp, a high pressure UV lamp, and an ultra-high pressure UV lamp.

In some embodiments of the present invention, a germicidal UV light source is a low pressure UV lamp. Low-pressure UV lamps are very similar to a fluorescent lamp, with a wavelength of 253.7 nm. Low pressure lamps are most effective, because they emit most of the radiant energy in the germicidal wavelength of 253.7 nm also known as the UV-C part of the spectrum. This is why low pressure lamps are mostly used in germicidal UV applications. The most common form of germicidal lamp looks similar to an ordinary fluorescent lamp but the tube contains no fluorescent phosphor. In addition, rather than being made of ordinary borosilicate glass, the tube is made of fused quartz. These two changes combine to allow the 253.7 nm UV light produced by the mercury arc to pass out of the lamp unmodified (whereas, in common fluorescent lamps, it causes the phosphor to fluoresce, producing visible light). Germicidal lamps still produce a small amount of visible light due to other mercury radiation bands. In some embodiments, a low pressure UV lamp looks like an incandescent lamp but with the envelope containing a few droplets of mercury. In this design, the incandescent filament heats the mercury, producing a vapor which eventually allows an arc to be struck, short circuiting the incandescent filament. Some low pressure lamps are shown in FIG. 27. Each of those low pressure UV lamp can be used in the present invention.

Preferred low pressure UV lamps for use in a portable UV device are low pressure mercury amalgam bulb supplied by, *e.g.,* Z-E-D Ziegler Electronic Devices GmbH, D 98704 Langewiesen, Germany ("Z-E-D") and Heraeus Noblelight Fusion UV Inc. 910 Clopper Road Gaithersburg, Maryland, 20878 USA.

Various low pressure UV lamps may be used in the UV devices, systems and methods described herein. Preferred are UV lamps from Z-E-D. Two of those are particularly preferred. Both have the same external dimensions of 1500 mm length and 32 mm diameter. The lamp current for the less powerful bulb is 5.0A, the lamp power is 550W with a 170 W (at 253.7nm) UVC output, 136W UVC (at 253.7nm) output when coated with Teflon. The life is 16,000 hours with a 15% loss at 253.7 nm after 12,000 hours. The second more powerful lamp draws a 6.5A current and has a total output of 700W and 200 W UVC (at 253.7nm). The life is 15,000 hours with a 15% loss at 253.7 nm after 12,000 hours. Both are low pressure mercury amalgam bulbs.

In some embodiments of the present invention, a germicidal UV light source is a medium-pressure UV lamp. Medium-pressure UV lamps are much more similar to high-intensity discharge (HID) lamps than fluorescent lamps. Medium-pressure UV lamps radiate a broad-band UV-C radiation, rather than a single line. They are widely used in industrial water treatment, because they are very intense radiation sources. They are as efficient as low-pressure lamps. A medium-pressure lamps typically produces very bright bluish white light. In some embodiments of the present invention, a germicidal UV light source is a high pressure UV lamp.

Preferred medium pressure UV lamps for use in a portable UV device are medium pressure mercury arc lamps provided by, e.g., Baldwin UV limited, 552 Fairlie Road, Trading Estate, Bershire, SL1 4PY, England.

In some embodiments of the present invention, a UV light source comprises a lamp selected from the group consisting of a low pressure mercury lamp, a medium pressure mercury lamp, a high pressure mercury lamp, an ultra-high pressure mercury lamp, a low pressure short arc xenon lamp, a medium pressure short arc xenon lamp, a high pressure short arc xenon lamp, an ultra-high pressure short arc xenon lamp, a low pressure long arc xenon lamp, a medium pressure long arc xenon lamp, a high pressure long arc xenon lamp, an ultra-high pressure long arc xenon lamp, a low pressure metal halide lamp, a medium pressure metal halide lamp, a high pressure metal halide lamp, an ultra-high pressure metal halide lamp, a tungsten halogen lamp, a quartz halogen lamp, a quartz iodine lamp, a sodium lamp, and an incandescent lamp.

Notably, any number of UV lamps including low pressure, medium pressure, high pressure, and ultra-high pressure lamps, which are made of various materials, e.g., most commonly mercury (Hg) can be used with the system configuration according to the present invention and in the methods described herein.

In some embodiments, a UV light source of the present invention comprises a low pressure mercury lamp. In some embodiments, a UV light source of the present invention comprises a medium pressure mercury lamp. In some embodiments, a UV light source of the present invention comprises a high pressure mercury lamp. In some embodiments, a UV light source of the present invention comprises an ultra-high pressure mercury lamp. Such mercury lamps are known in the art and are commercially available, e.g., Steril Aire Model SE series UVC Emitters™.

In some embodiments, a UV light source of the present invention comprises a low pressure short arc xenon lamp. In some embodiments, a UV light source of the present invention comprises a medium pressure short arc xenon lamp. In some embodiments, a UV light source of the present invention comprises a high pressure short arc xenon lamp. In some embodiments, a UV light source of the present invention comprises an ultra-high pressure short arc xenon lamp. Short arc xenon lamps are known in the art and are commercially available, *e.g*., Ushio #5000371-UXL-75XE Xenon Short Arc Lamp.

In some embodiments, a UV light source of the present invention comprises a low pressure long arc xenon lamp. In some embodiments, a UV light source of the present invention comprises a medium pressure long arc xenon lamp. In some embodiments, a UV light source of the present invention comprises a high pressure long arc xenon lamp. In some embodiments, a UV light source of the present invention comprises an ultra-high pressure long arc xenon lamp. Long arc xenon lamps are known in the art and are commercially available, *e.g*., Lumi-Max XLA1500W Long Arc Xenon Lamp.

In some embodiments, a UV light source of the present invention comprises a low pressure metal halide lamp. In some embodiments, a UV light source of the present invention comprises a medium pressure metal halide lamp. In some embodiments, a UV light source of the present invention comprises a high pressure metal halide lamp. In some embodiments, a UV light source of the present invention comprises an ultra-high pressure metal halide lamp. Metal halide lamps are known in the art and are commercially available, e.g., Venture Lighting product number 32519, open rated 175 watt probe start lamp.

### 4. Dimensions Of Germicidal UV Light Sources

Different sized and shaped UV light sources may be used to practice a method of the present invention, largely depending on the size and shape of the container, room, space or defined environment and the desired duration of the sterilization cycle. In some embodiments, a longer and more powerful UV lamp will provide for shorter duration cycles.

In some embodiments of the present invention, the UV light source is a UV-C lamp of 64" in length with an output of 190 microwatts/cm² at 254 nm (American Air and Water®, Hilton Head Island, SC 29926, USA). Such exemplary UV light source can be used in UV device Model UVT-4. In some embodiments of the present invention, the UV light source is a UV-C lamp of 36" in length with an output of 120 microwatts/cm² at 254 nm (American Air and Water®, Hilton Head Island, SC 29926, USA). Such exemplary UV light source can be used in UV device Model UV6K In some embodiments of the present invention, the UV light source is a UV-C lamp of 48" in length with an output of 250 microwatts/cm² at 254 nm (American Air and Water®, Hilton Head Island, SC 29926, USA). Such exemplary UV light source can be used in UV device Model UV2K Other useful UV-C lamps for use in the systems and methods of the present invention are shown in FIG. 27.

In some embodiments of the present invention, a germicidal UV lamp is a hot cathode germicidal UV lamp, examples of which are shown in FIG. 27.

In some embodiments of the present invention, a germicidal UV lamp is a slimline germicidal UV lamp, examples of which are shown in FIG. 27.

In some embodiments of the present invention, a germicidal UV lamp is a high output germicidal UV lamp, examples of which are shown in FIG. 27.

In some embodiments of the present invention, a germicidal UV lamp is a cold cathode germicidal UV lamp, examples of which are shown in FIG. 27.

In some embodiments of the present invention, a germicidal UV lamp is an 18" single ended low pressure mercury lamp, *e.g*., as made commercially available by Steril-Aire.

### 5. Power Output And UV Intensity Of Germicidal UV Light Sources

UV disinfection is a photochemical process. The effectiveness of UV-C is directly related to intensity and/or exposure time. Environmental factors, such as, air flow, humidity, airborne mechanical particles and distance of microorganism to the UV light source can also affect the performance of a UV device. While those environmental factors when present make it somewhat difficult to calculate the effective UV dosage required to kill or to inhibit the growth of a microorganism of interest, it has been shown that UV light will kill or inhibit the growth of any microorganism given enough UV dosage. The terms "UV dosage," "UV dose," and "UV intensity" are used interchangeably herein. As one of ordinary skill in the art will appreciate, the UV dosage, UV dose, or UV intensity calculated herein by using an algorithm (see below for details) is different than applying a mere exposure time as it is used by prior art UV devices.

For UV disinfection and sterilization, the microorganisms present on a surface in a container or on a surface of a room, space or defined environment are exposed to a lethal dose of UV energy. UV dose is measured as the product of UV light intensity times the exposure time within the UV lamp array. The microorganisms are exposed to a determined UV intensity/UV dosage, i.e., for a sufficient period of time to a germicidal UV light source, in order for the UV rays to penetrate the cellular membrane and breaking down the microorganisms' genetic material. (for details, see Algorithms, below).

### B. Algorithms

UV devices of the present invention use an algorithm for determining a sterilization cycle. More specifically, UV devices of the present invention use an algorithm for determining a UV intensity (UV dosage) necessary to be applied during a sterilization cycle seeking to reduce the growth rate of a microorganism present on an interior surface within a container, or on an interior surface within a room, space or defined environment. Multiple variables are taken into account when determining a required UV intensity/UV dosage. For any given UV light source, the calculated UV intensity/UV dosage then translates into a time the respective UV light source must remain activated to achieve the desired growth inhibition rate (also referred to herein as kill rate) for a given microorganism.

Calculating the UV intensity/UV dosage (and, hence, time) that is required to ensure meeting the sanitization objective takes into account multiple variables. Variables affecting the required UV dose for effective sanitization include: (i) type or species of microorganisms present on the surface of the container, room, space or defined environment to be sterilized, (ii) size of the container, room, space or defined environment, (iii) shape of the container, room, space or defined environment, (iv) the material of the surface of the container, room, space or defined environment to be sterilized, (v) extinction depth of the UV light source(s) at 254 nm, (vi) distance of the UV light source(s) from the surface of the container, room, space or defined environment to be sterilized, (vii) distribution and positioning of the UV light source(s), *e.g*., angular orientation of UV light sources in embodiments wherein a UV device comprises more than one UV light source, (viii) configuration of the UV light source(s), and (ix) intensity of the UV light source(s). A systematic approach to calculating a required UV intensity/UV dosage takes into account these variables.

In some embodiments of a UV device of the present invention, an algorithm determines a sterilization cycle based on at least two parameters selected from the group consisting of: (i) type or species of microorganisms present on the surface of the container, room, space or defined environment to be sterilized, (ii) size of the container, room, space or defined environment, (iii) shape of the container, room, space or defined environment, (iv) the material of the surface of the container, room, space or defined environment to be sterilized, (v) extinction depth of the UV light source(s) at 254 nm, (vi) distance of the UV light source(s) from the surface of the container, room, space or defined environment to be sterilized, (vii) distribution and positioning of the UV light source(s), *e.g*., angular orientation of UV light sources in embodiments wherein a UV device comprises more than one UV light source, (viii) configuration of the UV light source(s), and (ix) intensity of the UV light source(s).

In some embodiments of a UV device of the present invention, an algorithm determines a sterilization cycle based on at least three parameters selected from the group consisting of (i) through (ix). In some embodiments of a UV device of the present invention, an algorithm determines a sterilization cycle based on at least four parameters selected from the group consisting of (i) through (ix). In some embodiments of a UV device of the present invention, an algorithm determines a sterilization cycle based on at least five parameters selected from the group consisting of (i) through (ix). In some embodiments of a UV device of the present invention, an algorithm determines a sterilization cycle based on at least six parameters selected from the group consisting of (i) through (ix). In some embodiments of a UV device of the present invention, an algorithm determines a sterilization cycle based on at least eight parameters selected from the group consisting of (i) through (ix). In some embodiments of a UV device of the present invention, an algorithm determines a sterilization cycle based on parameters (i) through (ix).

The following describes in more detail individual sequences of steps leading to the calculation of a UV intensity required to sterilize an interior surface of a container, room, space or defined environment to a desired level. While some of the steps below are described as first, second, final, etc., one of ordinary skill in the art will appreciate that the sequences of steps can also be performed in a different order.

In some embodiments of a UV device of the present invention, the UV device uses an algorithm wherein the algorithm comprises the step of dividing radiant energy, i.e., the required energy dose of UV radiation (in µW/cm²) needed to kill a species of microorganism by an intensity factor, wherein the intensity factor takes into consideration the distance of the UV light source from the surface of the container, room, space or defined environment to be sterilized. Such algorithm is detailed, *e.g*., in Example 1.

In some embodiments of a UV device of the present invention, the UV device uses an algorithm for determining a sterilization cycle, wherein the algorithm is based on simulating an optical property of the UV light source and uses ray tracing to determine a plurality of UV intensities at a plurality of angular orientations relative to the UV light source. Such algorithm is particularly useful in UV devices comprising more than one UV light sources. It is further particularly useful in UV devices comprising more than one UV light source wherein the UV light sources are configured in an angular position with respect to each other. Details are described below.

A first step in determining a required UV intensity is verifying the type of microorganism(s) that is present in the container, room, space or defined environment to be sterilized. The identity of such microorganism(s) may be known from available literature or in some circumstances, may require a separate study.

Once the species of microorganism is known, the UV light dose that is required to obtain a specific or desired kill rate is determined. The UV dose, expressed in energy per area units, for example µW/cm², can be obtained from available literature, or, in some circumstances, may also require targeted experiments and studies. Such UV doses, *e.g*., are shown herein in the following Tables 1-5, providing approximate required UV intensities to kill or growth inhibit ("Kill Factor") a certain percentage of microorganisms, referred to as Log₁₀ reduction (American Water & Air® Inc., Hilton Head Island, SC 29926, USA).

**Table 1 provides the approximate required UV intensities to kill or growth inhibit ("Kill Factor") either 90% or 99% of mold spores (American Water & Air® Inc., Hilton Head Island, SC 29926, USA):**

| Mold Spores | Energy Dosage of UV Radiation (UV Dose) | |
|---|---|---|
| | in µWs/cm² Needed for Kill Factor | |
| | 90% (1 log Reduction) | 99%* (2 log Reduction) |
| *Aspergillius flavus* | 60,000 | 99,000 |
| *Aspergillius glaucus* | 44,000 | 88,000 |
| *Aspergillius niger* | 132,000 | 330,000 |
| *Mucor racemosus A* | 17,000 | 35,200 |
| *Mucor racemosus B* | 17,000 | 35,200 |
| *Oospora lactis* | 5,000 | 11,000 |
| *Penicillium expansum* | 13,000 | 22,000 |
| *Penicillium roqueforti* | 13,000 | 26,400 |
| *Penicillium digitatum* | 44,000 | 88,000 |
| *Rhisopus nigricans* | 111,000 | 220,000 |

| | | |
|---|---|---|
| *, it is noted that American Ultraviolet Company (Lebanon, IN, USA) states that the energy dosage of UV radiation (UV Dose) shown above to kill 99% of the indicated mold spores, is sufficient to achieve a 100% kill factor of the indicated mold spores. | | |

**Table 2 provides the approximate required UV intensities to kill or growth inhibit ("Kill Factor") either 90% or 99% of bacteria (American Water & Air® Inc., Hilton Head Island, SC 29926, USA):**

| Bacteria | Energy Dosage of UV Radiation (UV Dose) | |
|---|---|---|
| | in µWs/cm² Needed for Kill Factor | |
| | 90% (1 log Reduction) | 99%* (2 log Reduction) |
| *Bacillus anthracis* - Anthrax | 4,520 | 8,700 |
| *Bacillus anthracis* spores - Anthrax spores | 24,320 | 46,200 |
| *Bacillus magaterium sp.* (spores) | 2,730 | 5,200 |
| *Bacillus magaterium sp.* (veg.) | 1,300 | 2,500 |
| *Bacillus paratyphusus* | 3,200 | 6,100 |
| *Bacillus subtilis* spores | 11,600 | 22,000 |
| *Bacillus subtilis* | 5,800 | 11,000 |
| *Clostridium tetani* | 13,000 | 22,000 |
| *Corynebacterium diphtheriae* | 3,370 | 6,510 |
| *Ebertelia typhosa* | 2,140 | 4,100 |
| *Escherichia coli* | 3,000 | 6,600 |
| *Leptospiracanicola* - infectious Jaundice | 3,150 | 6,000 |
| *Microccocus candidus* | 6,050 | 12,300 |
| *Microccocus sphaeroides* | 1,000 | 15,400 |
| *Mycobacterium tuberculosis* | 6,200 | 10,000 |
| *Neisseria catarrhalis* | 4,400 | 8,500 |
| *Phytomonas tumefaciens* | 4,400 | 8,000 |
| *Proteus vulgaris* | 3,000 | 6,600 |
| *Pseudomonas aeruginosa* | 5,500 | 10,500 |
| *Pseudomonas fluorescens* | 3,500 | 6,600 |
| *Salmonella enteritidis* | 4,000 | 7,600 |
| *Salmonela paratyphi* - Enteric fever | 3,200 | 6,100 |
| *Salmonella typhosa* - Typhoid fever | 2,150 | 4,100 |
| *Salmonella typhimurium* | 8,000 | 15,200 |
| *Sarcina lutea* | 19,700 | 26,400 |
| *Serratia marcescens* | 2,420 | 6,160 |
| *Shigella dyseteriae* - Dysentery | 2,200 | 4,200 |
| *Shigella flexneri* - Dysentery | 1,700 | 3,400 |
| *Shigella paradysenteriae* | 1,680 | 3,400 |
| *Spirillum rubrum* | 4,400 | 6,160 |
| *Staphylococcus albus* | 1,840 | 5,720 |
| *Staphylococcus aerius* | 2,600 | 6,600 |
| *Staphylococcus hemolyticus* | 2,160 | 5,500 |
| *Staphylococcus lactis* | 6,150 | 8,800 |
| *Streptococcus viridans* | 2,000 | 3,800 |
| *Vibrio comma* - Cholera | 3,375 | 6,500 |

| | | |
|---|---|---|
| *, it is noted that American Ultraviolet Company (Lebanon, IN, USA) states that the energy dosage of UV radiation (UV Dose) shown above to kill 99% of the indicated microorganisms, is sufficient to achieve a 100% kill factor of the indicated microorganism. | | |

**Table 3 provides the approximate required UV intensities to kill or growth inhibit ("Kill Factor") either 90% or 99% of protozoa (American Water & Air® Inc., Hilton Head Island, SC 29926, USA):**

| Protozoa | Energy Dosage of UV Radiation (UV Dose) | |
|---|---|---|
| | in µWs/cm² Needed for Kill Factor | |
| | 90% (1 log Reduction) | 99%* (2 log Reduction) |
| *Chlorella vulgaris* (Algae) | 13,000 | 22,000 |
| Nematode Eggs | 45,000 | 92,000 |
| Paramecium | 11,000 | 20,000 |

| | | |
|---|---|---|
| *, it is noted that American Ultraviolet Company (Lebanon, IN, USA) states that the energy dosage of UV radiation (UV Dose) shown above to kill 99% of the indicated protozoa, is sufficient to achieve a 100% kill factor of the indicated protozoa. | | |

**Table 4 provides the approximate required UV intensities to kill or growth inhibit ("Kill Factor") either 90% or 99% of viruses (American Water & Air® Inc., Hilton Head Island, SC 29926, USA):**

| Virus | Energy Dosage of UV Radiation (UV Dose) | |
|---|---|---|
| | in µWs/cm² Needed for Kill Factor | |
| | 90% (1 log Reduction) | 99%* (2 log Reduction) |
| Bacteriophage - *E. Coli* | 2,600 | 6,600 |
| Infectious Hepatitis | 5,800 | 8,000 |
| Influenza | 3,400 | 6,600 |
| Poliovirus - Poliomyelitis | 3,150 | 6,600 |
| Tobacco mosaic | 240,000 | 440,000 |

| | | |
|---|---|---|
| *, it is noted that American Ultraviolet Company (Lebanon, IN, USA) states that the energy dosage of UV radiation (UV Dose) shown above to kill 99% of the indicated viruses, is sufficient to achieve a 100% kill factor of the indicated viruses. | | |

**Table 5 provides the approximate required UV intensities to kill or growth inhibit ("Kill Factor") either 90% or 99% of yeast (American Water & Air® Inc., Hilton Head Island, SC 29926, USA):**

| Yeast | Energy Dosage of UV Radiation (UV Dose) | |
|---|---|---|
| | in µWs/cm² Needed for Kill Factor | |
| | 90% (1 log Reduction) | 99% (2 log Reduction) |
| Brewers yeast | 3,300 | 6,600 |
| Common yeast cake | 6,000 | 13,200 |
| *Saccharomyces carevisiae* | 6,000 | 13,200 |
| *Saccharomyces ellipsoideus* | 6,000 | 13,200 |
| *Saccharomyces* spores | 8,000 | 17,600 |

| | | |
|---|---|---|
| *, it is noted that American Ultraviolet Company (Lebanon, IN, USA) states that the energy dosage of UV radiation (UV Dose) shown above to kill 99% of the indicated yeast, is sufficient to achieve a 100% kill factor of the indicated yeast. | | |

Taking the UV light distribution in the to-be sterilized container, room, space or defined environment into consideration requires simulation of the optical properties of the UV light source. Such simulation takes into account properties of the UV light source such as the angle dependent light emittance, the geometrical shape of the light source, the material properties of the source and the number of emitters. Such information typically is provided by the vendor of such UV light sources. It is also desirable to include properties of the container, room, space or defined environment to be sterilized, such as shape and wall material, in particular when reflection of the walls is significant.

Containers of various reflective nature can be used in the methods of the present invention. As indicated in the following table, different wall materials reflect different percentages of UV light (254 nm). One of skill in the art will appreciate the contribution of the reflectance of a material will have for achieving a desired UV intensity useful for UV disinfection and sterilization (*see*, Table 6). The wall material and % reflectance is considered in the algorithm described herein when determining the required UV intensity/UV dosage growth inhibit a desired species of microorganisms.

**Table 6. Reflective Factors On Various Surfaces At 254 Nm Wavelength. The values are obtained at normal incidence. The percentage reflectances increases rapidly at angles greater than 75%. (American Ultraviolet Company , Lebanon, IN 46052, USA)**

| Material | % Reflectance |
|---|---|
| Aluminum, etched | 88 |
| Aluminum, foil | 73 |
| Aluminum, polished commercial | 73 |
| Chromium | 45 |
| Glass | 4 |
| Nickel | 38 |
| Silver | 22 |
| Stainless steel | 20-30 |
| Tri-plated steel | 28 |
| Water paints | 10-30 |
| White cotton | 30 |
| White oil paint | 5-10 |
| White paper | 25 |
| White porcelain | 5 |
| White wall plaster | 40-60 |

When practicing methods of the present invention and developing an algorithm for calculating UV dosages/UV intensities, the extinction depths of the UV light source at 254 nm wavelength in various liquids needs to be taken into consideration, unless the surface of the container to be sterilized is completely dry. The application of UV light to sterilize a surface following a pressure wash would have to take into account the extinction depth of UV light at 254 nm in the remaining tap water. However, the depth of tap water the UV light must penetrate is minimal and would be equivalent to that of a film of water or at most interspersed water droplets. In some instances, the effect of depth of tap water on the UV intensity/UV dosage required for determining the sterilization and kill rate will have to be tested using methods described herein and available in the art. This is due to the fact that following pressure washing of a container (*e*.*g*., a fermentation vessel), the remaining layer of water covering the container may not be homogeneous. Maximum depths of water drops can be used to calculate extra time needed for the sterilization cycle. Although the extinction coefficient could theoretically be used to calculate this, it would not take into account the reflection and scattering caused by uneven surfaces of the water film and water droplets, as such empirical data would be more useful for determining how to adjust sterilization timing. The following table provides guidance:

**Table 7. Extinction Depths at 254 nm Wavelength (relationship to clear water) (American Ultraviolet Company , Lebanon, IN 46052, USA)**

| Liquid | Extinction Depth |
|---|---|
| Apple juice | 1.0 |
| Beer | <1.3 |
| Liquid sugar | 1.0 |
| Milk - whole, raw | <0.1 |
| Vinegar | <5.0 |
| Water - concrete cistern | <75 |
| Water - distilled | 3,000 |
| Water - tap or mains | 125-180 |
| Wine | <2.5 |

Typically such simulation to arrive at a required UV intensity/UV dosage can be done with the help of software that performs non-sequential ray tracing, taking into account the variables cited above. As a non-limiting example, commercially available software that can be used includes OpticsStudio (available from Zemax, 10230 NE Points Dr. Suite 540, Kirkland, WA 98033 USA) or LightTools (available from Synopsis, 690 East Middlefield Road Mountain View, CA 94043).

The ray trace software uses the light source configuration and parameters to calculate the UV light irradiance specific spatial locations relative to the source. Using these calculation a multidimensional lookup table is created, wherein the calculated irradiance is listed with the table variables being distance from the source and angular orientation. Typically, due to symmetry, two angular orientations may suffice, one parallel and one perpendicular to the UV light source orientation. In some embodiments, when symmetry does not exist, more orientations may be needed.

The final calculation of the required illumination time uses the tabulated data to find the area of the space where the illumination is at a minimum, as that area determines the longest illumination time. This determination can be done by manual search of the table or by a dedicated search software routine.

Once the lowest irradiance level is determined, the required illumination time is calculated by dividing the required kill dose by the irradiance.

By way of example, using a germicidal UV lamp with 190 microwatts/cm² output at 254 nm, it would take approximately about 1 minute and 26 seconds to kill or growth inhibit ("Kill Factor") 100% of *Saccharomyces* sp. (which requires 17,600 microwatt/cm²) at a distance of 36" and 3 minutes 41 seconds at a distance of 60". *See also*, Examples 1 and 2.

In embodiments where a UV device comprises more than one UV light source, irradiance tables (*see, supra*) are calculated for each UV light source individually. Once calculated individually, they are added to arrive at a sum irradiance.

Using an algorithm described herein, Table 8, below, shows calculated times for UV sterilization of interior surfaces a container of a given volume (in gallons), diameter (in feet) and height (in feet) when a UV device of the UVT-4 family of UV devices (*see*, FIGS. 3-13) is being used. More specifically, the UV light sources employed were four (4) UV lamps of the type ZLA550/1500/4p-R comprising a protective Teflon sleeve (a UV light permissible housing) manufactured by LightSources (Article code: 200-00091; Connecticut, USA). Such bulb is approximately 1.5 m in length, i.e., about 59.05". Each bulb had a lamp power of 550 watts and a UV output of 148 watts at 253.7 nm. The UV intensity at 3m was 162 µW/cm². Without a Teflon sleeve, the UV output was 190 watts and UV intensity at 3m was 200 µW/cm². In this exemplary embodiment, the UV device UVT-4 UV device comprises four (4) UV light sources, of which, when in operation, two (2) are in a horizontal orientation and two (2) are in a vertical orientation (*e.g., see*, FIGS. 16, 17). When this UV device is used to sterilize interior surfaces of a large container (*e*.*g*., Table 8; 100,818 gallons, 23.8 feet diameter and 30.6 feet height), the UV device is placed on a surface in the middle of the container (*e.g., see*, FIG. 17). When this UV device is used to sterilize interior surfaces smaller containers (*e.g.*, see Table 8), the UV device is operatively attached to an opening of the container (*e.g., see*, FIG. 11).

**Table 8. UV sterilization times for UV device of UVT-4 family calculated using an algorithm described herein and based on container size and UV light source ZLA550/1500/4p-R.**

| **Container (Gallons)** | **Container (Diameter, feet)** | **Container (Height, feet)** | **Time (min)** |
|---|---|---|---|
| 2513 | 4.6 | 20.9 | 20 |
| 3425 | 5.8 | 16.2 | 12 |
| 5045 | 6.5 | 20.9 | 19 |
| 6372 | 8.8 | 14.9 | 9 |
| 6396 | 9.5 | 12.8 | 7 |
| 6658 | 9.8 | 12.6 | 7 |
| 6798 | 9.8 | 12.8 | 7 |
| 7061 | 9.8 | 13.2 | 7 |
| 7958 | 9.9 | 15.0 | 9 |
| 10307 | 8.5 | 24.8 | 25 |
| 10675 | 10.2 | 19.0 | 16 |
| 13892 | 11.7 | 18.6 | 14 |
| 15450 | 12.0 | 19.3 | 16 |
| 15610 | 11.8 | 19.0 | 16 |
| 18853 | 11.8 | 23.9 | 21 |
| 20481 | 12.0 | 24.8 | 25 |
| 23216 | 15.0 | 17.8 | 24 |
| 50809 | 17.0 | 30.9 | 37 |
| 51657 | 16.9 | 32.0 | 37 |
| 100818 | 23.8 | 30.6 | 139 |

As one of ordinary skill in the art will appreciate, the calculated UV intensity required to kill the species of microorganisms is converted in Table 8 into time, i.e., minutes of operation of the UV device, in other words, the time the UV device will be activated.

### C. Frames And Frame Assemblies

UV devices of the present invention comprise a frame to which a UV light source is operatively attached to. The structure of frame is not important as long as the UV light source after being operatively attached to the frame is functional and a sterilization cycle can be applied.

Exemplary frames, parts of frames and additional parts attached to such frames are shown in FIGS. 1-13, 15-19, 21, 22A-D, and 23-25.

Frames can be of a variety of material. Frames can be made of plastic, metal or wood. Preferred are metal frames. For making the UV devices more light weight, frames may have a plurality of openings. Also preferred are plastic frames.

### D. UV Detectors And Sensors

The present invention describes a variety of UV devices. In some embodiments of the present invention, a UV device comprises a detector or a sensor. The terms UV detector and UV sensor are used interchangeably herein. In the drawings, an exemplary UV sensor is indicated as **17** in FIGS. 3, 4 and 6-9. The use of a detector or sensor ensures that in addition to the algorithm (taking into account vessel size and shape, size and shape of a room, a space or defined environment, lamp intensity, distance of lamp or lamps from surfaces to be sterilized) a required or predetermined UV light intensity is achieved. Further, a detector ensures that all areas known to specifically accumulate microorganisms also receive the required or predetermined dose of UV radiation.

The use of a detector solves a significant problem existing using the chemical and ozone disinfection methods. When those methods are used, there is no established protocol for verifying the level of sterilization achieved. In contrast thereto, methods of the present invention comprising the use of a detector offers a unique, quick, and reliable means of providing verifiable levels of the sterilization achieved. Once set at a predetermined UV dose, the detector upon measuring that predetermined UV dose, will shut of the UV light source when this predetermined amount of UV radiation has been attained.

In some embodiments of the present invention, a UV light source is connected to one or more UV detectors or UV sensors. In some embodiments of the present invention, a germicidal light source is connected to one or more UV detectors or UV sensors. One or more UV detectors may be mounted to a different position within the UV device or may be placed elsewhere in the container, room, space or defined environment to be sterilized. In FIGS. 3, 4 and 6-9, a UV sensor **17** is exemplary attached to a second upper frame end **29** (see detailed description below).

UV devices described herein are adapted to use a variety of commercially available detectors and sensors. UV-C detectors commercially available include, *e.g.,* a PMA2122 germicidal UV detector (Solar Light Company, Inc., Glenside, PA 19038, USA). Detectors, such as the PMA2122 Germicidal UV detector, provide fast and accurate irradiance measurements of the effective germicidal radiation. Thus, in some embodiments of a UV device of the present invention, a UV detector is PMA2122 germicidal UV detector. Another preferred UV detector is Digital UV-sensor type with RS485 interface (Ziegler Electronic Devices GmbH, In den Folgen 7, D 98704 Langewiesen Germany). Thus, in some embodiments of a UV device of the present invention, a UV detector is Digital UV-sensor type with RS485 interface. A UV producing lamp is monitored to insure that the microorganisms, such as bacteria, are receiving a desired dose of germicidal UV radiation. Using a UV detector, the UV lamps can also be monitored to get maximum life out of the lamp before replacement. A germicidal UV detector can also be used to insure that the proper lamp has been installed after replacement.

In some embodiments of the present invention, a germicidal UV light source is operatively connected (e.g., electrically) to one or more UV detectors. In some embodiments, a UV detector is operatively connected by wire to a radiation meter, which in turn can communicate via the wire with a UV lamp and instruct it to turn it off, *e.g.*, when a desired radiation level (dosage, intensity) has been attained.

In some embodiments of the present invention, a germicidal light source is operatively connected to one or more UV detectors via a signal.

In some embodiments, a detector is placed at a location within a container where microorganisms, which negatively impact production and flavor of an alcoholic beverage, a dairy product, a liquid dairy, a liquid dairy composition, or a dry dairy composition, are known to accumulate. In some embodiments, a detector is placed within a room, space or defined environment.

In some embodiments of the present invention, the one or more UV detectors are placed in conjunction with a UV light source, preferably, a germicidal UV light source, so that the one or more detectors ensure that a desired UV intensity has been attained and/or maintained. In some embodiments, a detector is placed strategically in corners or on uneven surfaces of containers such as weld seams where microorganisms may accumulate.

In some embodiments, a detector is arranged so that it is both furthest away from the UV light source and closest to the most uneven interior surface of a container (*e.g.*, weld seam or a corner), a room, a space or defined environment. The purpose of the UV detector is to ensure that the required or predetermined UV dose is attained at a given interior location of a container, room, space or defined environment in order to achieve the desired log reduction of microorganisms. By placing a UV detector or more than one detector (i.e., at least two UV detectors) in one or more positions in the interior of the container or within a room, a space or defined environment to be sanitized, it will be ensured that the even surfaces and those closer to the UV light source will receive sufficient UV radiation (UV dose, UV intensity) to achieve the desired log reduction of microorganisms and that the more problematic interior surfaces of a container (*e.g.*, weld seams and corners) or uneven surfaces in a room, space or defined environment will receive the required or predetermined UV dose.

In some embodiments of the present invention, a UV light source communicates back and forth with a UV detector so that the UV light source is shut off when a desired specified germicidal level of UV radiation has been attained. As will be appreciated by one of skill in the art, a desired specified germicidal level (UV dose, UV intensity) is dependent on the log reduction or percentage reduction of microorganisms desired. If sterilization is required, a six log reduction in microorganisms may be specified. In the interest of saving time and electricity, however, a five log reduction or a four-log reduction may be desired. Once the desired UV intensity has been attained, the UV detector will cause the UV light source to shut off.

One of skill in the art using a UV detector in combination with a UV device to sterilize a container, a room, a space or defined environment according to a method of the present invention would not need to know the diameter of the container or dimension of a room, a space or defined environment as the detector would automatically detect the appropriate UV dose necessary to achieve a predetermined sterilization rate (log reduction value).

The use of a UV detector, however, is optional. Detectors are not required to practice methods of the present invention provided that the timing of the sterilization cycle has been calculated correctly. UV detectors can be used as a redundant system if the shape of the container, room, space or defined environment and/or UV lamp does allow the skilled artisan to apply a simple programmable calculation of the sterilization cycle duration.

### E. Housings

In some embodiments of the present invention, a UV device comprises a housing **15**. Various housings for UV light sources **16** are indicated in the exemplary UV devices in FIGS. 4-13, 15-19, 21, 22B, 22C, 22D, and 23-25 by **15**. In some embodiments of the present invention, a germicidal UV light source **16** resides in a housing **15**. In FIGS. 4-13, 15-19, 21, 22B, 22C, 22D, and 23-25, such arrangements is indicated by "**15,16**." In some embodiments of the present invention, a germicidal UV light source is positioned within a housing **15**. In some embodiments of the present invention, a housing **15** surrounds or encloses a germicidal UV light source. Exemplary surroundings or enclosures of a UV light source by a housing are shown in FIGS. 13, 15-19, 21, 22B, 22C, 22D, and 23-25. The surrounding or enclosure may be complete or partial. Exemplary complete surroundings or enclosures of a UV light source by a housing **15** are shown in FIGS. 13, 15-19, 21, 22B, 22C, 22D, and 23-25. Housings **15** are designed to protect the UV light source from damage, *e.g.*, during transport, during use, or when the UV light source is retracted from a container, a room, a space, or a defined environment according to a method of the present invention. A UV light source can be directly or indirectly attached to a housing **15** or alternatively, resides within a housing **15**. Housings **15**, however, are not necessary for a UV device of the present invention to function. They are optional.

A housing **15** can be made of a variety of materials. It can be made from a polymer (*e.g.*, plastic) or metal depending on the desired weight. In some embodiments, a housing is made of DuPont Teflon®FEP (Fluorinated Ethylene Propylene).

A housing **15** can have various shapes and forms. In some embodiments of the present invention, a housing is a mesh cage allowing the UV light to pass through. A housing **15** may comprises one or more circular structures, such as metal rings. When using housings **15** that allow passing through of the UV light, the UV light source does not need to be released from the housing **15** to practice a method of the invention. Preferably, a UV light source **16** is fully enclosed by a housing **15**, which allows UV light to pass through.

In some embodiments of the present invention, a housing **15** is a housing **15** which does not allow the UV light to pass through or which only allows the UV light to pass through partially. When using such a housing in the methods of the present invention, the UV light source is being released from the housing **15**. Upon release of the germicidal UV light source from the housing **15**, the germicidal UV light source may be stationary or mobile. The housing can be of any shape. The shape of the housing is largely depending on the size and shape of the UV light source or of the size and shape of a UV lamp cluster.

In some embodiments, a single longitudinal UV lamp is used as a UV light source. In those embodiments, the housing may surround or enclose the UV lamp either completely or partially. In some embodiments, a housing **15** comprises two arms, a first arm and a second arm. The first arm may be positioned in a fixed position, while the second arm may be movably attached to the first arm. In some embodiments the second arm can reside completely or partially within the first arm. The movable attachment of the second arm to the first arm may be through a pivot point. In some embodiments, a UV light source is operatively attached to such housing through an opening in the second arm and further connected to a part of the UV device through a rope, string, or a power cord. In a configuration wherein the second arm resides within the first arm, the UV lamp would then reside within the second arm. In some embodiments, a rope, a string or a power cord prevents the second arm of the housing from moving downwardly. Upon lowering the rope, the string, or the power cord, the UV light source along with the second arm will be released from the first arm of the housing. The rope, the string or the power cord can be lowered to a point whereupon the second and first arm form a 90 degree. Thereby the UV light source can be moved into almost any position within the confines of a container, room, space, or defined environment. As one of ordinary skill in the art will appreciate, such positioning depends on the length of the first arm, the length of the second arm and the angle formed between the first and second arm. Provided herein are various lengths of the first and second arms, depending, as one of ordinary skill in the art will appreciate, on the diameter and height of a container, a room, a space or defined environment to be sterilized. For example, if the diameter of a container is about 5 meters, then a second arm having a length of about 2.5 meters could position a UV device approximately in the middle of the container when the UV device is attached to an outer part on top of the container. The height positioning of a UV light source within a container can then be controlled conveniently by the extent to which the rope, string or power cord is further lowered. Lowering of the UV light source into a container, room, space or defined environment can be achieved as described herein by use of a motorized unit.

### F. Guides, Range-Finding Devices, And Circuit Boards

In some embodiments of the present invention, a UV device or system comprises a range-finding device or guide, such as a laser range finder. A range-finding device may be placed or aligned at some point along the longitudinal axis of the UV device in order to prevent the UV light source(s) or UV device from contacting either the top or bottom surface of the container (depending on the embodiment the device may be suspended from the top of the container or supported from below by a mount). If the embodiment uses lateral movement to position the UV light source(s) closer to the internal surface the container or to a predetermined position in a room, a space or defined environment, the range finder may be aligned in the same orientation ensuring that the UV light source(s) is positioned at the desired distance depending on the internal diameter of the container or dimension of the room, space or defined environment. In some embodiments of the present invention, a range-finding device is used in conjunction with a system to guarantee that the UV light source(s) is in correct distance from the interior surface of a container to be sterilized or the surface, walls or ceilings of a room, a space or defined environment to be sterilized as well as preventing the UV lamp from impacting the interior surfaces of the container, room, space or defined environment.

In some embodiments of the present invention, a range-finding device is a radiofrequency identifier (RFID), which is used to position a UV light source at a desired or predetermined position within a container, room, space or defined environment. An RFID receives information about the dimensions of the container, room, space or defined environment to be sterilized, such as depth, width, or radius. An RFID may be attached to a UV device of the present invention. In some embodiments, an RFID is attached to the container, room, space or defined environment to be sterilized.

FIGS. 28A-D and 29A-D schematically depict exemplary circuit boards for use in a UV device of the present invention. Circuit boards can be placed within a circuit board cavity within a frame. A circuit board may also be enclosed in a control box **1** (see below).

A circuit board for use in a UV device of the present invention may have a variety of functionalities. Various exemplary circuit boards are described herein, *e.g.,* in FIGS. 20A-C, 28A-D and 29A-D. UV devices of the present invention comprise a circuit board comprising at least three functionalities selected from the group consisting of (A) comprising a radiofrequency identifier reader, (B) communicating with a radiofrequency identifier, (C) controlling a movement of the first germicidal UV light source within the container, room, space, or defined environment, (D) controlling a rate of descent or ascent of the first germicidal UV light source within the container, room, space, or defined environment, (E) controlling a positioning of first germicidal UV light source within the container, room, space, or defined environment, (F) controlling an on/off status of a motor, wherein the motor controls the positioning of the first germicidal UV light source within the container, room, space, or defined environment, (G) controlling an on/off status of the first germicidal UV light source based on measuring whether a pre-determined UV intensity has been attained, (H) controlling extension of the first germicidal UV light source from a housing, (I) controlling retraction of the first germicidal UV light source into the housing, (J) responding to a jammed position status of the first germicidal UV light source, (K) controlling an optical sensor which initiates a timer upon placing the first germicidal UV source into the container, room space, or defined environment, (L) controlling a speaker emitting an audible signal at the beginning or completion of the sterilization cycle, (M) controlling a plurality of LED lights indicting a status of the sterilization cycle, (N) relaying UV light intensity via a UV sensor to the container, room, space, or defined environment, (O) uploading and relaying information from the radiofrequency identifier, (P) generating a report on time of the sterilization cycle, (Q) generating a report on duration of the sterilization cycle, (R) generating a report on UV light intensity attained during the sterilization cycle, (S) emailing, phoning or texting the report on time of the sterilization cycle, (T) emailing, phoning or texting the report on duration of the sterilization cycle, (U) emailing, phoning or texting the report on UV light intensity attained during the sterilization cycle, (V) emailing, phoning or texting an alert to an individual that the sterilization cycle is initiated, interrupted or complete, (W) emailing, phoning or texting an alert that a UV light source requires replacement, (X) logging date, time or individual who used the UV device, and (Y) logging information of the container, room, space, or defined environment in which the UV device will be or has been used.

In some embodiments, the circuit board comprises at least four functionalities selected from (A) through (Y). In some embodiments, the circuit board comprises at least five functionalities selected from (A) through (Y). In some embodiments, the circuit board comprises at least six functionalities selected from (A) through (Y). In some embodiments, the circuit board comprises at least seven functionalities selected from (A) through (Y). In some embodiments, the circuit board comprises at least eight functionalities selected from (A) through (Y). In some embodiments, the circuit board comprises at least nine functionalities selected from (A) through (Y). In some embodiments, the circuit board comprises at least ten functionalities selected from (A) through (Y).

As one of ordinary skill in the art will appreciate various combinations of functionalities (A) through (Y) may be included in a circuit board. Non-limiting examples of combinations of at least three functionalities selected from (A) through (Y) include, functionalities (A), (B), and (C); functionalities (D), (E), and (G); functionalities (K), (L), and (O); functionalities (A), (B), and (C); functionalities (A), (B), (F) and (G); functionalities (B), (K), (L) and (T); functionalities (A), (B), (H), (K) and (Y); functionalities (A), (B), and (C); functionalities (L), (M), (P), (Q), and (X).

In some embodiments of the present invention, the functionality of the circuit board is comprising a radiofrequency identifier.

In some embodiments of the present invention, the functionality of the circuit board is communicating with a radiofrequency identifier.

In some embodiments of the present invention, the functionality of the circuit board is controlling a movement of a germicidal UV light source within a container, room, space, or defined environment.

In some embodiments of the present invention, the functionality of a circuit board is controlling a rate of descent of a germicidal UV light source within a container, room, space or defined environment.

In some embodiments of the present invention, the functionality of a circuit board is controlling a rate of ascent of a germicidal UV light source within a container, room, space or defined environment.

In some embodiments of the present invention, the functionality of a circuit board is controlling a positioning of a germicidal UV light source within a container, room, space or defined environment.

In some embodiments of the present invention, the functionality of the circuit board is controlling an on/off status of a motor, wherein the motor controls the positioning of a germicidal UV light source within a container, room, space, or defined environment.

In some embodiments of the present invention, the functionality of a circuit board is controlling an on/off status of a germicidal UV light source based on measuring whether a pre-determined UV intensity has been attained.

In some embodiments of the present invention, the functionality of the circuit board is controlling extension of a germicidal UV light source from a housing.

In some embodiments of the present invention, the functionality of the circuit board is controlling retraction of a germicidal UV light source into a housing.

In some embodiments of the present invention, the functionality of the circuit board is responding to a jammed position status of a germicidal UV light source.

In some embodiments of the present invention, the functionality of the circuit board is controlling an optical sensor which initiates a timer upon placing the first germicidal UV source into a container, room space, or defined environment.

In some embodiments of the present invention, the functionality of the circuit board is controlling a speaker emitting an audible signal at the beginning or completion of a sterilization cycle.

In some embodiments of the present invention, the functionality of the circuit board is controlling a plurality of LED lights indicting a status of the sterilization cycle.

In some embodiments of the present invention, the functionality of a circuit board is relaying UV light intensity via a UV sensor to a container, room, space or defined environment.

In some embodiments of the present invention, the functionality of a circuit board is uploading and relaying information from a radiofrequency identifier.

In some embodiments of the present invention, the functionality of a circuit board is generating a report on time of a sanitization cycle.

In some embodiments of the present invention, the functionality of a circuit board is generating a report on duration of a sanitization cycle.

In some embodiments of the present invention, the functionality of a circuit board is generating a report on UV light intensity attained during a sanitization cycle.

In some embodiments of the present invention, the functionality of a circuit board is relaying a message to an individual. A message relayed by a circuit board of a UV device of the present invention may be an email notification, an automated telephone voice mail message or a special message system to a hand held device such as a cell phone or tablet type device. The individual can receive an email notification that documents or reports generated are available to view and download online.

In some embodiments of the present invention, the functionality of a circuit board is emailing, phoning or texting a report on time of a sanitization cycle.

In some embodiments of the present invention, the functionality of a circuit board is emailing, phoning or texting a report on duration of a sanitization cycle.

In some embodiments of the present invention, the functionality of a circuit board is emailing, phoning or texting a report on UV light intensity attained during a sanitization cycle.

In some embodiments of the present invention, the functionality of a circuit board is emailing, phoning or texting an alert to an individual that a sanitization cycle is in progress, interrupted or complete.

In some embodiments of the present invention, the functionality of a circuit board is emailing, phoning or texting an alert that a UV light source requires replacement.

In some embodiments of the present invention, the functionality of a circuit board is logging date, time and individual who used the portable UV device.

In some embodiments of the present invention, the functionality of a circuit board is logging information of a container, a room, or a defined environment in which the portable UV device will be and/or has been used.

In some embodiments of the present invention, the functionality of a circuit board is relaying UV intensity via a sensor to a container, a room, a defined environment to ensure that a desired or predetermined irradiation is achieved during a specified time or duration.

While certain functionalities of a circuit board, *e.g.,* (A) through (Y) as described herein, can be used for a variety of UV devices, some functionalities, as one of skill in the art will appreciate, are used for a specifically configured UV device. For example, UV device UVT-4 comprises a lower frame and an upper frame. In some embodiments of the present invention, the functionality of a circuit board is controlling the rate of moving an upper frame and UV light source(s) attached thereto from a horizontal position to an angular position with respect to a lower frame and attached UV light source(s) of a UV device. In some embodiments of the present invention, the functionality of a circuit board is controlling the rate of moving an upper frame and UV light source(s) attached thereto from a perpendicular/vertical or angular position to a horizontal position with respect to a lower frame and attached UV light source(s) of a UV device. In some embodiments of the present invention, the functionality of a circuit board is controlling the rate of moving an upper frame and UV light source(s) attached thereto from a first angular position to a second angular position with respect to a lower frame and attached UV light source(s) of a UV device.

Other useful functionalities that can be combined with either of the functionalities described above, include, but are not limited to: adjusting a current being sent to a UV light source to maximize efficiency of a sanitization cycle, controlling a servo or a motor and/or the rate with which a servo or motor operate, connecting to one or more fuses to protect the UV device against electrical surges, connecting to Zcon mini which measures incoming UVC in real time from a UV-C sensor, connecting a Zcon mini to a programmable logic control (PLC) unit, or using a PLC unit to connect with a touchscreen interface **5** located on an outside of a control box **1**, interfacing with a PLC unit to indicate whether a bulb intensity is sufficient or inefficient for a desired sanitization cycle. PLC unit may have sanitization cycle times programmed into it.

### G. Means For Attaching A UV Device To A Container, A Room, Space Or Defined Environment

The UV devices described herein can be used to practice the methods described herein. A UV device of the present invention can be operatively attached - movably, adjustably, temporarily, or permanently - to a container, to a surface of an object, to a floor, to a ceiling or to a wall of a room, space or defined environment by using a means for attaching. Such means for attaching include, but are not limited to a fastener, a screw, amounting tab, a bracket, a hanger, and the like.

In some embodiments of the present invention, a UV device is positioned on top of a container **49**, as *e.g.*, schematically depicted in FIGS. 23, and 25. In some embodiments of the present invention, a UV device is positioned on the bottom of a container **49**, as *e*.*g*., schematically depicted in FIG. 17. In some embodiments of the present invention, a UV device is attached to an opening in a side wall of a container **49**, as *e.g.*, schematically depicted in FIG. 11.

The UV devices described herein can be attached temporarily to a container, a room, space, or defined environment, *e.g.*, for the time required to perform a method described herein. The UV devices described herein can also be attached to a container, a room, space or defined environment for a prolonged time, *e.g.*, for the time required to perform a method described herein and an extended period of time before or after practicing the method. The UV devices described herein can also be configured to be attached permanently to a container, a room, space, or defined environment.

The means for attaching the UV device to a container, a room, space or defined environment essentially serves to operatively attach the UV device on or at an outer perimeter of an opening of the container, to a fixture within the room or defined environment so that the UV light source and other parts of the UV device necessary to perform a method of the present invention can be movably inserted through the opening of the container into the interior part of the container and into the room, space or defined environment.

In some embodiments of the present invention, the means for attaching the UV device to a container is a bracket, also referred to as mounting bracket. Non-limiting exemplary bracket embodiments **21** are depicted in the exemplary UV devices shown in FIGS. 4, 11, 12, 15, 16, 23, 25, and 26. A bracket **21** may comprise one or more of the following: a bracket tightening knob **26** and a plurality of rope or line posts **27**, **28**. A preferred configuration of those parts is shown in FIGS. 4, 11, 15, and 26. A bracket can have any shape or size as long as it is configured to operatively attach a UV device to a container, a room, space or defined environment to be sterilized.

Other parts of an exemplary bracket shown in FIG. 26, include an extension of the mounting bracket **85**, a main support channel **94** for guiding rope/cable **13** of a UV device. In some embodiments of the present invention, a bracket, the bracket is an adjustable bracket in which the extension **85** can be extended or tilted so that the UV device can be positioned at any desired position within a container, a room, space or defined environment, *e.g.*, in the center of the container, the room, space or defined environment or non-centrically and closer to a preferred wall of the container, the room, space or defined environment.

### H. Optical Components

To increase the UV intensity over a reduced area, to focus the UV intensity, or to control the UV intensity, in some embodiments of the present invention, a UV device of the present invention comprises an optical component. Optical components include, but are not limited to, a reflector, a shutter, a lens, a splitter, a mirror, and the like. The optical component may be of any shape.

In some embodiments of the present invention, a UV device comprises a reflector. A reflector can have a variety of configurations. In some embodiments, the reflector is a parabolic reflector. In some embodiments, the reflector is an elliptical reflector. In some embodiments, the reflector is a circular reflector. Exemplary embodiments comprising a reflector are depicted in the exemplary UV devices shown in FIGS. 21, 22A-D, and 23-25.

Reflectors may be obtained from the manufacturer of UV light sources. For example, reflectors of circular, elliptical and parabolic cross sections can be purchased from Hill Technical Sales Corp (Arlington Heights, Illinois, USA). A preferred supplier for parabolic reflector is Baldwin UV limited, 552 Farilie Road, Trading Estate, Berkshire, SL1 4PY, England.

UV devices comprising a reflector are schematically shown in FIGS. 21, 22A-D, and 23-25. However, as one of ordinary skill in the art will appreciate, a reflector can be configured into other UV devices described herein. In the exemplary UV device schematically shown in FIGS. 21, 22A-D, and 23-25, the UV devices two parabolic reflectors, referred to as a first or upper parabolic reflector **68** and a second or lower parabolic reflector **78**, wherein each reflector surrounds a UV light source **16**. In some embodiments, reflectors individually and partially surrounding a UV light source may form a continuous reflecting wall.

The UV devices schematically shown in FIGS. 21, 22A-D, and 23-25 are configured to be inserted through an opening located on top of a container so that the reflectors and UV light source move inwardly into the container while the sterilization cycle is being performed. Alternatively, the sterilization cycle begins when the UV device has been placed at a desired position within the container.

### I. Additional Components Of A UV Device

FIGS. 21, 22A-D, and 23-25 depict exemplary embodiments of UV devices of the present invention and uses thereof. Those figures also show additional components of UV devices of the present invention, their positioning and how those components may be operatively connected to a container, a room, space or defined environment, a UV light source, a UV detector, a frame, a bracket, a housing, and a range-finding device, and the like. As one of ordinary skill in the art will appreciate, individual components described herein can be combined in various ways and configurations in a UV device for a use described herein without deviating from the scope of the present invention.

In some embodiments of the present invention, a UV device comprises a motorized unit. A motorized unit can provide various functions, including, but not limited to positioning a UV light source within a container, a room, space or defined environment. A motorized unit may move a UV light source within a container, a room, space or defined environment to a horizontal position, to a vertical position or combination of both. As one of ordinary skill in the art will appreciate the moving of a UV light source within a container, a room, space or defined environment depends on parameters, such as size and power of a UV light source, diameter and height of the container, the room, space or defined environment and areas within the container, the room, space or defined environment a practitioner desires to sterilize as described herein.

In some embodiments of the present invention, a UV device comprises a rope, a cable or a rigid rod (indicated by **13** and **22** in the figures). A rope, a cable or a rigid rod is also useful for the positioning of a UV light source within a container, a room, space or defined environment. For example, as schematically depicted in FIGS. 23 and 25, a cable **13** is used to lower the UV devices shown inwardly and downwardly into the container **49**. In some embodiments, a cable **13** comprises a power cord **2** and additional cables connecting the UV light source **16** with a control box **1**. In some embodiments, *e.g.*, in some members of the UVT-4 family of portable UV devices, a rope **22** is used to position or to move a germicidal UV light source connected to an upper frame into an angular or vertical position with respect to another germicidal UV light source connected to a lower frame (see below and FIGS. 12, 13, and 16).

In some embodiments of the present invention, a UV device comprises one or more protective rods (indicated by **31** in the figures).

In some embodiments of the present invention, a UV device comprises a hanging hook. A hanging hook provides a convenient way of storing a UV device when not in use, by e.g., hanging it on a hook. A hanging hook can be attached to a UV device at various locations. Form, shape, positioning of a hanging hook are not critical. A handle **19** or **51**, *e.g.,* as shown schematically in FIGS. 3, 10, 12, 13, 16, 18, 21, 22C, 22D, 23, can also be used as a hanging hook. As such, the terms hanging hook and handle are used interchangeably herein. In addition to also serving as a hanging hook, a handle provides for the convenient transport of a UV device by a user. A handle **51** can be part of a frame, i.e., an extension of a frame or attached to a frame (*e*.*g*., *see* 3, 10, 12, 13, 16, 18, 21, 22C, 22D, 23). The handle may be of a different thickness than the frame. The handle and the frame can be made of the same material or a different one. A preferred material is a plastic. A preferred plastic is Delrin. Another preferred material for a handle is a metal, preferably, a light-weight metal. A handle can be attached to a UV device at various locations. Form, shape, positioning and function of an exemplary handle **51** are schematically in FIGS. 3, 10, 12, 13, 16, 18, 21, 22C, 22D, 23.

In some embodiments of the present invention, a UV device comprises an on/off or reset button (indicated by **3** in the figures). As one of ordinary skill in the art an on/off or reset button provides for the activation of the UV device. An on/off or reset button can be attached to a UV device at various locations. Form, shape, positioning and function of an exemplary on/off or reset button **3** are described in detail in the UV device embodiment UVT-4 *(see* FIGS. 1, 3 and below).

In some embodiments of the present invention, a UV device comprises a power cord (indicated by **2** in the figures). A power cord can be attached to a UV device at various locations. Form, shape, positioning and function of an exemplary power cord **2** are described in detail in the UV device embodiment UVT-4 *(see* FIGS. 1, 2 and below). In some embodiments, a power cord resides in rope **13**, *e.g.*, in exemplary UV devices UV6K and UV2K (FIGS. 21-25).In some embodiments of the present invention, a UV device comprises one or more UV lamp/UV light source sockets or adaptors (indicated by **20** in the figures). A UV lamp socket or adaptor **20** attaches a UV lamp **16** to a UV device, preferably through pins. A UV lamp socket or adaptor can be attached to a UV device at various locations. Typically, each UV lamp **16** is attached to a UV lamp socket or adaptor **20**. Form, shape, positioning and function of an exemplary UV lamp socket or adaptor **20** are described in detail in the UV device embodiment UVT-4. FIGS. 4-7 and 12 show non-limiting embodiments wherein UV lamp sockets or adaptors **20** are attached to either a lower frame **23** or an upper frame of a UV device of the UVT-4 family of UV devices.

In some embodiments of the present invention, a UV device comprises a power supply of UV lamp ballast. A power supply can be attached to a UV device at various locations. Preferably, a power supply is not visible from the outside of a UV device and housed in an inner compartment (*e.g., see* control box **1** in FIGS. 1-3, 20A) or in a cavity within the UV device. A ballast/power supply may power one or more UV light sources **16**. In some embodiments, a single ballast/power supply powers a UV lamp cluster. In some embodiments, a single ballast/power supply powers eight (8) UV light sources **16**. In some embodiments, a single ballast/power supply powers six (6) UV light sources **16** (*e.g.,* UV device model UV6K). In some embodiments, a single ballast/power supply powers four (4) UV lamps **16**. When eight (8) UV light source **16** are configured into a UV device, two ballasts/power supplies may be employed. In some embodiments, the ballast/power supply powers each UV light source **16** separately, i.e., each UV light source is powered by a separate electrical cable, wire or connector connecting the ballast/power supply with that particular UV lamp **16**. In some embodiments, the ballast/power supply powers in parallel a plurality of UV light sources **16**, i.e., a plurality of UV light sources **16** is powered by a single electrical cable, wire or connector connecting the ballast/power supply with the plurality of UV light sources **16**. In some embodiments, the ballast/power supply powers in parallel a UV light source **16** of a UV lamp cluster, i.e., the UV light sources **16** of the UV lamp cluster are powered by a single electrical cable, wire or connector connecting the ballast/power supply with the UV light source **16** of the UV lamp cluster.

In some embodiments, the ballasts/power supplies are separated from the UV light source(s) **16**. That distance can vary. Distances can be about 1m, about 2m, about 3m, about 4m, about 5m, about 6m, about 7m, about 8m, about 9m, about 10m, about 11m, about 12m or even more. For example, a UV device of the UVT-4 family of UV devices is preferably used to sanitize large containers, large rooms, large spaces or large defined environments. In those embodiments, the UV light sources and the power supply are physically separated from each other. This option provides for a more light-weight portable UV device and also provides greater flexibility with respect to moving and positioning the UV device on its own or within such large container, large room or large defined environment. In those embodiments, the UV light source(s) attached to those UV devices are powered by a power supply that resides in a control box **1** and wherein a cable **13** connects the power supply with the UV device and thus, with the germicidal UV light source(s) **16**. In some embodiments, cable **13** consists of two cables **13**, one being attached to the control box **1** as shown in FIGS. 1 and 2 and one being attached to the UV device as shown in FIG. 4. When in use and when power is to be provided to the UV device, those two cables **13** are then joined via a socket **57** (FIG. 17). In other embodiments, a single long cable **13** is being used to connect the control box 1 to the UV device directly, i.e., without connecting two sockets as described above *(e.g., see* FIGS. 21-25).

In some embodiments of a system of the present invention, a container **49** comprises manhole or port (indicated by **48** in the figures) as an opening. A manhole or port **48**, typically is found at large containers **49**, such as tanks and fermenters. A manhole or port **48** can be positioned at a container **49** at various locations, preferably at a position close to the periphery of the upper part of the container **49** as exemplary depicted in FIGS. 23 and 25. A manhole or port can also be located at a lower portion of a side wall of a container **49**, as exemplary depicted in FIGS 8-11, and 15. The manhole or port **48** is wide enough to allow insertion of a UV device of the present invention (*e.g., see* FIGS. 8-11, 15, 23 and 25).

In some embodiments of the present invention, a UV device comprises a interface comprising a touchscreen interface **5**. In some embodiments, an interface comprises an on/off/reset button permitting a user to activate or inactivate or reset a UV device, by *e.g.*, pushing the on/off/reset button. In some embodiments, an interface permits a user to set the time it takes to perform a sterilization cycle. In some embodiments, an interface permits a user to read the time remaining to complete a sterilization cycle. An interface can be attached to a UV device at various locations. Form, shape, and positioning of an interface are not critical. An exemplary touchscreen interface as used in a UV device system comprising an external control box **1**, is indicated by **5** in FIGS. 1 and 2. The touchscreen interface is adapted to provide various inputs for functionalities as described herein.

Some UV devices of the present invention comprise an easily accessible control box **1** with an on/off/reset switch **3** to activate and shut off (deactivate) the UV device Further, UV devices comprise circuitry for activating and shutting off (deactivating) the UV light source. The control box **1** may further include an emergency shutdown **4** and a status indicator/alarm light **6** to show whether power is being sent to the UV device and or alert the user to an alarm situation.

Additional components that can be operatively attached to a UV device or to a system of the present invention are shown in FIGS. 1-26.

### J. Positioning Of A UV Light Source In A Container, Room, Space Or Defined Environment

As will be appreciated by one of ordinary skill in the art, the positioning of a UV light source at a desired or predetermined position for the UV sterilization of a container, a room, space or defined environment will be determined by *e.g.*, the shape and volume (dimension) of the container, the room, space or defined environment and the shape, size and power output of the UV light source. Given the guidance provided herein, one of ordinary kill in the art will be able to properly position one or more UV light sources in a container, room, space or defined environment to achieve a desired level of sanitization, a desired killing or growth inhibition of one or more microorganisms using a method of the invention.

In some embodiments of the present invention, a UV light source is suspended from a manhole **48** of a container **49** of various dimensions.

In some embodiments of the present invention, e.g., when a UV device is used to sterilize a rather large container, the UV light source may be moved within the container from a first position to a second position and from a second position to a third position. For example, as shown in Figures 23 and 25, the UV light source is positioned in the approximate middle (center position) of a container **49** to practice a method of the invention. The height within a container at which a UV light source is positioned may also depend on the shape and volume (dimension) of the container, vessel, steel type used, and the shape, size and power output of the UV light source. For example, UV device Models UV6K and UV2K (described below in greater detail) permit descending a UV light source to a desired position within a container **49** by moving the UV light source from a first vertical position downwardly to a second vertical position within the container **49**. Likewise, UV device Models UV6K and UV2K permit ascending a UV light source to a desired position within a container **49** by moving the UV light source from a first vertical position upwardly to a second vertical position within the container **49**.

Further, as demonstrated by UV device Model UVT-1, when placed on the floor of a container **49** (see FIGS. 16 and 17), a plurality of wheels **37** attached to the frame of that UV device, permits the UV device to be moved into any desired position on the floor of the container **49** and subsequently deploy the UV light source attached to an upper frame so that it can be positioned at a desirable position within the container.

In some embodiments of the present invention, e.g., when a UV device is used to sterilize a rather large room, space or defined environment, the UV light source may be moved within the room, space or defined environment from a first position to a second position and from a second position to a third position. As described herein, for a large container, large room, or large defined environment, a UV device may be positioned on a bottom surface of such large container, large room, or large defined environment using an extension tool, as exemplary shown in FIGS. 14-17.

### K. Multiple UV Lamps/UV Light Sources

For use in the methods of the present invention, UV light sources, also referred to herein as UV lamps, can be configured in a variety of ways in a UV device. The configuration of one or more UV lamps within a UV device is referred to herein also as a UV lamp assembly or UV lamp cluster. In some embodiments of the present invention more than one UV lamp is used for the sterilization of a container, a room, a space or defined environment. Multiple UV lamps can be clustered together or spaced apart either symmetrically or asymmetrically in order to achieve the desired reduction in microorganisms in a timely and efficient manner.

For example, FIGS. 4-13 and 15-19 depict an embodiment of the present invention showing a UV lamp assembly having four UV lamps arranged in two parallel configurations, two attached to an upper frame and two attached to a lower frame. FIGS 21-23 show an arrangement of six UV lamps and FIGS 24 and 25 show a UV device having a single UV light source. Figures 3-13 and 15-19 depict non-limiting embodiments of the present invention, a member of a UVT-4 family UV device, showing four UV lamps, of which two UV lamps **16** are attached to a lower frame **23** and two UV lamps **16** are attached to an upper frame. Alternatively, those UV lamps **16** are attached to or enclosed in a housing **15**. When more than one UV lamp is used in an UV assembly or in a method of the present invention, each UV lamp may be the same or different.

In some embodiments of the present invention a UV device comprises more than one UV lamp. In some embodiments, at least two UV lamps are clustered together. In some embodiments, at least three UV lamps are clustered together. In some embodiments, at least four UV lamps are clustered together. In some embodiments, four UV lamps are clustered together. In some embodiments, five UV lamps are clustered together. In some embodiments, six UV lamps are clustered together. In some embodiments, seven UV lamps are clustered together. In some embodiments, eight UV lamps are clustered together. The clustering of the lamps may be at perpendicular angles or at any other angle. The more than one UV lamps in a UV lamp cluster can be positioned to each other at various angles ranging from about 0 to about 90 degree, from about 5 to about 45 degree, preferably from about 10 to about 30 degree, more preferably from about 15 to about 20 degree. In some embodiments of the present invention, the more than two UV lamps are positioned to each other in an about 5 degree angle. In some embodiments of the present invention, the more than two UV lamps are positioned to each other in an about 10 degree angle. In some embodiments of the present invention, the more than two UV lamps are positioned to each other in an about 15 degree angle. In some embodiments of the present invention, the more than two UV lamps are positioned to each other in an about 20 degree angle. In some embodiments of the present invention, the more than two UV lamps are positioned to each other in an about 25 degree angle. In some embodiments of the present invention, the more than two UV lamps are positioned to each other in an about 30 degree angle. In some embodiments of the present invention, the more than two UV lamps are positioned to each other in an about 40 degree angle. In some embodiments of the present invention, the more than two UV lamps are positioned to each other in an about 50 degree angle. In some embodiments of the present invention, the more than two UV lamps are positioned to each other in an about 60 degree angle. In some embodiments of the present invention, the more than two UV lamps are positioned to each other in an about 70 degree angle. In some embodiments of the present invention, the more than two UV lamps are positioned to each other in an about 80 degree angle. In some embodiments of the present invention, the more than two UV lamps are positioned to each other in an about 90 degree angle.

In some embodiments, more than one UV lamp is operatively attached to a frame. In some embodiments, at least two UV lamps are operatively attached to a frame. In some embodiments, at least three UV lamps are operatively attached to a frame. In some embodiments, at least four UV lamps are operatively attached to a frame. Four UV lamps may be operatively attached to a frame as shown exemplary in FIGS. 4-13 and 15-19. In some embodiments, at least five UV lamps are attached to a frame. In some embodiments, at least six UV lamps are attached to a frame. Six UV lamps may be operatively attached to a frame as shown exemplary in FIGS. 21-23. In some embodiments, at least seven UV lamps are attached to a frame. In some embodiments, at least eight UV lamps are attached to a frame.

### L. UVT-4 Family Of UV Devices

In some embodiments of the present invention, a UV device is a UV device depicted in Figures 3-19 and referred to herein as a member of the UVT-4 family of UV devices ("UVT-4"). UV device Model UVT-4 is a portable UV device. While referred to collectively herein as UVT-4, the portable UV devices of the UVT-4 family comprise various embodiments.

As a characteristic feature, members of the UVT-4 family of portable UV devices comprise a lower frame **23**, an upper frame, a first hinge (or pivot) **38**, at least one first germicidal UV light source, and at least one second germicidal UV light source. The first hinge **38** permits the multiple UV light sources of the UV device to move into different configurations as explained in greater detail below. Multiple orientations of UV light sources are particularly advantageous in view of UV light sources emitting UV light not symmetrically. As known in the art UV light sources may emit UV light strong at a direction perpendicular to the lamp axis and weaker at an angle to the axis, approaching zero in the orthogonal direction. As described herein, the UV device of the UVT-4 family comprise two UV light sources which can move into a perpendicular orientation with respect to each other, i.e., when activated, one UV light source resides in a horizontal configuration and one UV light source moves into a vertical configuration. Thus, UV devices of the UVT-4 family of devices use multiple orientations and configurations of UV light sources to compensate for the asymmetrical light emittance. Those and other features of that UV device are described in detail below and are depicted in FIGS. 1-20.

### 1. Lower Frame

In some embodiments of a lower frame of a UVT-4 UV device, the lower frame **23** comprises a first lower frame end **25** and a second lower frame end **30**. In some embodiments as described herein and as shown in FIGS. 3-19, additional parts are attached to the lower frame **23**, and, in particular to either first lower frame end **25** or second lower frame end **30**.

Preferably, the lower frame **23** is made of stainless steel. Parts attached to it may be also made of stainless steel or of aluminum. The lower frame **23** of a UVT-4 family member of UV devices may be described as having a rectangular shaped form and comprising four sides, i.e., a first (left) side, a second (right) side, an upper side and a lower side and two ends, i.e., a first lower frame end **25** and a second lower frame end **30** (*see* FIGS. 4, 5, 10). While the drawings for a UVT-4 UV device depict an exemplary rectangular lower frame **23**, the lower frame can also be round, oval or irregularly shaped. In some embodiments, to provide for a light-weight lower frame **23**, the lower frame **23** comprises one or more openings **60**.

In some embodiments, the lower side of the lower frame **23** comprises a coating. A preferred coating is a plastic coating. Another preferred coating is a teflon coating. Another preferred coating is ultra-high molecular weight polyethylene UHMP.

In some embodiments, the second lower frame end **30** comprises a first side plate **42** and a second side plate **43**. Preferably, the form thereof is rounded, but may also be not rounded. In some embodiments, a side plate spacer **41** connects the first side plate **42** to the second side plate **43**. As with all frames of UV devices, the first side plate **42** and the second side plate **43** may comprise one or more openings **60**.

In some embodiments, a set of wheels **37** is operatively attached to the first side plate **42** and to the second side plate **43**, so that each side plate has at least one wheel attached to it. Wheels **37** facilitate moving and positioning of the UV device in a container, a room, space or defined environment. The material for making the wheels is not critical. For example, the wheels can be made of plastic, metal or wood. Preferred are plastic wheels. In some embodiments, the wheels **37** are swiveling so that the UV device can be easily maneuvered around from a first position into a second position within a container **49**, a room, space or defined environment. In some embodiments, the wheels **37** are attached in a fixed position and adapted to move the UV device forward and backwards into a desired position within a container **49**, a room, space or defined environment.

In some embodiments, the second lower frame end **30** comprises a cross connector **44**. The cross connector **44** has at least one opening **45** suitable for accommodating a UV lamp socket/adaptor **20** and for operatively attaching at least one first germicidal UV light source **16**. In embodiments, wherein the portable UV device comprises more than one at least first germicidal UV light source, for each additional first germicidal UV light source, the cross connector **44** comprises an additional opening **45** into which an additional UV lamp socket **20** can be inserted.

As depicted in FIG. 19, when the upper frame and lower frame are attached to each other, the second upper frame end **29**, is positioned in between the first side plate **42** and the second side plate **43** of the lower frame **23** and is fastened to an upper side of both the first side plate **42** and the second side plate **43** so that the upper frame can be moved from the horizontal position with respect to the position of the lower frame **23** (as depicted in FIG. 19) into an angular position ranging from about 0 degree to 90 degrees with respect to the lower frame **23**. Such movement is possible because fasteners **47** do not hold the upper frame in a rigid position, but rather, upon activation of a means for controlling or facilitating movement of the upper frame to an angular position with respect to the position of the lower frame **23**, permit the upper frame to swing into such angular position.

In some embodiments, a handle **51** is attached to the lower frame **23**. The handle **51** allows easy maneuvering of the portable UV device from a first position into a second position within a container **49**, a room, a space or within a defined environment, in addition to convenient transportation (*e.g.*, hand carrying) and storing.

In some embodiments, a second anchoring post **50** for anchoring an extension spring **18** (see below) is attached to the lower frame **23**.

In some embodiments, the handle **19**, **51** is part of the second anchoring post **50**. An exemplary embodiment of a portable UV device comprising such arrangement is shown in FIG. 18.

In some embodiment, a T-shaped cap **35** is attached to the first lower frame end **25**. A bulb clamp **36** may be in between the T-shaped cap **35** and the first lower frame end **25**. The T-shaped cap **35** keeps the bulb clamp **36** in place.

In some embodiments, the first lower frame end **25** comprises at least one opening **60** suitable for accommodating a UV lamp socket/adaptor **20** and for attaching at least one first germicidal UV light source **16**. In embodiments, wherein the portable UV device comprises more than one at least first germicidal UV light source **16**, for each additional first germicidal UV light source, the cross connector **44** comprises an additional opening **60** into which an additional UV lamp socket **20** can be inserted.

In some embodiments, the length of the lower frame **23** is determined by the length of the UV light sources, i.e., the UV lamps **16**. As depicted, *e.g.,* in FIG. 18, the first lower frame end **25** and the second lower frame end **30** are spaced apart to accommodate the UV light source, i.e., the UV lamp **16**, which is attached to UV lamp sockets **20** that are attached to either lower frame end.

In some embodiments, a portable UV device comprises a means for attaching the portable UV device, temporarily or permanently for the time of sanitization to an opening of a container **49**, to a fixture in a room, or to a fixture in or at a defined environment. In some embodiments, such means is a mounting bracket or hanger **21**. In some embodiments, the mounting bracket or hanger **21** comprises a bracket tightening knob **26** (FIGS. 4, 11, 12, 15) or a clamping knob with stud **99** (FIG. 26), Upon engaging of the mounting bracket or hanger **21** with an opening of a container **49**, a fixture in a room, space or defined environment, the bracket tightening knob **26** or clamping knob with stud **99** can be fastened so to keep the portable UV device in position for a desired time.

A means for attaching the portable UV device, temporarily or permanently for the time of sanitization to an opening of a container **49**, to a fixture in a room, space or defined environment can be operatively attached to a portable UV device in a several ways. As a non-limiting example, the means for attaching the portable UV device, temporarily or permanently for the time of sanitization to an opening of a container **49**, to a fixture in a room, space or defined environment is attached to the lower frame **23** via a second hinge **34**. Such exemplary arrangement is shown, *e*.*g*., in FIGS. 4, 11, 15, 16 and 18. In some embodiments, the second hinge **34** movably connects the lower frame **23** to the means for attaching the portable UV device, temporarily or permanently for the time of sanitization to an opening of a container **49**, to a fixture in a room, space or defined environment.

In some embodiments, the means for attaching the portable UV device UVT-4, temporarily or permanently for the time of sanitization to an opening of a container **49**, to a fixture in a room, space or defined environment, comprises additional parts useful for performing an additional function of the portable UV device, *e*.*g*., moving the upper frame of the portable UV device into an angular position with respect to the lower frame **23**. Thus, in some embodiments, the means for attaching the portable UV device, temporarily or permanently for the time of sanitization to an opening of a container **49**, to a fixture in a room, space or defined environment, comprises a first rope post **27**. In some embodiments, such means further comprises a second rope post **28**. Exemplary and non-limiting arrangements are shown in FIGS. 4, 11, 15, and 18. The functionality of the rope posts will be described further below.

### 2. Upper Frame

In some embodiments of an upper frame of a UV device of the UVT-4 family , the upper frame comprises a first upper frame end **24** and a second upper frame end **29**. In some embodiments, as described herein and as shown in FIGS. 3-19, additional parts are attached to either first upper frame end **24** or second upper frame end **29**.

In some embodiments, a T-shaped cap **35** is attached to each of the first upper frame end **24**, first lower frame end **25**, second upper frame end **29**, and second lower frame end **30**. The T-shaped caps **35** hold in place UV bulb clamps **36**.

In some embodiments, a plurality of rods **31** are positioned in between the first upper frame end **24** and the second upper frame end **29**. The plurality of rods **31** are fastened to the first upper frame end **24** and to the second upper frame end **29** using fasteners. The plurality of rods **31** provides protection to the germicidal UV light source(s) **16**. In some embodiments, at least one rod **31** is positioned between the first upper frame end **24** and the second upper frame end **29**. In some embodiments, two rods **31** are positioned between the first upper frame end **24** and the second upper frame end **29**. In some embodiments, three rods **31** are positioned between the first upper frame end **24** and the second upper frame end **29**. In some embodiments, four rods **31** are positioned between the first upper frame end **24** and the second upper frame end **29**. In some embodiments, between two and ten rods **31** are positioned between the first upper frame end **24** and the second upper frame end **29**. The number of rods **31** between the first upper frame end **24** and the second upper frame end **29** is not critical. For best functionality of the portable UV device, sufficient UV light should be provided and not blocked by the rods. In view thereof, it is desirable, to use the thin sturdy rods, i.e., allow as much UV light as possible to pass through and provide sufficient protection of the UV light source, *e.g.,* so that objects that may damage the UV light source may not directly fall on it. An exemplary member of a portable UV device of the UVT-4 family is shown in FIG. 18 showing two rods **31** positioned on top of two UV light sources **16** (here, surrounded by a see-through housing **15** permitting the UV light to pass through and indicated by **15,16** in FIG. 18) Another exemplary member of a portable UV device of the UVT-4 family is shown in FIG. 19 showing two rods **31** positioned on top of two UV light sources **16** (here, surrounded by a see-through housing **15** permitting the UV light to pass through and indicated by **15,16** in FIG. 19) and two rods **31** positioned beneath two UV light sources **16** (the two lower rods are not well seen in this drawing; however, discernable by the four fasteners attached to the second upper frame end, which are used to attach the rods **31** to the upper frame ends **24** and **29**). A further exemplary member of a portable UV device of the UVT-4 family is shown, *e.g.*, in FIGS. 5-10 showing four rods **31** positioned around each UV light source **16** attached to the upper frame (here, surrounded by a see-through housing **15** permitting the UV light to pass through, and indicated by **15,16** in FIGS. 5-10). In some embodiments, the rods **31** penetrate a plurality of cross connectors **32**. The cross connectors **32** provide stability to the upper frame stabilizing the plurality of rods **31**. One of ordinary skill in the art will appreciate that the number of cross connectors is chosen, *e*.*g*., based on the length of the rods **31** and UV light sources **16**. The exemplary UV devices depicted in FIGS. 4 and 18 each comprise two cross connectors **32**.

In some embodiments, the length of the upper frame is determined by the length of the UV light sources, i.e., the UV lamps **16**. As depicted, *e.g.*, in FIG. 18, the first upper frame end **24** and the second upper frame end **29** are spaced apart to accommodate the UV light source, i.e., the UV lamp, which is attached to UV lamp sockets **20** that are attached to either upper frame end.

In some embodiments, the upper frame is made of stainless steel. Parts attached to the upper frame may also be made of stainless steel or, alternatively, of aluminum.

In some embodiments, the upper frame end **29** is configured to comprise a handle **51**. An exemplary member of a portable UV device of the UVT-4 family comprising a handle **51** at the upper frame end **29** is shown, *e.g.*, in FIG. 18.

In some embodiment, a first hinge (pivot) **38** is attached to the second upper frame end **29**. The first hinge (pivot) **38** will be described below in greater detail.

When the portable UV device UVT-4 is not in use (as described further below), then the upper frame is positioned on top of the lower frame. Such arrangement is depicted, *e.g.*, in FIGS. 3-11.

### 3. First Hinge (Pivot)

As shown in FIGS. 18 and 19 (and others), the first hinge **38** movably connects the lower frame **23** to the upper frame. Further, the first hinge **38** is adapted to permit movement of the upper frame into an angular position with respect to the position of the lower frame **23**. In that regard, the first hinge **38** can also be described as a "swing."

FIG. 18 depicts the attachment of the first hinge (pivot) **38** to the second upper frame end **29**. In some embodiments, the first hinge (pivot) **38** comprises a first opening to allow a cable **58** running through. The first opening may be located at the lower side of the first hinge (pivot) **38** so that the cable **58** running through that opening can be connected to an extension spring **18** (see further below). In some embodiments, the first hinge (pivot) **38** comprises a second opening to allow a cable **58** becoming fastened therein. Thus, the second opening is adapted to serve as a cable anchoring point **62**. In some embodiments, a first end of the cable **58**, is anchored at the second opening (cable anchoring point **62**) and the cable is guided on a cable guide **61** formed as part of the first hinge (pivot) **38** towards the first opening at the lower end of the hinge (pivot) **38**, and extrudes therefrom so that the second end of the cable **61** forms a first anchoring post **46** with the first hook **59** of the extension spring **18** (see further below).

In some embodiments, the first hinge (pivot) **38** is made of stainless steel, or, alternatively, of aluminum.

### 4. At Least One First Germicidal UV Light Source Operatively Attached To The Upper Frame

A UV device of the UVT-4 family of UV devices comprises at least one first germicidal UV light source operatively attached to the upper frame. In some embodiments, the at least one first germicidal UV light source is operatively connected to the upper frame, via a UV lamp socket or adaptor **20**.

In some embodiments, a UV device comprises additional first germicidal UV light sources connected to the upper frame. In some embodiments, the at least first germicidal UV light source is a member of a plurality of first germicidal UV light sources, selected from the group consisting of two first germicidal UV light sources, three first germicidal UV light sources, four first germicidal UV light sources, five first germicidal UV light sources, six first germicidal UV light sources, seven first germicidal UV light sources, eight first germicidal UV light sources, nine first germicidal UV light sources, and ten first germicidal UV light sources. As one of ordinary skill in the art will appreciate, the number of first germicidal UV light sources connected to the upper frame is not limited and may comprise more than ten. In some embodiments, members of the plurality of first germicidal UV light sources are the same germicidal UV light sources. In some embodiments, members of the plurality of first germicidal UV light sources are different germicidal UV light sources.

### 5. At Least One Second Germicidal UV Light Source Operatively Attached To The Lower Frame

A UV device of the UVT-4 family of UV devices comprises at least one second germicidal UV light source operatively attached to the lower frame **23**. In some embodiments, the at least one second germicidal UV light source is connected to the lower frame **23**, via a UV lamp socket or adaptor **20**.

In some embodiments, a UV device comprises additional second germicidal UV light sources connected to the lower frame **23**. In some embodiments, the at least second germicidal UV light source is a member of a plurality of second germicidal UV light sources, selected from the group consisting of two second germicidal UV light sources, three second germicidal UV light sources, four second germicidal UV light sources, five second germicidal UV light sources, six second germicidal UV light sources, seven second germicidal UV light sources, eight second germicidal UV light sources, nine second germicidal UV light sources, and ten second germicidal UV light sources. As one of ordinary skill in the art will appreciate, the number of second germicidal UV light sources connected to the lower frame is not limited and may comprise more than ten. In some embodiments, members of the plurality of second germicidal UV light sources are the same germicidal UV light sources. In some embodiments, members of the plurality of second germicidal UV light sources are different germicidal UV light sources.

### 6. At Least One First Germicidal UV Light Source Operatively Attached To The Upper Frame And At least One Second Germicidal UV Light Source Operatively Attached To The Lower Frame

In some embodiments, a UV device of the UVT-4 family of UV devices comprises at least one first germicidal UV light source operatively attached to the upper frame and at least one second germicidal UV light source operatively attached to the lower frame **23**

In some embodiments of a UV device, the first germicidal UV light source operatively attached to the upper frame and the second germicidal UV light source operatively attached to the lower frame **23** are the same germicidal UV light sources. In some embodiments, the first germicidal UV light source operatively attached to the upper frame and the second germicidal UV light source operatively attached to the lower frame **23** are different germicidal UV light sources.

In some embodiments, a UV device comprises a lower frame to which two second germicidal UV light sources are operatively attached and an upper frame to which two first germicidal UV light sources are operatively attached.

Suitable UV lamps for use in a UV device of the UVT-4 family of devices are described herein. First and second germicidal UV light sources for use in UV devices of the UVT-4 family are not limited and include, without limitation, low pressure mercury amalgam bulbs. It has been found that low pressure mercury amalgam bulbs are very efficient and cost effective UV light sources. In some embodiments, medium pressure UV bulbs or pulsed UV Xenon type lamps are used. They are significantly higher priced. Medium pressure lamps typically operate at temperature in excess of 500F, making them somewhat less preferred. For sanitization of smaller containers (having a volume in the range of from about 50 gallons to about 500 gallons), smaller rooms, spaces or defined environments, LED bulbs can also be used; however they lack the power necessary for large volumes (*e.g.*, tanks up to and exceeding 500,000 gallons). Those UV light sources can also be used as a part or component of other portable UV devises described herein.

The choice of first and second germicidal UV light source for use in UV devices of the UVT-4 family may depend on the size and volume of the container, room, space or defined environment to be sanitized. As one of ordinary skill in the art will appreciate, increasing the number of UV light sources **16** will decrease sanitization time and, in addition, will allow for greater sized and larger volume containers, rooms, spaces or defined environments to be sanitized. One of ordinary skill in the art will appreciate that UV devices described herein can be adapted easily to accommodate a desired number and a desired size of UV light sources.

The UV light intensity of the combined UV light sources (i.e., the combination of first germicidal UV light source(s) and second germicidal UV light source(s)) of a UV device of the UVT-4 family can be configured to efficiently irradiate interior surfaces (side walls, bottom and ceiling) of a container, a room, space or defined environment at a desired UV intensity. In some embodiments, the combined UV light sources of a UV device of the UVT-4 family are configured to irradiate interior surfaces (side walls, bottom and ceiling) of a container, a room, space or defined environment with at least 10,000 microjoules/cm². In some embodiments, the combined UV light sources of a UV device of the UVT-4 family are configured to irradiate interior surfaces (side walls, bottom and ceiling) of a container, a room, space or defined environment with at least 20,000 microjoules/cm². In some embodiments, the combined UV light sources of a UV device of the UVT-4 family are configured to irradiate interior surfaces (side walls, bottom and ceiling) of a container, a room, space or defined environment with at least 30,000 microjoules/cm². In some embodiments, the combined UV light sources of a UV device of the UVT-4 family are configured to irradiate interior surfaces (side walls, bottom and ceiling) of a container, a room, space or defined environment with at least 40,000 microjoules/cm². In some embodiments, the combined UV light sources of a UV device of the UVT-4 family are configured to irradiate interior surfaces (side walls, bottom and ceiling) of a container, a room, space or defined environment with at least 50,000 microjoules/cm². In some embodiments, the combined UV light sources of a UV device of the UVT-4 family are configured to irradiate interior surfaces (side walls, bottom and ceiling) of a container, a room, space or defined environment with at least 60,000 microjoules/cm². In some embodiments, the combined UV light sources of a UV device of the UVT-4 family are configured to irradiate interior surfaces (side walls, bottom and ceiling) of a container, a room, space or defined environment with at least 70,000 microjoules/cm². In some embodiments, the combined UV light sources of a UV device of the UVT-4 family are configured to irradiate interior surfaces (side walls, bottom and ceiling) of a container, a room, space or defined environment with at least 80,000 microjoules/cm². In some embodiments, the combined UV light sources of a UV device of the UVT-4 family are configured to irradiate interior surfaces (side walls, bottom and ceiling) of a container, a room, space or defined environment with at least 90,000 microjoules/cm². In some embodiments, the combined UV light sources of a UV device of the UVT-4 family are configured to irradiate interior surfaces (side walls, bottom and ceiling) of a container, a room, space or defined environment with at least 100,000 microjoules/cm². In some embodiments, the combined UV light sources of a UV device of the UVT-4 family are configured to irradiate interior surfaces (side walls, bottom and ceiling) of a container, a room, space or defined environment with at least 110,000 microjoules/cm². In some embodiments, the combined UV light sources of a UV device of the UVT-4 family are configured to irradiate interior surfaces (side walls, bottom and ceiling) of a container, a room, space or a defined environment with at least 120,000 microjoules/cm². In some embodiments, the combined UV light sources of a UV device of the UVT-4 family are configured to irradiate interior surfaces (side walls, bottom and ceiling) of a container, a room, space or a defined environment with at least 130,000 microjoules/cm². In some embodiments, the combined UV light sources of a UV device of the UVT-4 family are configured to irradiate interior surfaces (side walls, bottom and ceiling) of a container, a room, space or defined environment with at least 140,000 microjoules/cm². In some embodiments, the combined UV light sources of a UV device of the UVT-4 family are configured to irradiate interior surfaces (side walls, bottom and ceiling) of a container, a room, space or defined environment with at least 150,000 microjoules/cm².

### 7. UV Light Permissible Housing

As described herein, UV devices may comprise a housing **15** surrounding or encasing fully or partially a germicidal UV light source and/or a UV lamp **16**. In some embodiments, the at least one first germicidal UV light source **16** of the UV device UVT-4, resides in a first housing **15**, In some embodiments, the first housing **15** fully surrounds the at least one first germicidal UV light source **16**. In some embodiments, the first housing **15** partially surrounds the at least one first germicidal UV light source. In the exemplary embodiments of UV devices of the UVT-4 family shown in FIGS. 3-13 and 15-19, a first housing **15** is a see-through housing **15** permitting the UV light to pass through, and surrounds a UV light source **16.** Both are indicated by **15, 16** in those figures.

Similarly as described above, in some embodiments, the at least one second UV light source **16** is surrounded by a housing **15**. In some embodiments, the at least one second germicidal UV light source **16** of the UV device UVT-4, resides in a second housing **15**, In some embodiments, the second housing **15** fully surrounds the at least one second germicidal UV light source **16**. In some embodiments, the second housing **15** partially surrounds the at least one second germicidal UV light source. In the exemplary embodiments of UV devices of the UVT-4 family shown in FIGS. 3-13 and 15-19, a second housing **15** is a see-through housing **15** permitting the UV light to pass through, and surrounds a UV light source **16.** Both are indicated by **15, 16** in those figures.

In embodiments wherein the housing permits the UV light to pass through, the at least one first germicidal UV light source and the at least one second germicidal UV light source will be fully functional for sanitization, as described herein, without being removed from the housing. A UV light permissible housing may be made of various materials known in the art, including, but not limited to, UV fused silica, CaF₂, MgF₂, BaF₂, quartz, sapphire, teflon, polydimethylsiloxane, TPX® or polymethylpentene (PMP). TPX®, is a 4-methylpentene-1 based polyolefin manufactured and marketed by Mitsui Chemicals, Inc. A preferred housing material permitting UV light to pass through is teflon.

### 8. Means For Controlling Movement Of The Upper Frame To An Angular Position With Respect To The Position Of The Lower Frame

As described herein, UV devices of the UVT-4 family comprise a lower frame **23** and an upper frame, wherein the upper frame can move from a horizontal position with respect to the lower frame into an angular position ranging from about 0 degree to about 90 degrees, including, moving the upper frame into a vertical, a perpendicular position with respect to the lower frame **23**. Members of the UV device family UVT-4 comprise various means for controlling or facilitating the movement of the upper frame to an angular position with respect to the position of the lower frame **23**. Thus, in some embodiments, a UV device comprises a means for controlling or facilitating the movement of the upper frame to an angular position with respect to the position of the lower frame. In some embodiments, the means for controlling or facilitating the movement of the upper frame permits the at least one first germicidal UV light source connected to the upper frame be positioned at an angle ranging from about 0 degree to about 90 degrees with respect to the position of the at least second germicidal UV light source connected to the lower frame **23**.

In some embodiments, a means for controlling or facilitating the movement of the upper frame to an angular position with respect to the position of the lower frame comprises an extension spring **18**. In some embodiments, a UV device comprises an extension spring **18** comprising a first end comprising a first hook **59** at and a second end comprising a second hook **50**.

In some embodiments, the first hook **59** connects to a first anchoring post **46**. In some embodiments, the first anchoring post **46** is comprised of the second end of the cable **58**. The second end of the cable **58** may form a loop and the loop connects with the first hook **59** of the extension spring **18.** Such an arrangement, *e.g.*, is depicted in FIG. 19.

In some embodiments, the second hook **50** connects to a second anchoring post **19**. In some embodiments, the second anchoring post **19** is attached to the lower frame **23** (see above). Such an arrangement, *e.g.*, is depicted in FIG. 18.

In some embodiments, the upper frame of a UV device of a UVT-4 family is held in a horizontal position with respect to the lower frame **23**, by virtue of an upper frame fixture clip **33**. In some embodiments, an upper frame fixture clip **33** is attached to the lower frame **23**, preferably to the first lower frame end **25**. Such arrangement, *e.g.*, is shown in FIG. 5. The upper frame fixture clip **33** engages with the first upper frame end **24** and when engaged prevents the upper frame from moving into an angular position with respect to the lower frame **23**. In FIG. 5, the upper frame fixture clip **33** is shown disengaged from the first upper frame end and the upper frame is shown in a slightly angular position with respect to the lower frame **23**.

In some embodiments, the upper frame of a portable UV device of a UVT-4 family is held in horizontal position with respect to the lower frame **23**, by virtue of a rope **22**. In some embodiments, a first end of the rope **22** is attached to the first upper frame end **24** at a rope anchoring point **52**. The second end of the rope **22** is movably wound around a first rope post **27** (attached to *e.g.*, a mounting bracket or hanger **21**, see above, or directly to the lower frame **23**). In some embodiments, wherein a second rope post **28** is present, the second end of the rope 7 may be wound around both the first rope post **27** and the second rope post **28**. A non-limiting arrangement comprising a first rope post **27** and a second rope post **28**, *e.g.,* is shown in FIG. 52. When the UV device is not in use, the rope **22** firmly would around the first rope post **27** and second rope post **28**, prevents the upper frame from moving into an angular position with respect to the lower frame **23**. Upon releasing the rope **22** from the first rope post **27** or first rope post **27** and second rope post **28**, the upper frame can move into an angular position with respect to the lower frame **23**. A partial release of the rope **22** and an angular position of the upper frame with respect to the position of the lower frame **23**, *e.g.,* is shown in FIG. 12. Upon further releasing the rope **22**, the upper frame moves into a vertical or perpendicular position with respect to the lower frame **23**. Such further release of the rope **22** and a vertical or perpendicular position of the upper frame with respect to the position of the lower frame **23**, *e.g.,* is shown in FIGS. 13 and 17.

With respect to the "extension spring" means for controlling or facilitating movement of the upper frame of the UV device to an angular position with respect to the position of the lower frame **23**, one of ordinary skill in the art reading the disclosure herein, will appreciate that, upon disengaging the upper frame fixture clip **33** and/or upon loosening the rope **22** (i.e., unwinding from the rope post(s)), the extension spring **18** exerts a pull pressure. This pull pressure leads to the extension spring **18** pulling the second end of the cable **58** towards the extension spring **18** resulting in a swing movement of the first hinge (pivot) **38** due to the flexibility of fasteners **47** and thereby moving the upper frame from a horizontal position into an angular position ranging from about 0 degree to about 90 degrees, with respect to the position of the lower frame **23**.

In some embodiments, a UV device of the UVT-4 family comprises at least one stop post **39**. In some embodiments, a portable UV device of the UVT-4 family comprises at least two stop posts **39**. In some embodiments, a first stop post **39** is attached to the first side plate **42**. In some embodiments, a second stop post **39** is attached to the second side plate **43**. The stop post **39** is adapted to prevent movement of the upper frame, and thereby movement of a second germicidal UV light source connected to that upper frame, beyond a desired position. Such desired position may be any predetermined angular position between the upper frame and the lower frame **23**. A preferred angular position is an about vertical or an about perpendicular position. As such the at least one stop post **39** is adapted to prevent movement of the at least one second germicidal UV light source (connected to the upper frame) beyond an about perpendicular position with respect to the position of the at least first germicidal UV light source (connected to the lower frame **23**).

In some embodiments, a means for controlling or facilitating the movement of the upper frame to an angular position with respect to the position of the lower frame is a pneumatic cylinder. Pneumatic cylinders (also known in the art as air cylinders) are mechanical devices which use the power of compressed gas to produce a force in a reciprocating linear motion. Like hydraulic cylinders, a piston is forced to move in a desired direction. A piston typically is a disc or cylinder, and a piston rod transfers the force it develops to the object to be moved, such as then upper frame of a portable UV device.

In some embodiments, a means for controlling or facilitating the movement of the upper frame to an angular position with respect to the position of the lower frame is a motor.

In some embodiments, a means for controlling or facilitating the movement of the upper frame to an angular position with respect to the position of the lower frame is a winch.

In some embodiments, a means for controlling or facilitating the movement of the upper frame to an angular position with respect to the position of the lower frame is a servo

### 9. UV Sensor

A UV device of the UVT-4 family of UV devices may comprise other components described herein. One of ordinary skill in the art will appreciate that those components, such as a UV sensor, a reflector, a mirror, etc., can be attached to either the lower frame **23** or the upper frame of the UV device. In some embodiments, a UV device comprises a UV sensor **17**. An embodiment, wherein the UV sensor **17** is attached to the upper frame is shown in FIGS. 3 and 6-8. In some embodiments, the UV sensor **17** is a UV-C sensor. In some embodiments, a UV-C sensor is adapted to keep real time track of UV-C output during a sanitization cycle.

### 10. Control Box

In some embodiments of the present invention, a UV light source is operatively connected to a control box **1**. In some embodiments described herein, a UV light source of a UV device comprises a control box **1** that is part of the UV device itself. In those embodiments, the control box **1** may be described as internal as it is an integral part of the respective UV device. In some embodiments, a UV light source of a UV device is operatively connected to a control box **1** via a cable **13**. In those embodiments, the control box **1** may be described as external as it is not an integral part of the UV device.

An external control box **1** can be made a various materials. In some embodiments, an external control box **1** is made of stainless steel. In some embodiments, the exterior of control box **1** is a stainless steel NEMA4 enclosure.

As described herein, a control box **1** controls various functionalities of a portable UV device. This control typically is controlled by a circuit board that may reside in the control box **1**. Thus, in some embodiments, a UV light source of a UV device is connected to a control box **1**, wherein the control box **1** comprises a circuit board controlling one or more functionalities of a UV device or relaying a response from the UV device. Those functionalities may be individually programmed and adjusted to the needs of an individual user. Non-limiting functionalities of a UV device controlled by or relayed by a circuit board are described herein.

In some embodiments of a UV device of the present invention, the control box **1** comprises a circuit board and at least three components selected from the group consisting of (1) a power supply, (2) electronics for activating/deactivating the first germicidal UV light source, (3) a timer for controlling the duration of the sterilization cycle, (4) a memory for storing a pre-determined sterilization cycle, (5) a safety switch, (6) an on/off/reset button, (7) a status indicator light, (8) an alarm light; and (9) a user interface selected from the group consisting of a touch screen and a keyboard.

In some embodiments, the control box **1** comprises at least four components selected from (1) through (9). In some embodiments, the control box **1** comprises at least five components selected from (1) through (9). In some embodiments, the control box **1** comprises at least six components selected from (1) through (9). In some embodiments, the control box **1** comprises at least seven components selected from (1) through (9). In some embodiments, the control box **1** comprises at least eight components selected from (1) through (9). In some embodiments, the control box **1** comprises all components (1) through (9).

As one of ordinary skill in the art will appreciate various combinations of components (1) through (9) may be included in a control box **1**. Non-limiting examples of combinations of at least three components selected from (1) through (9) include: components (1), (2) and (3); components (4), (5) and (6); components (1), (3) and (6); components (1), (5) and (9); components (1), (3), (5) and (7); components (2), (4), (6) and (8); components (1), (2), (3), (4) and (5); components (7), (8) and (9); components (5), (6), (7), (8) and (9).

In some embodiments, the control box **1** comprises a touchscreen interface **5**. A control box **1** having a touchscreen interface **5** is shown, *e.g.*, in FIGS. 1 and 3. In some embodiments, the touchscreen interface **5** is adapted to provide an input for a functionality. As one of ordinary skill in the art will appreciate input for a variety of functionalities may be provided. In some embodiments, a touchscreen interface is adapted to provide an input for a functionality selected from the group consisting of activating a portable UV device, deactivating a portable UV device, providing time input for completing a UV sterilization of a container, a room, or a defined environment, providing time elapsed for UV sterilization of the container, the room, or the defined environment, setting a desired UV intensity level, adjusting a UV intensity level and logging in a code for a user. For example; a UV intensity level may be adjusted based on the condition of a container **49**, a room, space or defined environment, such as wet or dry interior surfaces, etc.

A control box **1** may comprise additional features. In some embodiments, a control box **1** comprises an on/off/reset switch **3**. The on/off/reset switch **3** permits an individual to activate, deactivate and reset the system and UV device. A control box **1** comprising an on/off /reset switch **3** is shown, *e*.*g*., in FIGS. 1 and 3.

In some embodiments, a control box **1** comprises a button for emergency shutdown **4**. The emergency shutdown button **4** permits an individual to quickly shut down the system and portable UV device. A control box **1** comprising an emergency shutdown button **4** is shown, *e.g.,* in FIGS. 1 and 3.

In some embodiments, a control box **1** comprises a status indicator light **6**. The status indicator light **6**, when lit, alerts an individual that the system and portable UV device are operating. The status indicator light **6**, when not lit, alerts an individual that the system and portable UV device are not operating. A control box **1** comprising a status indicator light **6** is shown, *e*.*g*., in FIGS. 1 and 3.

In some embodiments, a control box **1** comprises an alarm light. The alarm light, when flashing, may alert an individual to a malfunction of the system or portable UV device, or to a completion of a sanitization cycle. In some embodiments, a control box **1** comprises a status indicator light **6** that also functions as an alarm light.

In some embodiments, a control box **1** comprises an audible alarm system. The audible alarm system may alert an individual to a malfunction of the system or portable UV device, or to a completion of a sanitization cycle,

Exemplary layouts of an interior of a control box **1** are shown in FIGS. 20A-C.

In some embodiments, a control box **1** comprises one or more lamp ballasts (or power supplies; FIG. 20B). In some embodiments, a lamp ballast connects to a motor or servo through an electrical cable. In some embodiments, a lamp ballast connects to a UV light source through an electrical cable.

In some embodiments, a control box **1** comprises a wireless communication device, including, but not limited to a wireless transponder and or transceiver to send a wireless signal to a user or to receive a wireless signal from a user.

While the above described various parts and features of members of the UV device Model UVT-4 family one of ordinary skill in the art will appreciate that any arrangement or positioning of parts described can be varied without deviating from the scope of the invention. In addition, UV device Model UVT-4 depicted schematically in FIGS. 3-13 and 15-19 may comprise any additional component described herein.

### M. UV Device UV6K Family

In some embodiments of the present invention, a UV device is a UV device of the UV6K family of UV devices. An exemplary family member thereof is depicted in FIGS. 21-23.

This UV device embodiment comprises (i) a UV light source, (ii) a frame, (iii) a support cable, and (iv) at least one reflector.

The UV light source of the UV6K family may comprise one or more UV lamps. In some embodiments, the UV light source comprises one UV lamp. In some embodiments, the UV light source comprises two UV lamps. In some embodiments, the UV light source comprises three UV lamps. In some embodiments, the UV light source comprises four UV lamps. In some embodiment, the UV light source comprises five UV lamps. In some embodiments, the UV light source comprises six UV lamps. A UV6K UV device having six UV bulbs is depicted schematically in FIGS. 21-23.

In some embodiments the frame of a UV6K family UV device is a frame assembly. The frame assembly holds in place the UV light source. The frame assembly further provides protection against damage to the UV light source. The frame assembly further provides protection against damage to the reflector(s). In some embodiments, the frame assembly comprises a means for holding the UV device during installation and transport.

A non-limiting frame assembly of a UV6K UV device having six UV bulbs is depicted schematically in FIGS. 21-23. Parts of the frame assembly may include a handle **51**, a top clamp **66**, a top outer frame **69**, an upper handle core **70**, a lower handle core **72**, a linear divider **73**, a divider disk **74**, a bottom clamp **76**, and a bottom outer frame **77**. Other parts of a non-limiting frame assembly of a UV6K UV device are shown in FIGS. 21-23.

The support cable **13** of a UV6K UV device provides a means for holding the UV light source suspended in the container, room, space or defined environment that is being sanitized. Inside the support cable **13** is the electrical wiring that is required for providing functionality to the UV device. Because of its inclusion within the support cable **13**, the electrical wiring is also protects from mechanical damage and excessive wear.

In some embodiments, the support cable **13** is operatively connected to a control box **1**. As detailed in the context of the UVT-4 UV device, such control box 1 may include all or some of the following: power supply, electronics for the UV light source(s), a controller and assorted electronics (circuit board) for measuring UV dose, a timer/clock for controlling the duration of a sterilization cycle, memory for storing pre-configured sterilization cycle(s), safety switch, user interface (*see also*, FIGS. 1-3, 20A-C).

When suspended into a container, room, space or defined environment, the UV device UV6K is operatively attached to a bracket **21**, as shown in FIG. 23.

A UV device of the UV6K family comprises one or more reflectors **68**, **78**. Those reflectors may be referred to as first or upper reflector **68** and second or lower reflector **78**. In some embodiments, a reflector is a parabolic reflector. Reflectors aid in distributing the UV light by directing it into angles where the UV light source itself has low remittance. One or more reflectors may be use and positioned close at the ends of the UV device as shown in FIGS. 21-23. Alternatively, the one or more reflectors **68**, **78** may be positioned elsewhere within the frame assembly, *e.g.*, in the middle.

The UV device depicted schematically in FIGS. 21-23 may comprise any additional component(s) described herein.

### N. UV Device UV2K Family

In some embodiments of the present invention, a UV device is a UV device of the UV2K family of UV devices. An exemplary, non-limiting family member thereof is depicted in FIGS. 24-25. This UV device embodiment comprises (i) a UV light source, (ii) a frame assembly (iii) a support cable, and (iv) at least one reflector.

The non-limiting UV device of the UV2K family shown in FIGS. 24-25 comprises a single UV lamp. Additional UV lamps may be added.

In some embodiments the frame of a UV2K family UV device is a frame assembly The frame assembly holds in place the UV light source. The frame assembly further provides protection against damage to the UV light source. The frame assembly further provides protection against damage to the reflector(s). In some embodiments, the frame assembly comprises a means for holding the UV device during installation and transport. In some embodiments such means is a plurality of openings in the frame (*see*, FIG. 24). Alternatively, a handle **51** can be operatively attached to the frame.

A non-limiting frame assembly of a UV2K UV device having a single UV bulb is depicted schematically in FIGS. 24-25. Parts of the frame assembly may include a top outer frame **69**, a bottom outer frame **77**, and a vertical frame **90**, Other parts of a non-limiting frame assembly of a UV2K UV device are shown in FIGS. 24-25. While the frame assembly of the UV2K UV device is different from that of the UV6K UV device, their functionalities are similar.

The support cable **13** of a UV2K UV device provides a means for holding the UV light source suspended in the container, room, space or defined environment that is being sanitized. Inside the support cable **13** is the electrical wiring that is required for providing functionality to the UV device. Because of its inclusion within the support cable **13**, the electrical wiring is also protects from mechanical damage and excessive wear.

In some embodiments, the support cable **13** is operatively connected to a control box **1**. As detailed in the context of the UVT-4 UV device, such control box 1 may include all or some of the following: power supply, electronics for the UV light source(s), a controller and assorted electronics (circuit board) for measuring UV dose, a timer/clock for controlling the duration of a sterilization cycle, memory for storing pre-configured sterilization cycle(s), safety switch, user interface (*see also*, FIGS. 1-3, 20A-C).

When suspended into a container, room, space or defined environment, the UV device UV2K is operatively attached to a bracket **21**, as shown in FIG. 25.

A UV device of the UV2K family comprises one or more reflectors **68**, **78**. Those reflectors may be referred to as first or upper reflector **68** and second or lower reflector **78**. In some embodiments, a reflector is a parabolic reflector. Reflectors aid in distributing the UV light by directing it into angles where the UV light source itself has low remittance. One or more reflectors **68**, **78** may be used, positioned close to the ends as shown in FIGS. 24-25. Alternatively, the one or more reflectors **68**, **78** may be positioned elsewhere within the frame assembly, e.g., in the middle.

The UV device depicted schematically in FIGS. 24-25 may comprise any additional component(s) described herein.

### III. CONTAINERS, ROOMS, SPACES AND DEFINED ENVIRONMENTS

In some embodiments, a UV device, preferably a UV light source, more preferably a germicidal UV light source, is introduced into a container, a room, space or defined environment.

### A. Containers

In some embodiments, a container is exposed to UV radiation. A container accepts a UV light source for the purpose of sterilization of the interior of the container, including any and all objects, fluids, materials, and surfaces contained within the interior of the container. In some embodiments, the objects, fluids, materials, and surfaces within the interior of the container are contained within the container temporarily. In other embodiments, they are contained within the container permanently.

The present invention provides a variety of containers. Containers, include, but are not limited to a vat, a silo, a tub, a basket, a case, a box, a barrel, a storage bin, a barrel, a keg, a tank (*e.g.,* a Porta tank), a container for biological fluids, a beverage container, and an aquarium.

A container for biological fluid includes, but is not limited, to a container for blood, a container for blood products, a container for a fermentation product, a container for a cell culture product, or a container for a biotechnology product. In some embodiments, a fermentation product is an alcoholic beverage. In some embodiments, a fermentation product is wine.

A beverage container includes, but is not limited, to a beverage container for water, milk, coffee, tea, juice, an alcoholic beverage, or a carbonated beverage. An alcoholic beverage includes, but is not limited to beer, wine, gin, vodka, or whisky. A preferred alcoholic beverage is wine. Thus, a preferred container is a container for the fermentation of wine.

A container also includes any container for storing, transporting or selling a dairy product, a liquid dairy, a liquid dairy composition or a dry dairy composition. A " liquid dairy composition" is any source of milk or milk ingredient. In exemplary embodiments, the milk is from sheep, goats, or cows. Liquid dairy compositions include without limitations, for example, liquid milk, liquid skim milk, liquid non-fat milk, liquid low fat milk, liquid whole milk, liquid half & half, liquid light cream, liquid light whipping cream, liquid heavy cream, liquid lactose free milk, liquid reduced lactose milk, liquid sodium free milk, liquid reduced sodium milk, liquid dairy fortified with nutrients, such as vitamins A, D, E, K, or calcium, liquid high protein dairy, liquid whey protein concentrate, liquid whey protein isolate, etc. Milk concentrates and milk protein concentrates are particularly contemplated liquid dairy compositions. The term "milk concentrate" means any liquid or dried dairy-based concentrate comprising milk, skim milk, or milk proteins. Dry dairy components include without limitation, for example, whole dry milk, non-fat dry milk, low fat milk powder, whole milk powder, dry whey solids, demineralized whey powders, individual whey protein, casein dairy powders, individual casein powders, anhydrous milk fat, dried cream, lactose free dairy powder, dry lactose derivatives, reduced sodium dairy powder, etc. Also included are calorie-free dairy, cholesterol free dairy, low calorie dairy, low cholesterol dairy, light dairy, etc. Also included are combinations of any of the above liquid or dry dairy components in any ratio.

Containers of various sizes, shapes, heights, and diameters can be used in the methods of the present invention as long as they have at least one opening through which a UV device or a UV lamp can be introduced.

In some embodiments, a container (tank) capacity is selected from the group consisting of at least about 5,000 gallons, at least about 6,000 gallons, at least about 10,000 gallons, at least about 15,000 gallons, at least about 20,000 gallons, at least about 25,000 gallons, at least about 50,000 gallons, at least about 75,000 gallons, at least about 100,000 gallons, at least about 125,000 gallons, at least about 150,000 gallons, at least about 175,000 gallons, at least about 200,000 gallons, at least about 225,000 gallons, at least about 250,000 gallons, at least about 300,000 gallons, at least about 350,000 gallons, at least about 400,000 gallons, at least about 450,000 gallons, at least about 500,000 gallons. In some embodiments, a container to be sanitized has a capacity of from about 100,000 gallons to about 500,000 gallons. In some embodiments, a container to be sanitized has a capacity of from about 200,000 gallons to about 500,000 gallons. In some embodiments, a container to be sanitized has a capacity of from about 300,000 gallons to about 500,000 gallons. Individual tank capacities are described in detail in the Examples.

Containers of various refractive indexes can be used in the methods of the present invention.

In some embodiments of the present invention, the interior surface of a container is UV reflective.

In some embodiments of the present invention, the interior surface of a container is stainless steel.

Typically, a container for use in a method of the present invention is a closed container with one or more openings at the top (*e*.*g*., *see* FIGS. 23 and 25), at a side wall (*e*.*g*., *see* FIGS. 8-11 and 15), or at the bottom part of a side wall (*e*.*g*., *see* FIGS. 8-11 and 15). In some embodiments, this opening is referred to as manhole and is shown in, *e*.*g*., FIGS 8-11, 15, 23 and 25. The manhole or port **48** provides access to the container **49** from the top of the container **49** and further allows, *e.g.*, for the attachment of various pressure washing devices. The manhole or port **48** also allows the positioning of a UV device for practicing a method of the invention. As shown in FIGS. 8-11, 15, 23, and 25, part of the UV device rests at or on top of the manhole or port **48** when the UV device is used for the UV sterilization of the container **49**..

In some embodiments, a means for attaching the UV device to a container **49**, attaches the UV device to the manhole or port **48**. This attachment is typically done using a hanger or a mounting bracket **21** (FIGS. 8-11, 15, 23, and 25).

In some embodiments, the means for attaching the UV device to a container, attaches the UV device to an opening at a side of a container. This attachment is typically done using a hanger, more specifically, using a mounting bracket **21** (*e*.*g*., *see*, FIGS. 8-11, 15).

In some embodiments of the present invention, a container comprises a lid. In some embodiments of the present invention, a container comprises a hinged lid. The lid itself may have one or more openings through which a UV device or parts thereof (such as the UV light source) may be inserted inwardly into the container. When a lid is present, upon beginning the UV sterilization process, the lid is closed so to not expose a practitioner or any other person to the UV light. If a lid cannot be completely closed because, *e.g.*, the attachment or placement of a UV device at an opening of the container, a protective shield can be used to prevent UV light from escaping the container.

In some embodiments of the present invention, a container is a container used in zymurgy or the production of an alcoholic beverage. A UV device of the present invention may be used in any large scale commercial steel vessel involved in the fermentation and production of an alcoholic beverage. The term "alcoholic beverage" is used to include the alcoholic beverage prescribed in Liquor Tax Law Chapter 1, Section 2.

A fermentation container may be of various size, shape, height, and can be used in a method of the present invention as long as it has at least one opening through which a UV device or UV lamp can be introduced.

A fermentation container may be made of a variety of materials, including stainless steel, wood, plastic, concrete, a polymer, or glass. A preferred fermentation container is made of wood.

### B. Room, Space Or Defined Environment

In some embodiments, a room, space or defined environment is exposed to UV radiation. A room, space or defined environment accepts a UV light source for the purpose of sterilization of the interior of the room, space or defined environment, including any and all objects, fluids, materials, and surfaces contained within the interior of the room, space or defined environment. In some embodiments, the objects, fluids, materials, and surfaces within the interior of the room, space or defined environment are contained within the room, space or defined environment temporarily. In other embodiments, they are contained within the room, space, or defined environment permanently.

The meaning of a room, space or defined environment is not limited to an enclosed room having walls, a ceiling, a floor or other barriers, but rather includes spaces open to at least on side and any defined environment. As exemplified herein, in some embodiments, a room, a space or defined environment is selected from the group consisting of a commercial kitchen, a medical facility, an acute care area, an operating room, a medical equipment storage cabinet, a clean room, a bathroom, a food production area, a nursery home, a trailer, a truck, a wagon, a rail car, an airplane, a boat, a grocery store display case, and a deli counter.

Rooms, spaces or defined environments of various sizes, shapes, heights, and diameters can be used in the methods of the present invention as long as they have at least one opening through which a UV device or a UV light source can be introduced.

### IV. SYSTEMS

In another aspect of the present invention, systems comprising a UV device described herein, are provided. In some embodiments of the present invention, a system comprises a UV device. A UV device may include one or more components as described herein, *e*.*g*., a germicidal UV light source, a detector, a housing, a range-finding device, a bracket, an optical component, a circuit board, a frame, an upper frame, a lower frame, a UV sensor, one or more hinges (pivots) and/or a motorized unit.

### A. System Comprising A UV Device And A Container

In some embodiments of the present invention, a system comprises (i) a UV device as described herein and (ii) a container as described herein. In some embodiments, the container of such a system is selected from the group consisting of a container for fermenting an alcoholic beverage, a container for storing or transporting a dairy product, a liquid dairy, a liquid dairy composition or a dry dairy composition; a container for water, milk, coffee, tea, juice, or a carbonated beverage; and a container for a biological fluid. In some embodiments, the container of such a system comprises wood, plastic, concrete, a polymer, etched aluminum, foil aluminum, polished aluminum, chromium, glass, nickel, silver, stainless steel, tri-plated steel, water paint, white cotton, white oil paint, white paper, white porcelain, white wall plaster or a fabric.

### B. System Comprising A UV Device And A Room, Space Or Defined Environment

In some embodiments of the present invention, a system comprises (i) a UV device as described herein and (ii) a room, space or defined environment as described herein.

### C. System Comprising A UV Device And A Container, Room, Space Or Defined Environment

In some embodiments of the present invention, a system comprises (i) a UV device as described herein and (ii) a container, room, space or defined environment as described herein.

### D. System Comprising A UV Device And A Control Box

In some embodiments of the present invention, a system comprises (i) a UV device as described herein and (ii) a control box **1** as described herein.

### E. System Comprising A UV Device And A Case

In some embodiments of the present invention, a system comprises (i) a UV device as described herein and (ii) a case, as described herein.

In some embodiments of the present invention, a system comprises (i) a UV device, and (ii) a case **7**, wherein, the UV device, when not in use, resides within the case **7**.

In some embodiment, the case **7** is attached to a control box **1**. In some embodiments a lower surface of the case **7** is attached to an upper surface of the control box **1** so that the case **7** resides on top of the control box **1**. In some embodiments and for easy maneuvering cart wheels **12** may be attached to the control box **1**. In some embodiments and for easy maneuvering one or more handrails **8** may be attached to the control box **1**. A system comprising (i) a UV device (residing in a case and operatively attached or attachable to a control box **1**) and (ii) a case 7 is shown, *e.g.*, in FIGS. 1-3.

For transportation, a system comprising (i) a UV device (residing in a case and operatively attached or attachable to a control box **1**), and a (ii) a case **7** may be strapped to a transportation rack **10**. Thus, in some embodiments of the present invention, a system comprises (i) a UV device, (ii) a case **7**, wherein, the UV device, when not in use, resides within the case **7**, and (iii) a transportation rack **10**.

The transportation rack **10** is configured to accommodate the control box **1** and case **7** for transportation. In some embodiments, a transportation rack comprises a plurality of fastening brackets **9**. The fastening brackets **9** comprise an opening through which fastenings **11** can be guided through to allow fastening of the control box **1** and case **7** to the transportation rack **10**. A system comprising a UV device (residing in a case), a case **7**, a control box **1** and a transportation rack **10**, is shown, *e.g.*, in FIGS. 1 and 2.

In some embodiments of the present invention, a system is for use in a method for ultraviolet (UV) sterilization of an interior surface of a container. In other embodiments of the present invention, a system is for use in a method for ultraviolet (UV) sterilization of a room, a space or a defined environment.

In some embodiments of the present invention, a system is for use in a method for inhibiting the growth of one or more species of microorganisms present in a container, preferably for inhibiting the growth of one or more species of microorganisms present on an interior surface of a container. In other embodiments of the present invention, a system is for use in a method for inhibiting the growth of one or more species of microorganisms present in a room, a space or a defined environment, preferably for inhibiting the growth of one or more species of microorganisms present on an interior surface of a room, a space or a defined environment.

### V. METHODS OF USE

In another aspect of the present invention, methods of using a UV device described herein, are provided. In some embodiments, a method of using a UV device is a method for ultraviolet (UV) sterilization of an interior surface of a container, a room, space or defined environment. In some embodiments, the method for UV sterilization of an interior surface of a container, room, space or defined environment comprises the steps of movably and inwardly inserting through an opening of the container, room, space or defined environment a germicidal UV light source of a UV device of the present invention and activating the germicidal UV light source.

In some embodiments, the method for UV sterilization of an interior surface of a container, room, space or defined environment further comprises the step of providing a container, room, space or defined environment having an opening through which a UV light source can be inserted.

In some embodiments, as described herein, the method for UV sterilization of an interior surface of a container, room, space or defined environment further comprises the step of moving the germicidal UV light source to a first vertical position within the container, room, space or defined environment. In some embodiments, as described herein, the method further comprises the step of moving the germicidal UV light source from the first vertical position to a second vertical position within the container, room, space or defined environment. Preferably, the movement is downwardly, however, depending on the UV device employed for practicing a method, the movement can also be upwardly into a first vertical position and then into a second vertical position. For example, although UV devices UV6K and UV2K are preferably descended from the top of a container into a first vertical position and then second vertical position, they can also be pulled upwardly into a first and a second vertical position.

In some embodiments, as described herein, the method for UV sterilization of an interior surface of a container, room, space or defined environment further comprises the step of moving the germicidal UV light source to a first horizontal position within the container, room, space or defined environment. In some embodiments, as described herein, the method further comprises the step of moving the germicidal UV light source from the first horizontal position to a second horizontal position within the container, room, space or defined environment.

In some embodiments, the method further comprises the step of moving the germicidal UV light source from a horizontal position to a vertical position within the container, room, space or defined environment. In some embodiments, the method further comprises the step of moving the germicidal UV light source from a vertical position to a horizontal position within the container, room, space or defined environment.

.In some embodiments, as described herein, the method for UV sterilization of an interior surface of a container, room, space or defined environment comprises the step of positioning a UV device on a bottom surface of a container, room, space or defined environment. In some embodiments, the method for UV sterilization of an interior surface of a container, room, space or defined environment further comprises the step of moving a UV device on a bottom surface of a container, room, space or defined environment from a first position to a second position.

In some embodiments, the method further comprises the step of attaching a UV device comprising the germicidal UV light source to the container, room, space or defined environment. Preferably, the attachment is at an opening at the container, room, space or defined environment. An opening at a container, room, space or defined environment can be on top of the container, room, space or defined environment, at a side wall of the container, room, space or defined environment or at a bottom part of a side wall of the container, room, space or defined environment.

In some embodiments, the method further comprises the step of movably positioning a UV device comprising the germicidal UV light source in a container, room, space or defined environment. Preferably, movably positioning a UV device in a container, room, space or defined environment comprises moving a UV device trough an opening into the container, room, space or defined environment. An opening at a container, room, space or defined environment can be on top of the container, room, space or defined environment at a side wall of the container, room, space or defined environment or at a bottom part of a side wall of the container, room, space or defined environment. The positioning of the UV device within the container, room, space or defined environment may be on the floor of the container, room, space or defined environment.

In some embodiments, a method of using a UV device is a method for inhibiting the growth of one or more microorganisms present on an interior surface of a container, room, space or defined environment. In some embodiments, the method for inhibiting the growth of one or more microorganisms present on an interior surface of a container, room, space or defined environment comprises the steps of movably and inwardly inserting through the opening of a container, room, space or defined environment a germicidal UV light source and activating the germicidal UV light.

In some embodiments, as described herein, the method for inhibiting the growth of one or more microorganisms present on an interior surface of a container, room, space or defined environment further comprises the step of providing a container, room, space or defined environment having an opening through which the UV light source can be inserted.

In some embodiments, as described herein, the method for inhibiting the growth of one or more microorganisms present on an interior surface of a container, room, space or defined environment further comprises the step of moving the germicidal UV light source to a certain position within the container, room, space or defined environment. Such movements and positioning of a UV light source have been described *supra* and can also be used in the method for inhibiting the growth of one or more microorganisms present on an interior surface of a container, room, space or defined environment. Similarly, methods of attaching a UV device to a container, room, space or defined environment, as described supra, can be used in those methods.

### A. Providing A Container, Room, Space Or Defined Environment

In some embodiments, a method for UV sterilization of an interior surface of a container, room, space or defined environment comprises the step of providing a container, room, space or defined environment having an opening. In some embodiments, a method for inhibiting the growth of one or more microorganisms present on an interior surface of a container, room, space or defined environment comprises the step of providing a container, room, space or defined environment having an opening. Containers, rooms, spaces or defined environments useful for practicing methods of the present invention are described herein.

### B. Attaching A UV Device To A Container, Room, Space Or Defined Environment

In some embodiments, a method of the present invention comprises the step of attaching a UV device to a container, room, space or defined environment. Attaching a UV device temporarily, for a prolonged time, or permanently to a container, room, space or defined environment is described herein. An exemplary embodiment of attaching a UV device to an opening at a side wall of a container is shown in FIGS. 11, 23 and 25. Thereby, a UV device is attached firmly and temporarily, *e.g.*, for the duration of a sanitization cycle positioned to an opening of the container and is restricted from moving.

### C. Movably And Inwardly Inserting A UV Light Source Into A Container, Room, Space Or Defined Environment

In some embodiments, a method of the present invention comprises the step of movably and inwardly inserting a germicidal UV light source through an opening of a container, room, space or defined environment. The opening of the container, room, space or defined environment may be on top of the container, room, space or defined environment (*e.g.*, FIGS. 23 and 25). Alternatively, an opening of the container, room, space or defined environment may also be at the bottom of a container, room, space or defined environment or at a side of a container, room, space or defined environment (*e.g.*, FIGS. 8-11).

One of skill in the art reading the instant specification will appreciate that a UV light source can be movably and inwardly inserted into a container, room, space or defined environment through an opening on the top of the container, room, space or defined environment, through an opening at the bottom of the container, room, space or defined environment, or through an opening at a side of the container, room, space or defined environment. As described herein, a UV light source, once movably an inwardly inserted into a container, room, space or defined environment can be moved to any desired or predetermined position within the container, room, space or defined environment. One of ordinary skill in the art will appreciate that the methods described herein for positioning a UV light source within a container, room, space or defined environment can be easily modified to account for the point of where the UV light source is being movably inserted into a container, room, space or defined environment. Those would be considered design choices in view of the disclosure provided herewith.

In some embodiments, once the UV light source is movably and inwardly inserted into a container, room, space or defined environment, it remains in a stationary position for the time of the sterilization process. In some other embodiments, once the UV light source is movably and inwardly inserted into a container, room, space or defined environment, it is mobile. In some embodiments, a UV light source moves longitudinally within the container, room, space or defined environment. In some embodiments, a UV light source moves laterally. In some embodiments, a UV light source rotates on its own axis or about an axis. In some embodiments, a combination of movements of some or all movements is used to achieve the desired result of positioning a UV light source at a desired or predetermined position within a container, room, space or defined environment. The movement of a UV light source can be achieved through use of a motorized unit, use of a hydraulic system, manually, or a combination thereof.

Mobility of the UV light source may depend on the size and shape of the container, room, space or defined environment and on the size, shape, and intensity of the UV lamp(s). The use of a mobile UV light source will depend on the desired sterilization rate. If, for example, a faster rate is desired, the UV light source preferably is positioned closer to the inner surface of the container, room, space or defined environment to be sterilized. Thus, in this embodiment, a means by which the UV light source is positioned in closer proximity to the inner surface is recommended. Similarly, in some embodiments, the positioning of the UV light source is altered to avoid an obstruction, such as an internally mounted thermometer or the like. As one of skill in the art will appreciate, the longitudinal movement of a UV light source depends on the height of the vessel. Further, the lateral movement of a UV light source depends on the diameter of the container. In embodiments where a rotating UV light source is used, the rate of rotation will depend on the type of UV lamp used (continuous UVC vs. pulsed UV) and on the intensity of the UV lamp.

### D. Activating And Deactivating A UV Light Source

In some embodiments, a method of the present invention comprises the step of activating a germicidal UV light source. Thereby a necessary or predetermined dose of radiation will be delivered. Activating of the UV light source initiates the process of sterilization, disinfection and growth inhibition of the one or more microorganisms by providing a UV dose for effective sterilization of microorganisms, disinfection of the interior surface of a container, room, space or defined environment and for the growth inhibition of the one or more microorganisms.

In some embodiments, a method of the present invention comprises the step of manually activating a germicidal UV light source. In some embodiments, a UV device comprises an on/off/reset switch for manually activating the germicidal UV light source. In some embodiments, a UV light source is connected to an external control box **1** comprising an on/off/reset switch **3** for manually activating the germicidal UV light source.

In some embodiments of the present invention, a UV device comprises an interface for activating the UV device, for inactivating the UV device, for making a user aware of the time elapsed in a sterilization cycle and/or making a user aware of the time remaining for completion of a sterilization cycle. Some interface function may be operatively connected to a visual or audible alert or to an email notification, telephonic contacting or texting. In some embodiments, a UV device is operatively connected to an external control box **1** comprising a touchscreen interface **5** adapted to provide input for functionalities as described herein.

In some embodiments, activation of the UV light source occurs at a predetermined time and may be controlled by an RFID communicating with a circuit board attached to the UV device.

In some embodiments, activation of the UV light source occurs for a predetermined time required to deliver a predetermined UV dose/UV intensity. Preferably the duration of the activation of the UV light source is provided for delivering a UV dose/UV intensity to cause an at least about 1 log reduction of microorganisms on the interior surface of a container, room, space or defined environment, an at least about 2 log reduction of one or more microorganisms on the interior surface of a container, room, space or defined environment, an at least about 3 log reduction of one or more microorganisms on the interior surface of a container, room, space or defined environment, an at least about 4 log reduction of one or more microorganisms on the interior surface of a container, room, space or defined environment, an at least about 5 log reduction of one or more microorganisms on the interior surface of a container, room, space or defined environment, or an at least about 6 log reduction of one or more microorganisms on the interior surface of a container, room, space or defined environment.

By inserting a UV light source into the interior of a container, room, space or defined environment and by activating the UV light source, the interior surface of the container, room, space or defined environment is exposed to a UV light dose. In some embodiments, the UV light dose is measured by a UV sensor **17**, as described herein. Data measured by the UV sensor are relayed to the control box **1** and may be shown on the touchscreen interface **5**.

Once the desired UV intensity has been applied to the interior surface of a container, room, space or defined environment, the UV light source may be deactivated. In some embodiments, deactivation is performed by a timer, which can be set to different times depending on the desired log reduction of the desired microorganisms (see calculations of killing rates in Example B). Deactivation can also be performed by a UV detector (or UV sensor **17**), which will automatically shut off the UV lamp(s) when the desired UV intensity has been attained and measured. In some embodiments of the present invention, deactivation may also be controlled by a RFID. In some embodiments of the present invention, deactivation, upon completing a sterilization cycle, is controlled by a circuit board attached to the UV device or by a circuit board residing in an external control box **1**. Again, the desired UV intensity will depend on the desired log reduction of the desired microorganisms. For example, using a UV lamp with an output of 190 microwatts/cm² at 254 nm (at a distance of 1 meter), placed within a fermentation vessel 60" from the interior surface, if a 2 log reduction of *Shigella dysentery* is desired, 4,200 microwatt seconds/cm² would be required. Once the UV detector has detected that 4,200 microwatt seconds/cm² have been attained it would automatically shut off the UV lamp. Thus, in some embodiments, the method for UV sterilization of an interior surface of a container, room, space or defined environment comprises the step deactivating a germicidal UV light source. As described herein, deactivation may occur automatically by using a preset UV detector. Alternatively, deactivation is performed manually. In some embodiments, a UV device comprises an on/off/reset switch for manually deactivating the germicidal UV light source. In some embodiments, a UV light source is connected to an external control box **1** comprising an on/off/reset switch **3** for manually deactivating the germicidal UV light source.

In some embodiments, the process of sterilizing the interior of a container, room, space or defined environment comprises the step of subjecting the interior of the container, room, space or defined environment to UV radiation.

While typically a single exposure of an interior surface of a container, room, space or defined environment by a necessary or predetermined UV dose is sufficient to achieve a desired log reduction of microorganisms, in some embodiments, the interior surface of the container is exposed multiple times to UV radiation.

Short-wave UV light is harmful to humans. In addition to causing sunburn and (over time) skin cancer, UV light can produce extremely painful inflammation of the cornea of the eye, which may lead to temporary or permanent vision impairment. It can also damage the retina of the eye. For this reason, the light produced by a germicidal UV lamp must be carefully shielded against both direct viewing and reflections and dispersed light that might be viewed. Thus, in some embodiments of the present invention, the methods of sterilization a container, room, space or defined environment and methods for inhibiting the growth of one or more microorganisms present on an interior surface of a container, room, space or defined environment comprise the step of covering the opening of the container through which the germicidal UV light source has been inserted with a lid, top, or cover. The lid, top or cover essentially does not allow the UV light to penetrate and thus, protects humans from the harmful UV light.

### E. Releasing A Germicidal UV Light Source From A Housing

In some embodiments, a method of the present invention comprises the step of releasing a germicidal UV light source from a housing. Thereby a germicidal UV light source, *e.g.,* a UV lamp, is released from a housing. In some embodiments, the releasing of the germicidal UV light source from the housing is accomplished by a motorized unit. The motorized unit may be operatively connected to a rope, cable or wire **22**, which is connected to a UV lamp **16** and thus, can move the UV lamp **16** in an downward direction for use and moves the UV lamp **16** in an upward direction after use. Alternatively, depending on the UV device being used, the UV lamp **15** is moved in an upwardly direction for use and in a downwardly direction after use.

As one of ordinary skill in the art will appreciate, releasing a germicidal UV light source from a housing is only necessary in the methods of the present invention, wherein the housing is not UV light permissible, i.e., wherein the housing is made of a material which does not allow UV light to penetrate through. In some UV devices of the present invention, the UV light source resides within a housing made of a material that permits UV light to pass through. The UV light source of such UV devices does not need to be released from its housing for use in a method of the present invention. For example, members of the UVT-4, UV6K and UV2K families of UV devices comprise a housing made of a material allowing UV light to pass through even when the housing fully encases the UV light source.

### F. Placing A UV Device At Or On An Upper Perimeter Of A Container

In some embodiments, a method of the present invention comprises the step of placing a UV device comprising a bracket **21** to which the germicidal UV light source is operatively attached on the upper perimeter of a container **49**. Thereby the UV device comprising the UV light source is firmly positioned on the upper perimeter of the container **49** is restricted from moving downwards due to the bracket **21**. An exemplary placing of a bracket **21** to which the germicidal UV light source is operatively attached on the upper perimeter of a container is shown in FIGS. 23 and 25. While the bracket **21** is firmly placed on the upper perimeter of a container, as shown in FIGS. 23 and 25 other parts of the UV device can be moved downwards into the container **49**. Other UV devices, such as members of the UVT-4 family can be operatively attached to an opening at a side wall of a container **49** via a bracket **21** (*e.g.,* FIGS. 8-11)

### G. Movably And Inwardly Inserting A Second Germicidal UV Light Source Through An Opening Of A Container, Or Into A Room, Space Or Defined Environment

In some embodiments, a method of the present invention comprises the step of movably and inwardly inserting through an opening of a container, into a room, space or defined environment a second germicidal UV light source. The second germicidal UV light source can be inserted similarly as the first germicidal light source or differently. Insertion of the second germicidal UV light source can be simultaneously with insertion of the first germicidal light source or subsequently. In embodiments comprising a member of the UVT-4 family of UV devices, wherein at least one first germicidal light source is operatively connected to an upper frame and wherein at least one second UV light source is operatively connected to a lower frame and wherein the lower frame and upper frame are operatively connected, both germicidal UV light sources are inserted simultaneously into the container, room, space or defined environment. In some embodiments, the second germicidal light source differs from the first germicidal light source in dimension and/or intensity.

### H. Moving A Germicidal UV Light Source To A First Vertical Position Within A Container, Room, Space Or Defined Environment

In some embodiments, a method of the present invention comprises the step of moving a germicidal UV light source to a first vertical position within a container, a room, space or defined environment. Moving a germicidal UV light source to a first vertical position within a container, a room or a defined environment is described herein.

### I. Moving A Germicidal UV Light Source From A First Vertical Position To A Second Vertical Position Within A Container, Room, Space Or Defined Environment

In some embodiments, a method of the present invention comprises the step of moving a germicidal UV light source from a first vertical position to a second vertical position within a container, a room, space or defined environment. As one of ordinary skill in the art will appreciate, moving a germicidal UV light source from a first vertical position to a second vertical position within a container, a room, space or defined environment, comprises moving the UV device either downwards or upwards into the second vertical position. As one of ordinary skill in the art will further appreciate, moving a germicidal UV light source from a first vertical position to a second vertical position within a container, a room, space or defined environment, comprises moving the UV light source in increments of inches or centimeters between the first vertical position and the second vertical position. Such movement can be terminated at any desired vertical position between the first vertical position and the second vertical position. Moving a germicidal UV light source from a first vertical position to a a second vertical position within a container, a room, space or defined environment is described herein. During the movement, the UV light source maybe in operation, i.e., turned on. Alternatively, during the movement, the UV light source may not be in operation, i.e., turned off.

### J. Moving A Germicidal UV Light Source From A First Vertical Position To A Horizontal Position Within A Container, Room, Space Or Defined Environment

In some embodiments, a method of the present invention comprises the step of moving a germicidal UV light source from a first vertical position to a horizontal position within a container, a room, space or defined environment. As one of ordinary skill in the art will appreciate, moving a germicidal UV light source from a first vertical position to a horizontal position within a container, a room, space or defined environment, comprises moving the UV device through angular positions between the first vertical position and the horizontal position. Such movement can be terminated at any desired angular position between the first vertical position and the horizontal position. Moving a germicidal UV light source from a first vertical position to a horizontal position within a container, a room, space or defined environment is described herein. During the movement, the UV light source maybe in operation, i.e., turned on. Alternatively, during the movement, the UV light source may not be in operation, i.e., turned off.

### K. Moving A Germicidal UV Light Source From a Horizontal Position To A Vertical Position Within A Container. Room. Space Or Defined Environment

In some embodiments, a method of the present invention comprises the step of moving a germicidal UV light source from a horizontal position within a container, a room, space or defined environment to a vertical position within a container, a room, space or defined environment. As one of ordinary skill in the art will appreciate, moving a germicidal UV light source from a horizontal position to a vertical position within a container, a room, space defined environment, comprises moving the UV light source through angular positions between the horizontal position and the vertical position. Such movement can be terminated at any desired angular position between the horizontal position and the vertical position. Moving a germicidal UV light source from a horizontal position within a container, a room, space or defined environment to a vertical position within a container, a room, space defined environment is described herein. During the movement, the UV light source maybe in operation, i.e., turned on. Alternatively, during the movement, the UV light source may not be in operation, i.e., turned off.

### L. Moving A Germicidal UV Light Source From a First Horizontal Position To A Second Horizontal Position Within A Container. Room, Space Or Defined Environment

In some embodiments, a method of the present invention comprises the step of moving a germicidal UV light source from a first horizontal position within a container, a room, space or defined environment to a second horizontal position within the container, room, space or defined environment. As one of ordinary skill in the art will appreciate, moving a germicidal UV light source from a first horizontal position to a second horizontal position within a container, a room, space or a defined environment, comprises moving the UV light source in increments of inches or centimeters between the first horizontal position and the second horizontal position. Such movement can be terminated at any desired position between the first horizontal position and the second horizontal position. Moving a germicidal UV light source from a first horizontal position within a container to a second horizontal position within a container is described herein. During the movement, the UV light source maybe in operation, i.e., turned on. Alternatively, during the movement, the UV light source may not be in operation, i.e., turned off.

For example, it has been found that members of the UVT-4 family of UV devices are particular useful for sanitizing large containers, large rooms, large spaces and large defined environments. As described in the Examples, UV devices have been used to sterilize tanks having a capacity ranging from about 5,000 gallons to more than 200,000 gallons, ranging in diameters from several yards or meters to more than about ten yards or ten meters. Sometimes, those large containers do not have sufficient breathable air to permit a user of a UV device to crawl into such container and move the UV light source from a first position to a second position, either horizontally, vertically or angularly. While in some embodiments, motorized units are used to accomplish such movements, other embodiments provide for a simple manual use. In such embodiments, an extension tool is provided (FIG. 14). In some embodiments, an extension tool comprises an extension rod **56**, which can be of varying length. In some embodiments, the extension rod **56** is extendable by itself and the length of extension is locked in by a fastening mechanism. In some embodiments, the extension tool comprises a base plate **55**, having a front side and a back side (FIG. 14). In some embodiments, the extension rod **56** is attached to the back side of the base plate (FIG. 14). In some embodiments, a plurality of wheels **37** are operatively attached to the base plate **55** (FIG. 62). Once connected to the extension tool (see below), wheels **37** facilitate movement of a UV light source within a container, room, space or defined environment. In some embodiments, an extension tool further comprises a top plate **54** having an upper side and a lower side (FIG. 14). In some embodiments of an extension tool, the top plate **54** is attached to the base plate **55** in a perpendicular orientation. Other attachments are within the art.

FIG. 15 shows an exemplary attachment of an extension tool to a UV device of the UVT-4 family of UV devices. In this non-limiting example, the extension tool is connected to a means for attaching the UV device to an opening of a container, to a fixture in a room, space or defined environment. More specifically, in this non-limiting example, the extension tool is connected to a mounting bracket **21** attached to the UV device. As shown un FIG. 15, the mounting bracket **21** is configured to operatively attach the UV device to an opening **48** of *e.g.,* a container **49**, and is further configured to operatively connect with the extension tool. More specifically, the top plate **54** of the extension tool is attached to the mounting bracket **21** and fastened to it by the bracket tightening knob **26**. As further shown in FIG. 16, once the extension tool is attached to the mounting bracket **21**, the UV device is then positioned on the bottom of the large container **49** at a first horizontal position. This is made possible because of the second hinge **34**, which moveably and operatively connects the bracket **21** with the lower frame **23** of the UV device. By manually pushing the extension tool and facilitated by the wheels **37** attached to the UV device and wheels **37** attached to the extension tool, a user can easily move the UV device, and hence, the UV light source, from the first horizontal position to a second horizontal position within the container (or room, space or defined environment). To ensure that the vertical interior surfaces are treated with approximately the same UV dose, in some embodiments, the second horizontal position is in the middle of the container, room, space or defined environment. Once the sanitization cycle is complete, as one of ordinary skill in the art will appreciate, a user will pull back the extension tool and thereby move the UV device from that second horizontal position back to the first horizontal position for easy retrieval from the container, room, space or defined environment.

### M. Inhibiting Growth Of Microorganisms

In some embodiments of the present invention, a germicidal UV light source is used to inhibit the growth of a microorganism or inhibit the growth of one or more microorganisms. The terms "inhibiting the growth of microorganisms," "growth arresting microorganisms," "reducing microorganisms," "killing microorganisms," or grammatically equivalents are used interchangeably herein.

In some embodiments of the present invention, a microorganism is a yeast species. The following provides a non-exhaustive list of yeast species that are typically found in a fermentation container, and more specifically on an interior surface of a fermentation container. Yeast species that have been investigated for wine and beer production include those from the *Candida, Kloeckera*, *Hanseniaspora*, *Zygosaccharomyces*, *Schizosaccharomyces*, *Torulaspora*, *Brettanomyces*, *Pichia*, *Hansenula*, *Metschnikowia*, *Torulespora*, *Debaryomyces*, *Saccharrmycodes* (species *ludwigii*), and *Williopsis* genera. Cultured yeast species include *Saccharomyces cerevisiae* and *Saccharomyces bayanus.* The growth of non-*Saccharomyces* yeast in wine production is also being investigated and can be inhibited. Thus, in some embodiments, it is particularly desirable to inhibit the growth of a yeast species using a method of the present invention. For example, 17,600 µWs/cm² is necessary for a 2 log killing of *Sacchahhmycodes* and 6,600 µWs/cm² for a 2 log killing of Brewer's yeast. UV intensities required for sterilization for unknown microorganism species can be determined by one of skill in the art using methods known in the art and described herein, including the algorithm provided herein.

Some of the microorganisms found in a container, a room, space or defined environment, more specifically, on an interior surface of a container, a room,, space or defined environment, are pathogenic. In some embodiments of the present invention, a microorganism is a pathogenic microorganism. Those microorganisms include, but are not limited to, *Escherichia coli*, *Corynebacterium diphtheria*, *Salmonella paratyphi* (causing enteric fever), *Salmonella typhosa* (causing typhoid fever), *Shigella dysenteriae* (causing dysentery), *Shigella flexerni* (causing dysentery), *Staphylococcus albus*, *Staphylococcus aureus*, *Streptococcus hemolyticus*, *Streptococcus lactis*, *Streptococcus viridians* and *Vibrio comma* (causing cholera). Thus, in some embodiments, it is particularly desirable to inhibit the growth of a pathogenic microorganism using a method of the present invention.

Other microorganisms found in a fermentation container, more specifically on an interior surface of a fermentation container, are detrimental in the production of a fermented beverage. Those microorganisms include, but are not limited to, *Brettanomyces (Dekkera),* lactic acid bacteria, *Pediococcus*, *Lactobacillus*, and *Oenococcus. Brettanomyces* species include *B. abstinens*, *B. anomalus*, *B. bruxellensis*, *B. claussenii*, *B. custersianus*, *B. custersii*, *B. intermedius*, *B. lambicus*, and *B. naardensis.* The genus *Dekkera* (the perfect form of *Brettanomyces,* meaning it can sporulate), *includes the species D. bruxellensis and D. intermedius.* Those microorganisms may also be found in a room, space, or defined environment. Thus, in some embodiments, it is particularly desirable to inhibit the growth of a microorganism, which is detrimental in the production of a fermented beverage, using a method of the present invention.

Other microorganisms found in a fermentation container, more specifically on an interior surface of a fermentation container, that are detrimental in the production of a fermented beverage are bacterial microorganisms. Bacteria genus include, but are not limited to, *Acetobacter*, *Lactobacillus*, *Pediococcus*, and *Leuconostoc. Acetobacter* species include, *e.g.*, *A. aceti*, *A. hansennii*, *A. liquefaciens*, and *A. pasteurienus. Lactobacillus* species (ML bacteria, spoilage) include, *e.g.*, *L. fructivorans* and others. *Pediococcus* species (ML bacteria, spoilage) include, *e.g.*, *P. damnosus* and others. *Leuconostoc* species (ML bacteria) include, *e.g.*, *L.* o and others. Those microorganisms may also be found in a room, space, or defined environment. Thus, in some embodiments, it is particularly desirable to inhibit the growth of a bacterial microorganism using a method of the present invention.

### 1. Duration of Sterilization

The duration of sterilization, i.e., the time of activating a UV light source and applying the calculated UV dosage/UV intensity, determines the percentage of how many microorganisms are growth arrested or killed. As one of skill in the art will appreciate and as described herein (*see*, Algorithm), the duration of a sterilization cycle is based on the power output of the UV lamp and the distance of the UV light source from the walls and surfaces of the container, room, space or defined environment to be sterilized.

In some embodiments, an algorithm is used (and hence, the duration of sterilization is performed for a time) to ensure that at least 90% of the microorganisms present on the surface of a container, room, space or defined environment are growth arrested or killed. One of skill in art will appreciate that a 90% growth arrest of microorganisms corresponds to a 1 log reduction.

In some embodiments, an algorithm is used (and hence, the duration of sterilization is performed for a time) to ensure that at least 99% of the microorganisms present on the surface of a container, room, space or defined environment are growth arrested or killed. One of skill in art will appreciate that a 99% growth arrest of microorganisms corresponds to a 2 log reduction.

In some embodiments, an algorithm is used (and hence, the duration of sterilization is performed for a time) to ensure that at least 99.9% of the microorganisms present on the surface of a container, room, space or defined environment are growth arrested or killed. One of skill in art will appreciate that a 99.9% growth arrest of microorganisms corresponds to a 3 log reduction.

In some embodiments, an algorithm is used (and hence, the duration of sterilization is performed for a time) to ensure that at least 99.99% of the microorganisms present on the surface of a container, room, space or defined environment are growth arrested or killed. One of skill in art will appreciate that a 99.99% growth arrest of microorganisms corresponds to a 4 log reduction.

In some embodiments, an algorithm is used (and hence, the duration of sterilization is performed for a time) to ensure that at least 99.999% of the microorganisms present on the surface of a container, room, space or defined environment are growth arrested or killed. One of skill in art will appreciate that a 99.999% growth arrest of microorganisms corresponds to a 5 log reduction.

In some embodiments, an algorithm is used (and hence, the duration of sterilization is performed for a time) to ensure that at least 99.9999% of the microorganisms present on the surface of a container, room, space or defined environment are growth arrested or killed. One of skill in art will appreciate that a 99.9999% growth arrest of microorganisms corresponds to a 6 log reduction.

Examples 6 and 7, in particular, provide useful guidance for establishing an algorithm for sanitization of various containers. Examples 10 and 11 provide exemplary comparative studies of sanitization using a UV device of the present invention and other sanitization methods.

### N. Assessing Microbial Concentration

Microbial concentration on interior surfaces of containers, rooms, spaces or defined environments may be assessed before and after performing a method of the present invention, such as the UV disinfection and UV sterilization methods described herein. A lower microbial concentration on interior surfaces of a container, room, space or defined environment after having performed a method of the present invention, *e.g.*, performing a UV disinfection or UV sterilization method evidences the effectiveness of the method used. Methods for assessing microbial concentration are known in the art. Exemplary methods are described herein.

### M. Sanitization Of A Room, Space Or Defined Environment

In some embodiments, a UV device, preferably a UV light source, more preferably a germicidal UV light source, is used to sanitize a room, a space or a defined environment. Some aspects of sanitization of a room, space or defined environment have been described above already. Some specific aspects of sanitization of a room, space or defined environment are set forth below.

The terms "sanitization" or "sanitation" and "UV sterilization" and grammatical equivalents thereof are used interchangeably herein. The meaning of a room is not limited to an enclosed room having walls, a ceiling, a floor or other barriers, but rather includes spaces open to at least on side and any defined environment. As exemplified herein, in some embodiments, a room, a space or defined environment is selected from the group consisting of a commercial kitchen, a medical facility, an acute care area, an operating room, a medical equipment storage cabinet, a clean room, a bathroom, a food production area, a nursery home, a trailer, a truck, a wagon, a rail car, an airplane, a boat, a grocery store display case, and a deli counter.

Thus, the present invention provides methods for sanitization (UV sterilization) of a room, space or defined environment. In some embodiments of this method, the method comprises the step of providing a room, space or defined environment in need of sanitization and exposing the room, space or defined environment to ultraviolet (UV) sterilization using a UV device described herein. In some embodiments of this method, the method comprises the step of selecting a room, a space or a defined environment in need of sanitization and exposing the room, the space or the defined environment to ultraviolet (UV) sterilization using a UV device. Suitable UV devices, *e.g.*, UV devices of the UVT-4, UV6K and UV2K families are described herein. Some embodiments of the method for sanitizing a room, space or defined environment comprise the step of attaching a UV device to a fixture within the room, space or defined environment. Some embodiments of the method for sanitizing a room, space or defined environment comprise the step of attaching a UV device to a wall within the room, space or defined environment. Some embodiments of the method for sanitizing a room, space or defined environment comprise the step of attaching a UV device to a ceiling of the room, space or defined environment. Some embodiments of the method for sanitizing a room, space or defined environment comprise the step of attaching a UV device to an object or structure present in the room, space or defined environment. Objects or structures to which a UV device can be attached include, but are not limited to, a conveyer belt, a hood, a cabinet, a display case, etc. A UV device can also be superimposed over or attached to a preexisting light fixture.

Some embodiments of the method for sanitizing a room, space or defined environment comprise the step of moving a UV light source from a closed position to an exposed position. Some embodiments of the method for sanitizing a room, space or defined environment comprise the step of moving a UV light source from a first position to a second position, as described herein.

Some embodiments of the method for sanitizing a room, space or defined environment comprise the step of activating the UV light source.

In some embodiments of a UV device being used for the sanitization of a room, space or defined environment, a portable UV device is used. In some embodiments, an RFID tag is mounted to a doorway of a room, space or defined environment intended to be sanitized. In some embodiments, an RFID tag reader is mounted to the UV device, such that when the UV device is brought into the room, space or defined environment, the tag is read. Information on the tag includes, but is not limited to, dimension and type of the room, space or defined environment, and a desired log reduction. This information is uploaded into the UV device and a sanitization cycle is preprogrammed.

As one of ordinary skill in the art will appreciate some embodiments of the method for sanitizing a room, space or defined environment comprise steps described herein for the sanitization (UV sterilization) of a container or interior surface of a container. Those steps are described in detail herein and one of ordinary skill in the art can easily adapt those steps for the use in the method for sanitizing a room, space or defined environment.

In some embodiments, a room, space or defined environment is exposed to UV radiation. It is to be understood that the invention can be applied to any defined environment. For example, an environment may be defined by solid surfaces or barriers, such as a wall or product packaging.

A room, a space or a defined environment accepts a UV light source for the purpose of sterilization of a wall, a ceiling or a floor, including any and all objects, fluids, materials, and surfaces contained within the room, space or defined environment. In some embodiments, the objects, fluids, materials, and surfaces within the room, space or defined environment are contained within the room, space or defined environment temporarily. In other embodiments, they are contained within the room, space or defined environment permanently.

The present invention provides for the sanitization of a variety of rooms, spaces or defined environments. Rooms, spaces or defined environments include, but are not limited to a commercial kitchen, an operating room, a clean room (ISO 1 - ISO 9), a food production area, a nursery home. An exemplary application of a UV device described herein would be for sanitizing a sensitive area of a medical facility, such as an acute care area or an operating room. Other areas in a medical facility that can be sanitized using a UV device described herein include a waiting room, a bathroom, and a medical equipment storage cabinet.

A UV device described herein may also be configured into a food processing equipment so that food is treated as it moves through the equipment, for example on a conveyor belt, automatic cutters and slicers and inspection areas. The product may be tumbled to promote uniform treatment. The UV device may also be configured to be placed in containers, trailers, cars, trucks, rail cars, airplanes or as a component to a refrigeration system of such containers, trailers, cars, trucks, rail cars and airplanes to sanitize the air therein while providing the beneficial preservative effects of ozone to any products stored therein.

Other exemplary applications of a UV device described herein include the provision or incorporation of the UV device into grocery store display cases, such as deli counters and meat, fish and poultry display cases and floral display cases, both refrigerated and non-refrigerated.

Still other examples of areas that can be sanitized with a UV device described herein include parcels, packages, and envelopes, also when moving on a conveyor belt. The parcels, packages, and envelopes may be tumbled or turned to promote uniform treatment.

A UV device described herein may be configured for general room sanitization, space sanitization or defined environment sanitization applications wherein the UV device, or components thereof, may be placed on a moving part, either permanently or temporarily during the sanitization procedure. In some embodiments, a moving part comprises a motorized unit. In some embodiments, a moving part comprises a railing system to which a UV device is movably and operatively attached, either temporarily or permanently. The railing system then determines the movement of the UV light source within the room, space or defined environment. In some embodiments, a railing system is attached to a ceiling of the room, space or defined environment.

In some embodiments of a UV device, when used for sanitization of a room, space or defined environment, the UV device comprises a range finding device to determine the size of the room, space or defined environment to be sanitized. The range-finder then provides information to preprogram an effective sanitization cycle.

In some embodiments of a UV device, when used for sanitization of a room, space or defined environment, a multi bulb UV cluster extends from the ceiling of a room, space or defined environment with the UV light sources extending at varying angles to optimize coverage and UV exposure of the room, space or defined environment.

In some embodiments of a UV device, when used for sanitization of a room, space or defined environment, a multi lamp UV cluster extends from the ceiling of a room, space or defined environment with individual UV light sources extending downwards independently at varying angles.

In some embodiments of a UV device, when used for sanitization of a room, space or defined environment, the UV lamp cluster and an optional housing are permanently and operatively attached to either a wall, a floor, or a ceiling of the room, space or defined environment.

In some embodiments of a UV device, when used for sanitization of a room, space or defined environment, the UV lamp cluster is operatively attached via a fixture to the ceiling of the room, space or defined environment. The fixture may be permanently attached and the UV bulb cluster and housing may be removable.

In some embodiments of a UV device, when used for sanitization of a room, space or defined environment, the dimensions of the room, space or defined environment are preprogrammed into the UV device allowing the application of a UV intensity/UV dosage (timing of sanitization) to be optimized and the minimal necessary UV intensity/UV dosage required for sanitization to be reached while minimizing power use. Preferred is an approximately 3 log reduction of microorganism or more, determined as described herein.

In some embodiments of a UV device, when used for sanitization of a room, space or defined environment, the UV device is operatively linked to a motion detector. This may be helpful to ensure people and/or animals are absent from the room, space or defined environment prior to the beginning of the sanitization cycle. It will also be helpful for shutting off and deactivating the UV sterilization process if a person enters a room, space or defined environment while a UV sterilization process is in process.

In some embodiments of a UV device, when used for sanitization of a room, space or defined environment, multiple UV lamp clusters are spread throughout the room, space or defined environment. Based on the dimension and shape of the room, space or defined environment, the positioning of the UV lamps and angles are accounted for and this information is programmed into an algorithm allowing the timing of sanitization to be optimized and the minimal necessary UV intensity/UV dosage required for sanitation to be reached while minimizing power use. Preferred is an approximately 3 log reduction of microorganism or more, determined as described herein. The positioning of the UV light sources and angles can also be communicated via wireless technology. In some embodiments, a rangefinder analyzes the shape and dimension of the room, space or defined environment and inputs that information into an algorithm allowing the timing of sanitization to be optimized and the minimal necessary UV dose required for sanitation to be reached while minimizing power use. Preferred is an approximately 3 log reduction of microorganisms or more, determined as described herein.

In some embodiments of a UV device, when used for sanitization of a room, space or defined environment, the UV light source is operatively attached to the bottom of a robot having wheels and follows programming allowing it to both perform an effective moving pattern on the floor covering desired areas. The robot may also have an object and wall avoiding programming and technology. The robot may move at a speed allowing an effective UV intensity/UV dosage required for sanitization to be reached while minimizing power use. Preferred is an approximately 3 log reduction of microorganisms or more, determined as described herein.

In some embodiments of a UV device, the UV light source is attached to the bottom of a robot crawler that uses suction allowing it to crawl vertically on walls and horizontally on ceilings of a room, space or environment. The robot crawler may follow programming allowing it to perform an effective pattern on the wall and ceiling covering desired areas. The robot crawler may move at a speed allowing an effective UV intensity/UV dosage required for sanitization to be reached while minimizing power use. Preferred is an approximately 3 log reduction of microorganisms or more, determined as described herein.

### VI. METHODS OF MANUFACTURING

In another aspect of the present invention, methods of manufacturing a UV device described herein, are provided. While the following provides steps for manufacturing a UV device of the UVT-4, UV6K, and UV2K family of portable UV devices, one of ordinary skill in the art will be able to deduce from thereon steps for manufacturing other UV devices described herein as well. As one of ordinary skill in the art will appreciate, the steps provided below may be performed in any order, unless clearly contradicted by content or explicitly stated. One of ordinary skill in the art reviewing Figures 1-29 will readily identify locations within the UV device to which individual parts and components have been attached. One of ordinary skill in the art reviewing Figures 1-29, however, will also appreciate that those locations to which individual parts are shown being attached, are non-limiting.

In some embodiments, a method of manufacturing a UV device comprises the steps of attaching at least one first germicidal UV light source to an upper frame, attaching at least one second germicidal UV light source **16** to a lower frame **23**, and attaching a first hinge **38** to the lower frame **23** and to the upper frame thereby connecting the lower frame **23** to the upper frame so that the upper frame can move in a position ranging from about 0 degree to about 90 degrees with respect to the position of the lower frame **23**. In some embodiments, a method of manufacturing a UV device comprises the step of attaching the first hinge **38** to the lower frame **23** and to the upper frame using fasteners **47** so that fasteners **47** movably connect the upper frame to the lower frame **23** wherein the upper frame is capable of swinging into an angular position with respect to the position of the lower frame **23**.

In some embodiments, a method of manufacturing a UV device comprises the step of attaching a means for controlling or facilitating movement of the upper frame into a position ranging from about 0 degree to about 90 degrees with respect to the position of the lower frame **23**. Suitable means for controlling or facilitating movement of the upper frame into a position ranging from about 0 degree to about 90 degrees with respect to the position of the lower frame **23** and individual components thereof for attaching are described herein.

In some embodiments, a method of manufacturing a UV device comprises the step of surrounding a first germicidal UV light source **16** with a UV light permissible housing **15**. Suitable housings **15** are described herein.

In some embodiments, a method of manufacturing a UV device comprises the step of surrounding a second germicidal UV light source **16** with a UV light permissible housing **15**. Suitable housings **15** are described herein.

In some embodiments, a method of manufacturing a UV device comprises the step of attaching to the lower frame **23** a means for operatively attaching the UV device to an opening **48** of a container **49**, to a fixture in a room, or to a fixture in or at a space or defined environment. Suitable means for operatively attaching the UV device to an opening **48** of a container **49**, to a fixture in a room, space or defined environment and individual components thereof for attaching are described herein and shown in the figures. In some embodiments, a method of manufacturing a UV device comprises the step of operatively attaching a bracket tightening knob **26** to the means for attaching the UV device to an opening **48** of a container **49**, to a fixture in a room, space or defined environment. In some embodiments, a method of manufacturing a UV device comprises the step of operatively attaching a first rope post **27** to the means for attaching the UV device to an opening **48** of a container **49**, to a fixture in a room, or defined environment. In some embodiments, a method of manufacturing a UV device comprises the step of operatively attaching a second rope post **28** to the means for attaching the UV device to an opening **48** of a container **49**, to a fixture in a room, space or defined environment. In some embodiments, a method of manufacturing a UV device comprises the step of operatively attaching a means for attaching the UV device to an opening **48** of a container **49**, to a fixture in a room, space or defined environment to the lower frame **23** via a second hinge **34**.

In some embodiments, a method of manufacturing a UV device comprises the step of operatively attaching a first upper frame end **24** to the upper frame. Suitable non-limiting, examples of first upper frame ends **24** are described herein and shown in the figures.

In some embodiments, a method of manufacturing a UV device comprises the step of operatively attaching a second upper frame end **29** to the upper frame. Suitable non-limiting examples of second upper frame ends **29** are described herein and shown in the figures.

In some embodiments, a method of manufacturing a UV device comprises the step of operatively attaching a first lower frame end **25** to the lower frame **23**. Suitable non-limiting examples of first lower frame ends **25** are described herein and shown in the figures.

In some embodiments, a method of manufacturing a UV device comprises the step of operatively attaching a second lower frame end **30** to the lower frame **23**. Suitable non-limiting examples of second lower frame ends **30** are described herein and shown in the figures.

In some embodiments, a method of manufacturing a UV device comprises the step of operatively attaching a UV sensor **17** to the upper frame.

In some embodiments, a method of manufacturing a UV device comprises the step of operatively attaching a UV sensor **17** to the lower frame **23**.

In some embodiments, a method of manufacturing a UV device comprises the step of operatively attaching a plurality of protective rods **31** between the first upper frame end **24** and the second upper frame end **29**.

In some embodiments, a method of manufacturing a UV device comprises the step of operatively attaching a plurality of protective rods **31** between the first lower frame end **25** and the second lower frame end **30**.

In some embodiments, a method of manufacturing a UV device comprises the step of operatively attaching a plurality of cross connectors **32** to the upper frame so that the plurality of protective rods **31** penetrates same.

In some embodiments, a method of manufacturing a UV device comprises the step of operatively attaching an upper frame fixture clip **33** to the first lower frame end **25** so that the upper frame fixture clip **33** can engage with the first upper frame end **24** and prevents the upper frame from moving.

In some embodiments, a method of manufacturing a UV device comprises the step of operatively running a cable **58** through a cable guide **61** of the first hinge **38** so that a first end of the cable **58** can engage with a first hook **59** of an extension spring **18** and so that the second end of cable **58** is fixed in a cable anchoring point **62** within the first hinge **38**.

In some embodiments, a method of manufacturing a UV device comprises the step of operatively attaching a cross connector **44** to lower frame **23**

In some embodiments, a method of manufacturing a UV device comprises the step of operatively attaching a first side plate **42** to the cross connector **44**.

In some embodiments, a method of manufacturing a UV device comprises the step of operatively attaching a second side plate **43** to the cross connector **44**.

In some embodiments, a method of manufacturing a UV device comprises the step of operatively attaching a side plate spacer **41** between the first and second side plates **42**, **43**.

In some embodiments, a method of manufacturing a UV device comprises the step of operatively attaching a stop post **39** to the first side plate **42** so that the stop post **39** prevents the upper frame of the UV device to move beyond a perpendicular/vertical position with respect to the lower frame **23** of the UV device.

In some embodiments, a method of manufacturing a UV device comprises the step of operatively attaching a stop post **39** to the second side plate **43** so that the stop post **39** prevents the upper frame of the UV device to move beyond a perpendicular/vertical position with respect to the lower frame **23** of the UV device.

In some embodiments, a method of manufacturing a UV device comprises the step of operatively attaching a second anchoring post **19** for an extension spring **18** to the lower frame **23**.

In some embodiments, a method of manufacturing a UV device comprises the step of operatively attaching a first hook **59** of an extension spring **18** to the first end of cable **58** to form a first anchoring post **46** for the extension spring **18**.

In some embodiments, a method of manufacturing a UV device comprises the step of operatively attaching a second hook **50** of an extension spring **18** to second anchoring post **19** for the extension spring **18**.

In some embodiments, a method of manufacturing a UV device comprises the step of operatively attaching a handle **51** to the second anchoring post **19**.

In some embodiments, a method of manufacturing a UV device comprises the step of coating the lower side of the lower frame **23** with a plastic or teflon.

In some embodiments, a method of manufacturing a UV device comprises the step of drilling an aperture into the first upper frame end **24** so that it can serve as a rope anchoring point **52**.

In some embodiments, a method of manufacturing a UV device comprises the step of operatively attaching UV lamp sockets **20** to a first germicidal UV light source and attaching the UV lamp sockets **20**/first germicidal UV light source to openings in the first upper frame end **24** and in the second upper frame end **29** so that the first germicidal UV light source is positioned in between the first upper frame end **24** and the second upper frame end **29**.

In some embodiments, a method of manufacturing a UV device comprises the step of operatively attaching UV lamp sockets **20** to a second germicidal UV light source **16** and operatively attaching the UV lamp sockets **20**/second germicidal UV light source to openings in the first lower frame end **25** and in the second lower frame end **30** so that the second germicidal UV light source **16** is positioned in between the first lower frame end **25** and the second lower frame end **30**.

In some embodiments, a method of manufacturing a UV device comprises the step of operatively attaching a rope **13** to the UV light source **16** and thereby operatively connecting the UV light source **16** with a control box **1** and permitting movement of the UV device in a container, room, space or defined environment.

In some embodiments, a method of manufacturing a UV device comprises the step of operatively attaching one or more reflectors **68** or **78** to a frame of a UV device.

In some embodiments, a method of manufacturing a UV device comprises the step of operatively attaching a UV light source **16** to a frame having a plurality of openings.

It is also understood that for the methods described herein, individual steps may be performed by more than one person or more than one entity. Thus, not every step of a method described herein must be performed by the same person or entity.

Preferred embodiments of this invention are described herein, including the best mode known to the inventor for carrying out the invention. Of course, variations on those preferred embodiments will become apparent to those of ordinary skill in the art upon reading the foregoing description. The inventors expect skilled artisans to employ such variations as appropriate, and the inventors intend for the invention to be practiced otherwise than specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

As can be appreciated from the disclosure above, the present invention has a wide variety of applications. While each of the elements of the present invention is described herein as containing multiple embodiments, it should be understood that, unless indicated otherwise, each of the embodiments of a given element of the present invention is capable of being used with each of the embodiments of the other elements of the present invention and each such use is intended to form a distinct embodiment of the present invention. The invention is further illustrated by the following examples, which are only illustrative and are not intended to limit the definition and scope of the invention in any way.

### VII. EXAMPLES

The below examples are meant to illustrate specific embodiments of the methods and compositions described herein and should not be construed as limiting the scope of the invention in any way.

### Example 1. Calculating Killing of Microorganisms

The following provides steps to calculate UV intensities and hence time of sterilization needed to kill a desired microorganism using compositions, algorithms and methods of the present invention. The required Energy Dosage of UV Radiation (UV Dose) in µWs/cm² (microwatts/cm²) needed for kill factor is provided herein in Tables 1-5. Per algorithm provided herein and to determine the UV intensity (UV dosage) on a surface at various distances from a germicidal UV light source a user divides the radiant energy (shown in microwatts per square centimeter at one meter distance) by the intensity factor as shown in Table 9 below.

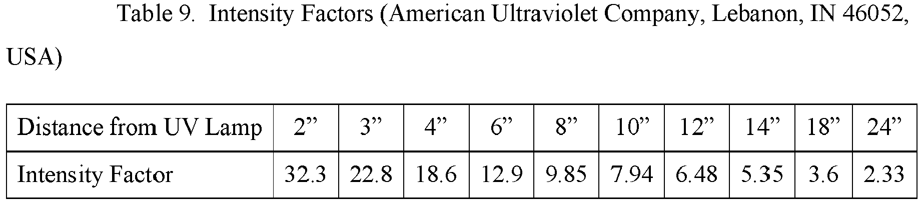

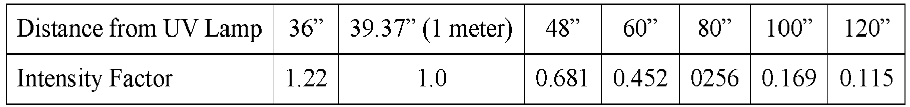

Using a UV light source with an output of 190 microwatts/cm² at 254 nm (at a distance of 1 meter), placed within a fermentation vessel 36" from the interior surface, the following calculations are used for achieving 99% killing of *Saccharomyces cerevisiae* (13,200 microwatt seconds/cm² required; see Table 5). Step 1: 13,200 microwatt seconds/cm² / 190 microwatts/cm² = 69.47 seconds. Step 2: The intensity factor at 36" is 1.22 (*see* Table 8), therefore 69.47 seconds / 1.22 = 56.94 seconds.

Using a UV light source with an output of 190 microwatts/cm² at 254 nm (at a distance of 1 meter), placed within a fermentation vessel 60" from the interior surface, the following calculations are used for achieving 99% killing of *Shigella dysentery* (4,200 microwatt seconds/cm² required; *see* Table 2). Step 1: 4,200 microwatt seconds/cm² / 190 microwatts/cm² = 22.10 seconds. Step 2: The intensity factor at 60" is 0.452 (*see* Table 8), therefore 22.10 seconds / 0.452 = 48.90 seconds.

Using a UV light source with an output of 190 microwatts/cm² at 254 nm (at a distance of 1 meter), placed within a fermentation vessel 60" from the interior surface, the following calculations are used for achieving 99% killing of *Sarcina lutea* (26,400 microwatt seconds/cm² required; see Table 2). Step 1: 26,400 microwatt seconds/cm² / 190 microwatts/cm² = 138.94 seconds. Step 2: The intensity factor at 60" is 0.452 (*see* Table 8), therefore 138.94 seconds / 0.452 = 307.40 seconds.

Since *Sarcina lutea* is one of the most UV resistant bacteria (more resistant than known species of yeast), a fermentation vessel where the UV source was 60" away from the internal surface could be left on for about 307.40 seconds at each sterilization interval within the vessel to ensure all yeast (known) and pathogenic microorganisms are killed.

### Example 2. Inhibiting the Growth of Bacillus Subtilis

To determine the effectiveness of a method of the present invention and efficacy of a UV device of the present invention for the sanitization of a stainless steel tank used in the wine making process, the killing/growth arrest of *Bacillus subtilis* (American Type Culture Collection, ATCC Number 82TM; designations: AMC [ATCC 8037, NRS 315]) was investigated. *Bacillus subtilis* forms spores, thereby making it a more UV resistant microorganism than microorganisms that do not form spores. In this experiment, as a UV light source, 30" SE UV-C lamps (Steril-Aire) were used. Three identical UV lamps were placed in a mount and put in a spiral configuration with each UV lamp set at a 15 degrees angle.

Two coupons (per time point) were spiked with a *Bacillus subtilis* suspension to give a final concentration of 9.6 x 10⁶ CFU (colony forming units)/coupon for the first three time points. The fourth (25 minute) time point was inoculated with a suspension of 1.3 x 10⁷ CFU/ coupon (since it was tested on a different day) and allowed to air dry inside a biological safety cabinet. The coupons were allowed to dry and attached to the inside of stainless steel tank. Then the coupons were exposed to the UV light at a distance of 60" from the UV light source for four all four (4) time points: 30 seconds, 5 minutes, 15 minutes and 25 minutes. After each exposure time was performed, the coupons were swabbed in order to perform the recovery process. Two additional stainless steel coupons were spiked to be used as positive controls.

UV readings to measure the UV-C exposure at various time points were done using a General UV512C Digital UV-C Meter (radiometer). Table 10 below provides the actual UV readings recorded for each exposure time:

**Table 10. UV Readings per Time Point and Interval.**

| 30 Seconds Time Point | | 5 Minutes Time Point | | 15 Minutes Time Point | | 25 Minutes Time Point | |
|---|---|---|---|---|---|---|---|
| Seconds | uW | Minutes | uW | Minutes | uW | Minutes | uW |
| 5 | 42 | 0.5 | 135 | 1 | 243 | 3 | 200 |
| 10 | 54 | 1 | 202 | 2 | 225 | 6 | 179 |
| 15 | 69 | 1.5 | 206 | 3 | 212 | 9 | 174 |
| 20 | 87 | 2 | 204 | 4 | 198 | 12 | 167 |
| 25 | 109 | 2.5 | 202 | 5 | 186 | 15 | 162 |
| 30 | 135 | 3 | 198 | 6 | 177 | 18 | 159 |
| | | 3.5 | 195 | 7 | 176 | 21 | 162 |
| | | 4 | 192 | 8 | 181 | 24 | 169 |
| | | 4.5 | 190 | 9 | 175 | | |
| | | 5 | 184 | 10 | 172 | | |
| | | | | 11 | 171 | | |
| | | | | 12 | 171 | | |
| | | | | 13 | 171 | | |
| | | | | 14 | 170 | | |
| | | | | 15 | 168 | | |

The recovery of *Bacillus subtilis* from the coupons after 30 seconds exposure to the UV light was 5.3 x 10⁵ CFU/ml. After 5 minutes exposure to the UV light, the recovery of *Bacillus subtilis* was reduced to 1.4 x 10³ CFU/ml. After 15 minutes exposure to the UV light, the recovery of *Bacillus subtilis* was further reduced to 1.5 x 10¹ CFU/ml. Finally, after 25 minutes exposure to the UV light, no microorganisms were recovered. The recovery positive control had a count of 6.4 x 10⁵ CFU/ml for the first three time points and 8.1 x 10⁵ CFU/ml for the fourth time point.

Table 11 below summarizes the results of the above experiment and provides the log reduction results based on calculations from *Bacillus subtilis* recovery from test coupon vs. positive control.

**Table 11. Inhibiting the growth of Bacillus subtilis.**

| Exposure Time | Concentration *Bacillus subtilis* Recovered (CFU/ml) | Log Reduction |
|---|---|---|
| 30 seconds | 5.3 x 10⁵ | 0.1 |
| 5 minutes | 1.4 x 10³ | 2.7 |
| 15 minutes | 1.5 x 10¹ | 4.6 |
| 25 minutes | 0 | 5.9 |

The results of this experiment demonstrated that the UV light source tested was effective in reducing the *Bacillus subtilis* microorganism population by about 3 logs at an exposure time of 5 minutes, by about 5 logs at an exposure time of 15 minutes and by about 6 logs at exposure time of 25 minutes.

One of skill in the art will appreciate that in view of the experiments described above, a lower UV dose will be required to kill or inhibit the growth of other microorganisms that do not produce spores. Thus, by having demonstrated that one of the most UV-resistant microorganisms can be efficiently killed or growth inhibited using a method of the present invention, one of skill in the art will appreciate that the methods of the present invention in combination with the UV devices of the present invention are useful to kill or growth inhibit other microorganism that might be present in a fermentation container, more specifically on a surface of a fermentation container

### Example 3. Comparative Efficacy Trial Of Sanitization Using UVC (UVT-4 Model) Versus Steam And Peracetic Acid On Reduction Of Microbial Populations On Stainless Steel Tanks

A comparative efficacy trial on three different tank sanitation methods was conducted at a winery in St. Helena, CA. The objective of this comparative trial was to evaluate the sanitization efficacies of various sanitizers (Steam, Peracetic Acid (PAA), and Ultraviolet Light C (UVC)) on the reduction of wine and environmental microbe populations on interior surfaces of stainless steel production tanks. The trial was conducted on four different tanks. Briefly, the methodology of the trial was as follows: (i) tanks were emptied of wine; (ii) pre-treatment microbiological swab samples were collected from the ceiling, wall, and floor of each tank; (iii) tanks underwent appropriate sanitation protocol; (iv) post-treatment microbiological swab samples were collected from ceiling, wall, and floor of each tank; (v) microbiological swab samples were processed at a microbiology laboratory; and (vi) the survivability, percent Colony Forming Units (CFU) reduction, and Logio reduction of microbe populations after treatment with the various sanitizers was determined and compared.

The objective and scope of this trial were as follows:
(1). Equipment: Four stainless steel tanks;
(2). Surface type: 316 grade stainless steel;
(3). Cleaning methods: (a) water rinse, (b) caustic cleaner;
(4). Sanitizing methods: (a) steam; (b) PAA; (c) UVC at 253.7 nm
(5). Efficacy testing method: Surface-based swab recovery method to detect microbial populations
(6). Test locations on interior of stainless steel tanks: (a) floor; (b) wall; (c) interior ceiling
(7). Types of microbes monitored for on interior surfaces of tanks: (a) wine and environmental yeast; (b) wine and environmental bacteria; (c) molds

The methodology of the trial was as follows:
(A). A total of four tanks were used for the trial.
(B). All four tanks were emptied of wine prior to start of trial.
(C). Pre-treatment surface swab samples were collected from the floor, wall, and interior ceiling of each tank: (1). A 4 inch x 4 inch square area (swab both horizontally and vertically in area) was swabbed at each location. (2). Samples collected at this stage were used to determine the starting levels of microbes, which were then used to determine the percent CFU reduction and Log₁₀ reduction in microbial load after each sanitizer treatment. (3). The total number of samples collected at starting point was: 4 tanks x 3 sample points = 12 samples.
(D). The tanks were exposed to the following sanitation treatment methods. (1). Tank 1 was rinsed with water and treated with steam. (2). Tank 2 was cleaned with caustic, rinsed with water, treated with PAA, and rinsed with water. (3) Tank G13 was rinsed with water and treated with UVC for 10 minutes (using Model UVT-4). (4). Tank G12 was cleaned with caustic, rinsed with water, treated with PAA, and rinsed with water.
(E). After application of sanitizer, post-treatment surface samples were collected from floor, wall, and interior ceiling of each tank. Samples were collected at different locations on tanks than locations used prior to treatments. The total number samples collected post sanitizer was: 4 tanks x 3 sample points = 12 samples.
(F). Samples were transported back to a microbiological laboratory and processed as follows: (1). All 24 samples and a saline blank were filter plated using Wallerstein Nutrient Media. (2). Plates were incubated at 29°C for 4 to 7 days depending on rate of microbial growth. (3). After 4 to 7 days, surviving microorganisms were counted.
(G). The efficacy of sanitizing methods on interior surfaces of stainless steel tanks was determined by measuring the survivability, percent CFU reduction, Log₁₀ reduction of microbe populations after treatment with sanitizers.

The data set of this trial and results are shown in FIGS. 30A-D. The results in FIGS 30B-D show that all three sanitizing methods significantly reduced microbial loads on the ceiling, wall, and floor of tanks. When steam was used as the sanitizer, the percent CFU reduction of microbial loads on the ceiling, wall, and floor was 81%, 94%, and 90%, respectively. The Logio reduction of microbial loads on the ceiling, wall, and floor was 0.7, 1.2, and 1.0, respectively. When PAA was used as the sanitizer, the percent CFU reduction of microbial loads on the ceiling, wall, and floor was 77%, 91%, and 99.5%, respectively, for trial #1. For trial #2, the percent CFU reduction on ceiling, wall, and floor was 85%, 90%, and 99%, respectively. The Log₁₀ reduction of microbial loads on the ceiling, wall, and floor was 0.6, 1.0, and 2.3, respectively, for trial #1. For trial #2, the Log₁₀ reduction on ceiling, wall, and floor was 0.8, 1.0, and 2.0, respectively. When UVC (UVT-4 Model) was used as the sanitizer, the percent CFU reduction of microbial loads on the ceiling, wall, and floor was 93%, 97%, and 99.8%, respectively. The Log₁₀ reduction of microbial loads on the ceiling, wall, and floor was 1.2, 1.6, and 2.8, respectively.

When comparing the results between the three sanitizer treatment methods, UVC (UVT-4 Model) was the most effective sanitizer at reducing microbial loads on the ceiling, wall, and floor of tanks. This was the case when looking at the data for both percent CFU reduction and Log₁₀ reduction of microbial loads. In FIGS 30C and 30D, a significant difference in reduction of microbe populations by UVC (UVT-4 Model) compared to the other sanitizing methods is indicated in grey shades. A comparison between steam and PAA showed the following with respect to reducing microbial loads: steam and PAA were approximately equivalent on the ceiling of tanks, steam was slightly more effective on the wall of tanks, and PAA was significantly more effective on the floor of tanks.

After comparing the data collected from the trial, UVC (UVT-4 Model) was determined to be the most effective of the three sanitizers at reducing microbial loads on all three surfaces sampled on interior of stainless steel tanks (ceiling, wall, and floor). Steam was significantly less effective than UVC and PAA at reducing microbial loads on the floor of tank.

The results from this trial demonstrate that UVC is a superior sanitizer for interior of winery stainless steel tanks compared to steam and chemical sanitizers currently used in the wine industry.

### Example 4. Comparative Efficacy Trial Of Sanitization Using UVC (UVT-4 Model) Versus Chlorine Dioxide On Reduction Of Microbial Populations On Stainless Steel Tanks

A comparative efficacy trial on two different tank sanitization methods (chlorine dioxide, ClO₂ (ozone) and UVC (UVT-4 Model)) was conducted at a winery in Sonoma, CA. The objective of this comparative trial was to evaluate the sanitation efficacies of two different sanitizers on the reduction of wine and environmental microbe populations on interior surfaces of stainless steel production tanks.

The trial was conducted on four different tanks with two of the tanks receiving treatment with UVC (UVT-4 Model) and two tanks receiving treatment with ozone (chlorine dioxide). Briefly, the methodology of the trial was as follows: (i) tanks were emptied of wine; (ii) pre-treatment microbiological swab samples were collected from the ceiling, wall, and floor of each tank; (iii) tanks underwent appropriate sanitation protocol; (iv) post-treatment microbiological swab samples were collected from ceiling, wall, and floor of each tank; (v) microbiological swab samples were processed at a microbiology laboratory (BevTrac Mobile Quality Systems LLC (BevTrac)); and the survivability, percent Colony Forming Units (CFU) reduction, and Log₁₀ reduction of microbe populations after treatment with sanitizers was determined and compared.

The objective and scope of this trial were as follows:
(1). Equipment: Ten stainless steel tanks;
(2). Tank Size: approximately 6,000 gallons
(3). Surface type: 316 grade stainless steel;
(4). Cleaning methods: 270 Extra;
(5). Sanitizing methods: (a) UVC at 253.7 nm; (b) chlorine dioxide [Chlorine dioxide kills microorganisms by attacking amino acids within the cell. Specifically, chlorine dioxide breaks chemical bonds of amino acids (disulfide bridges and aromatic ring structures), which destroys proteins within the cell];
(6). Positive Control: tank not exposed to cleaner or sanitizer;
(7). Negative Control: tank not exposed to *Saccharomyces* inoculum and not cleaned or sanitized after study was initiated;
(8). Efficacy testing method: Surface-based swab recovery method to detect microbial populations;
(9). Test locations on interior of stainless steel tanks: (a) floor; (b) wall; (c) interior ceiling;
(10). Types of microbes monitored for on interior surfaces of tanks: (a) wine and environmental yeast; (b) wine and environmental bacteria; (c) molds.

The methodology of the trial was as follows:
(A). A total of ten tanks were used for the trial.
(B). The tanks were cleaned and sanitized using winery standard operating procedure for tanks.
(C). The interior surface of all tanks, except Negative Control tank, were contaminated with *Saccharomyces cerevisiae* using a tank washer to spray inoculum.
(D). Pre-treatment surface swab samples were collected from the floor, wall, and interior ceiling of each tank after application of inoculum as follows: (1). A 4 inch x 4 inch square area (swab both horizontally and vertically in area) was swabbed at each location. (2). Samples collected at this stage were used to determine the starting levels of microbes, which were then be used to determine the Log₁₀ reduction in microbial load after each sanitizer treatment. (3). The total number of samples collected at starting point was: 10 tanks x 3 sample points = 30 samples.
(E). The tanks were exposed to the following treatment methods. Tanks were exposed to chlorine dioxide for 10 minutes and exposed to UVC for 12 minutes. (1). Negative Control (Tank 61) - tank not exposed to Saccharomyces inoculum and not cleaned or sanitized. (2). Positive Control (Tank 62) - tank contaminated with *Saccharomyces*, but not treated with cleaner or sanitizer. (3). Short wide tank (Tank 63) treated with cleaner (270 Extra) and chlorine dioxide. (4). Short wide tank (Tank 64) treated with cleaner (270 Extra) and UVC. (5). Tall thin tank (Tank 67) treated with cleaner (270 Extra) and chlorine dioxide. (6). Tall thin tank (Tank 68) treated with cleaner (270 Extra) and UVC. (7). Short wide tank (Tank 65) treated with chlorine dioxide. (8). Short wide tank (Tank 66) treated with UVC. (9). Tall thin tank (Tank 69) treated with chlorine dioxide. (10). Tall thin tank (Tank 57) treated with UVC.
(F). After application of sanitizer, post-treatment surface samples were collected from floor, wall, and interior ceiling of each tank. Samples were collected at different locations on tanks than locations used prior to treatments. The total number samples collected post sanitizer was: 10 tanks x 3 sample points = 30 samples.
(G). Samples were transported back to BevTrac laboratory and processed as follows: (1). Because the Pre-Treatment samples were expected to have a high population of yeast, these 30 samples were serial diluted in saline solution using test tubes. (2). All 60 samples and a saline blank were filter plated using Wallerstein Nutrient Media. (3). Plates were incubated at 29°C for 4 to 7 days depending on rate of microbial growth. (4). After 4 to 7 days, surviving microorganisms were counted.
(H). The efficacy of sanitizing methods on interior surfaces of stainless steel tanks was determined by measuring the survivability and Log₁₀ reduction of microbe populations after treatment with sanitizers.

The data for this trial are shown in FIGS 31A-I. The Negative Control tank (not exposed to yeast inoculum and not cleaned or sanitized after start of study) showed very low levels of microbial contamination both at the pre- treatment and the post-treatment sample collection times (FIG. 31A). This demonstrates that the tanks were effectively sanitized prior to the study and that tanks very likely did not accumulate environmental microbe contamination during the course of the study. The Positive Control tank (inoculated with yeast and not treated with cleaner or sanitizer) displayed high levels of microbial contamination both at the pre-treatment and the post- treatment sample collection times (FIG. 31A). These results show that the interior of the tanks were effectively inoculated with yeast and that the yeast populations did not significantly decrease during the course of the study.

FIG. 31B schematically depicts the effect of cleaning two short wide tanks (tanks 63 and 64) with 270 Extra and then sanitizing with either chlorine dioxide or UVC, respectively, on the survivability of microbial populations on ceiling, wall, and floor of each tank. Microbe survival is represented as Log₁₀ CFU. For reference, the microbe survival results for the Negative Control tank and Positive Control tank are shown in FIG. 31B. The results in FIGS 31B and 31C show that both sanitizing methods in combination with the cleaner significantly reduced microbial loads on the ceiling, wall, and floor of tanks. Both sanitizers helped to produce a ≥3 Logio reduction. The Log₁₀ reduction of microbial loads on the ceiling, wall, and floor after cleaning and chlorine dioxide sanitation were 3.6, 3.5 and 3.9, respectively. The Logio reduction of microbial loads on the ceiling, wall, and floor after cleaning and UVC sanitation were 3.0, 5.0, and 4.6, respectively. Based on the results obtained in this trial, the cleaner and chlorine dioxide was more effective at reducing microbial load on ceiling of tank than the combination of cleaner and UVC. However, surprisingly and unexpectedly application of the cleaner and UVC produced a significantly higher reduction of microbe load on the wall and floor compared to use of cleaner and chlorine dioxide. In FIG. 31C, a significant difference in reduction of microbe populations by cleaner/chlorine dioxide or cleaner/UVC is indicated in grey shades.

FIG. 31D schematically depicts the effect of cleaning two tall thin tanks (tanks 67 and 68) with 270 Extra and then sanitizing with either chlorine dioxide or UVC, respectively, on the survivability of microbial populations on ceiling, wall, and floor of each tank. Microbe survival is represented as Log₁₀ CFU. For reference, the microbe survival results for the Negative Control tank and Positive Control tank are shown in FIG. 31D. The results in FIGS. 31D and 31E show that both sanitizing methods in combination with the cleaner significantly reduced microbial loads on the ceiling, wall, and floor of tanks. Both sanitizers helped to produce a ≥2.1 Log₁₀ reduction. The Log₁₀ reduction of microbial loads on the ceiling, wall, and floor after cleaning and chlorine dioxide sanitation were 3.3, 3.9, and 4.3, respectively. The Log₁₀ reduction of microbial loads on the ceiling, wall, and floor after cleaning and UVC sanitation were 2.1, 4.4 and 5.3, respectively.

The results are very similar to the short wide tanks above where the cleaner and chlorine dioxide were more effective at reducing microbial load on ceiling of tank than the combination of cleaner and UVC, and the cleaner and UVC produced a significantly higher reduction of microbe load on the wall and floor compared to use of cleaner and chlorine dioxide. In FIG. 31E, a significant difference in reduction of microbe populations by cleaner/chlorine dioxide or cleaner/UVC is indicated in grey shades.

The results for both the short wide tanks and the tall thin tanks after application of cleaner and sanitizer show that the cleaner/UVC combination has a higher efficacy on the walls and floors of tanks, while the cleaner/chlorine dioxide combination has a higher efficacy on the tank ceiling. At two tank sites sampled (wall of tank 64 and floor of tank 68), UVC in combination with cleaner completely eliminated all microbes. Chlorine dioxide did not completely eliminate microbes at any site sampled.

The results suggest that UVC can kill microbes more effectively than chlorine dioxide when surfaces are close to the ultraviolet light (i.e, walls and floors). The efficacy of UVC on the ceiling of tanks could possibly be improved by increasing UVC exposure time or increasing intensity of ultraviolet lights. For chlorine dioxide, the reduction in microbe populations was pretty consistent for both tanks shapes on all surfaces sampled (3.3 to 4.3 Log₁₀ reduction

FIG. 31F schematically depicts the effect of sanitizing two short wide tanks (tanks 65 and 66) with either chlorine dioxide or UVC, respectively, on the survivability of microbial populations on ceiling, wall, and floor of each tank. For this trial, no cleaning agent was used prior to application of sanitizer. Microbe survival is represented as Log₁₀ CFU. For reference, the microbe survival results for the Negative Control tank and Positive Control tank are shown in FIG. 31F. The results in FIGS. 31F and 31G show that both sanitizing methods significantly reduced microbial loads on the ceiling, wall, and floor of tanks. Both sanitizers helped to produce a ≥2.4 Log₁₀ reduction. The Log₁₀ reduction of microbial loads on the ceiling, wall, and floor after chlorine dioxide sanitation were 3.6, 3.9, and 4.6, respectively. The Logio reduction of microbial loads on the ceiling, wall, and floor after UVC sanitation were 2.4, 4.7, and 4.0, respectively.

Based on the results of this trial, the chlorine dioxide was more effective at reducing microbial load on ceiling and floor of tank than the UVC. However, UVC produced a significantly higher reduction of microbe load on the wall compared to use of chlorine dioxide. In FIG. 31G a significant difference in reduction of microbe populations by chlorine dioxide or UVC is indicated in grey shades.

FIG. 31H schematically depicts the effect of sanitizing two tall thin tanks (tanks 69 and 57) with either chlorine dioxide or UVC, respectively, on the survivability of microbial populations on ceiling, wall, and floor of each tank. For this trial, no cleaning agent was used prior to application of sanitizer. Microbe survival is represented as Log₁₀ CFU. For reference, the microbe survival results for the Negative Control tank and Positive Control tank are shown in FIG. 31H. The results in FIGS. 31H and 31I show that both sanitizing methods significantly reduced microbial loads on the ceiling, wall, and floor of tanks. Both sanitizers helped to produce a ≥2.7 Log₁₀ reduction. The Log₁₀ reduction of microbial loads on the ceiling, wall, and floor after chlorine dioxide sanitation were 2.8, 4.2 and 2.7, respectively. The Log₁₀ reduction of microbial loads on the ceiling, wall, and floor after UVC sanitation were 2.7, 4.2 and 4.6, respectively.

Based on the results of this trial, UVC produced a significantly higher reduction of microbe loads on the floor compared to using chlorine dioxide. For the ceiling and wall, both sanitation methods were equally effective. In FIG. 31I, a significant difference in reduction of microbe populations by chlorine dioxide or UVC is indicated in grey shade.

The results for both the short wide tanks and the tall thin tanks after use of sanitizer show that UVC has a higher or equal efficacy compared to chlorine dioxide on the walls and floor. However, again chlorine dioxide demonstrated a higher efficacy on tank ceiling than UVC. This confirms that UVC, even in the absence of a cleaner, can kill microbes more effectively than or just as effectively as chlorine dioxide when surfaces are close to UVC (i.e. walls and floors).

There are several benefits that can be realized by wineries if they use UVC instead of chemicals as a sanitizer for stainless steel tanks: 1) significantly less water usage; 2) reduced wastewater generated; 3) more environmentally friendly due to reduced chemical usage; 4) reduced labor costs; and 5) more effective reduction in microbe populations on stainless steel surfaces.

### Example 5. Comparative Efficacy Trial Of Sanitization Using UVC (6K Model) Versus Caustic/Citric Acid On Reduction Of Microbial Populations On Stainless Steel Tanks

A comparative efficacy trial on two different tank sanitization methods (caustic cleaner/citric acid) and UVC (UV6K Model) was conducted at a winery in Rutherford, CA. The objective of this comparative trial was to evaluate the sanitation efficacies of two different sanitizers on the reduction of wine and environmental microbe populations on interior surfaces of stainless steel production tanks.

The trial was conducted on two different tanks. Briefly, the methodology of the trial was as follows: tanks were emptied of wine; pre-treatment microbiological swab samples were collected from the floor, wall, ceiling, valve connection, bottom valve, and bottom door rim of each tank; tanks underwent appropriate sanitation protocol; post-treatment microbiological swab samples were collected from floor, wall, ceiling, valve connection, bottom valve, and bottom door rim of each tank; microbiological swab samples were processed at the BevTrac laboratory; and the survivability, percent Colony Forming Units (CFU) reduction, and Log₁₀ reduction of microbe populations after treatment with the two different sanitation methods was determined and compared.

The objective and scope of this trial were as follows:
The purpose of this trial was to determine the efficacy of two different sanitation methods (UVC, [UV6K Model] vs. Caustic Cleaner/Citric Acid) on the reduction of wine and environmental microbe populations on interior surfaces of winery stainless steel production tanks. The following factors were used to conduct the study:
(1). Equipment: Two stainless steel tanks;
(2). Sanitizing methods: (a) caustic cleaner followed by citric acid; (b) UVC at 253.7 nm [UV6K];
(3). Efficacy testing method: Surface-based swab recovery method to detect microbial populations;
(4). Test locations on interior of stainless steel tanks: (a) floor; (b) wall; (c) interior ceiling; (d) valve connection; (e) bottom valve; (f) bottom door rim;
(5). Types of microbes monitored for on interior surfaces of tanks: (a) wine and environmental yeast; (b) wine and environmental bacteria; (c) molds.

The methodology of the trial was as follows:
.A. Two tanks were used for the trial.
B. Both tanks were emptied of wine prior to start of trial.
C. Pre-treatment surface swab samples were collected from the floor, wall, interior ceiling, valve connection, bottom valve, and bottom door rim of each tank: (1). An approximate 4 inch x 4 inch square area was swabbed at each location; (2). Samples collected at this stage were used to determine the starting levels of microbes, which were then be used to determine the percent CFU reduction and Log₁₀ reduction in microbial load after each sanitation treatment; (3). The total number of samples collected at starting point was: 2 tanks x 6 sample points = 12 samples.
D. The tanks were exposed to the following sanitation treatment methods. (1). Tank 29 was cleaned with caustic cleaner, rinsed with water, neutralized with citric acid, and rinsed with water; (2). Tank 10 was rinsed with water and treated with UVC for 40 minutes.
E. After each sanitation method, post-treatment surface samples were collected from the floor, wall, interior ceiling, valve connection, bottom valve, and bottom door rim of each tank. Swab samples were collected at different locations on tanks than locations used prior to treatments. The total number samples collected post treatment was: 2 tanks x 6 sample points = 12 samples.
F. Samples were transported back to BevTrac laboratory and processed as follows: (1). All 24 samples and a saline blank were filter plated using Wallerstein Nutrient Media; (2). Plates were incubated at 29°C for 4 to 7 days depending on rate of microbial growth; (3). After 4 to 7 days, surviving microorganisms were counted.
G. The efficacy of sanitation methods on interior surfaces of stainless steel tanks was determined by measuring the survivability, percent CFU reduction, and Log₁₀ reduction of microbe populations after sanitation treatment methods.

The data for this trial are shown in FIGS. 32A-D. FIG. 32A depicts the data set for the trial. The data set includes a description of two sanitation treatment methods performed on interior of stainless steel tanks; the tank number; the sites sampled on each tank (floor, wall, ceiling, valve connection, bottom valve, and bottom door rim); the total microbial load (includes yeast, bacteria, and mold) determined prior to each treatment; the total microbial load determined after each treatment; the percent CFU reduction in microbe populations after application of sanitation method; and the Logio reduction in microbe populations after application of sanitation method.

FIG. 32B schematically depicts the effect of caustic cleaner/citric acid and UVC on the survivability of microbial populations on floor, wall, ceiling, valve connection, bottom valve, and bottom door rim of each stainless steel tank. Microbe survival is represented as Log₁₀ CFU.

FIG. 32C shows the percent CFU reduction in microbes on floor, wall, ceiling, valve connection, bottom valve, and bottom door rim of stainless steel tanks after application of sanitation methods.

FIG. 32D shows the Log₁₀ reduction in microbes on floor, wall, ceiling, valve connection, bottom valve, and bottom door rim of stainless steel tanks after application of sanitation methods.

The results in FIGS. 32B, 32C, and 32D show that both sanitation methods significantly reduced microbial loads on the floor, wall, ceiling, valve connection, bottom valve, and bottom door rim of tanks.

When comparing the results between the two sanitation treatment methods, UVC (Model UV6K) was significantly more effective at reducing microbial loads on the floor and at the valve connection sites on tanks. This was the case when looking at the data for both percent CFU reduction and Logio reduction of microbial loads in FIGS. 32C and 32D. The caustic cleaner/citric acid method reduced microbial loads to a greater extent on the bottom valve compared to UVC. For the wall, ceiling, and bottom door rim, both sanitation methods reduced microbial populations by an approximately equivalent amount. Overall, based on these results, it is apparent that UVC was more effective than or equivalent to caustic cleaner/citric acid at reducing microbial loads on tank sites with the bottom valve being the only exception. UVC showed effective anti-microbial activity even in the absence of a cleaner.

In the following text, a set of items or embodiments is disclosed. The items are numbered in order to facilitate referencing features of one item in other items by referring to the respective number(s).
1. A portable ultraviolet (UV) device for applying a sterilization cycle to a surface within a container, a room, a space, or a defined environment, the UV device comprising:
   (i) a first germicidal UV light source;
   (ii) a frame to which the first germicidal UV light source is operatively attached;
   (iii) a control box for controlling a sterilization cycle;
   (iv) a cable operatively connecting the control box and the first germicidal UV light source; and
   (v) a means for operatively attaching the UV device to the container, room, space or defined environment,
   wherein the UV device is configured to movably and inwardly insert the first germicidal UV light source through an opening of the container, room, space or defined environment into the container, room, space or defined environment when the UV device is operatively attached to the container, room, space or defined environment;
   wherein the UV device uses an algorithm for determining the sterilization cycle; wherein the control box comprises a circuit board and at least three components selected from the group consisting of:
   (1) a power supply,
   (2) electronics for activating/deactivating the first germicidal UV light source,
   (3) a timer for controlling the duration of the sterilization cycle,
   (4) a memory for storing a pre-determined sterilization cycle,
   (5) a safety switch,
   (6) an on/off/reset button;
   (7) a status indicator light;
   (8) an alarm light; and
   (9) a user interface selected from the group consisting of a touch screen and a keyboard; and
   wherein the circuit board comprises at least three functionalities selected from the group consisting of:
   (A) comprising a radiofrequency identifier reader;
   (B) communicating with a radiofrequency identifier;
   (C) controlling a movement of the first germicidal UV light source within the container, room, space, or defined environment;
   (D) controlling a rate of descent or ascent of the first germicidal UV light source within the container, room, space, or defined environment;
   (E) controlling a positioning of first germicidal UV light source within the container, room, space, or defined environment;
   (F) controlling an on/off status of a motor, wherein the motor controls the positioning of the first germicidal UV light source within the container, room, space, or defined environment;
   (G) controlling an on/off status of the first germicidal UV light source based on measuring whether a pre-determined UV intensity has been attained;
   (H) controlling extension of the first germicidal UV light source from a housing;
   (I) controlling retraction of the first germicidal UV light source into the housing;
   (J) responding to a jammed position status of the first germicidal UV light source.
   (K) controlling an optical sensor which initiates a timer upon placing the first germicidal UV source into the container, room space, or defined environment;
   (L) controlling a speaker emitting an audible signal at the beginning or completion of the sterilization cycle;
   (M) controlling a plurality of LED lights indicting a status of the sterilization cycle;
   (N) relaying UV light intensity via a UV sensor to the container, room, space, or defined environment;
   (O) uploading and relaying information from the radiofrequency identifier;
   (P) generating a report on time of the sterilization cycle;
   (Q) generating a report on duration of the sterilization cycle:
   (R) generating a report on UV light intensity attained during the sterilization cycle;
   (S) emailing, phoning or texting the report on time of the sterilization cycle;
   (T) emailing, phoning or texting the report on duration of the sterilization cycle;
   (U) emailing, phoning or texting the report on UV light intensity attained during the sterilization cycle;
   (V) emailing, phoning or texting an alert to an individual that the sterilization cycle is initiated, interrupted or complete;
   (W) emailing, phoning or texting an alert that a UV light source requires replacement;
   (X) logging date, time or individual who used the UV device; and
   (Y) logging information of the container, room, space, or defined environment in which the UV device will be or has been used.
2. The UV device according to item 1, wherein the UV device is a portable UV device.
3. The UV device according to item 1, wherein the first germicidal UV light source resides in a housing.
4. The UV device according to item 3, wherein the housing permits UV light to pass through.
5. The UV device according to item 3, wherein the housing is made of UV fused silica, CaF₂, MgF₂, BaF₂, quartz, sapphire, teflon, polydimethylsiloxane, TPX® or polymethylpentene (PMP).
6. The UV device according to item 1, further comprising:
   (vi) a rope, a cable or a rigid rod for moving the first UV light source to a position within the container, room, space or defined environment.
7. The UV device according to item 1, wherein the frame comprises a plurality of openings.
8. The UV device according to item 1, further comprising:
   (vi) a second germicidal UV light source; and
   wherein the frame comprises
   (a) a lower frame comprising a first lower frame end and a second lower frame end;
   (b) an upper frame comprising a first upper frame end and a second upper frame end;
   (c) a first hinge movably and operatively connecting the lower frame to the upper frame and configured to move the upper frame into an angular position with respect to the position of the lower frame;
   wherein the first germicidal UV light source is operatively attached to the upper frame; wherein the second germicidal UV light source is operatively attached to the lower frame; and
   wherein, when not in use, the upper frame is positioned on top of the lower frame.
9. The UV device according to item 1, wherein (v) is configured to operatively attach the portable UV device to an opening of the container, to a fixture in or at the container, room, space or defined environment and to permit the moving the first germicidal UV light source to a position within the container, room, space or defined environment.
10. The UV device according to item 1, wherein (v) is a mounting bracket.
11. The UV device according to item 8, further comprising:
   (vii) a second hinge movably and operatively connecting the lower frame to (v).
12. The UV device according to item 8, further comprising:
   (vii) a means for controlling or facilitating movement of the upper frame to an angular position with respect to the position of the lower frame.
13. The UV device according to item 12, wherein (vii) permits the first germicidal UV light source be positioned at an angle ranging from about 0 degree to about 90 degrees with respect to the position of the second germicidal UV light source.
14. The UV device according to item 12, wherein (vii) is selected from the group consisting of a pneumatic cylinder, a motor, a winch, an upper frame fixture clip, an extension spring, a rope and a cable.
15. The UV device according to item 14, wherein (vii) is a rope or a cable and wherein the rope or cable is operatively connected to a first rope or cable anchoring point at the upper frame and operatively connected to a second rope or cable anchoring point located on either the lower frame or located on a mounting bracket movably attached to the lower frame and wherein, upon release of the rope or cable from the second rope or cable anchoring point, the upper frame moves from a horizontal position to an angular position with respect to the position of the lower frame.
16. The UV device according to item 15, wherein the second rope or cable anchoring point is a first rope post or a second rope post attached to the mounting bracket.
17. The UV device according to item 14, wherein (vii) is an upper frame fixture clip, wherein the upper frame fixture clip is configured to restrict movement of the upper frame, and wherein, upon release from the upper frame fixture clip, the upper frame moves from a horizontal position to an angular position with respect to the position of the lower frame.
18. The UV device according to item 14, wherein (vii) is an extension spring comprising a first hook attached to a first anchoring post and a second hook attached to a second anchoring post.
19. The UV device according to item 14, wherein (vii) is a motor.
20. The UV device according to item 14, wherein (vii) is a winch.
21. The UV device according to item 12, further comprising
   (viii) at least one stop post;
   wherein the at least one stop post is configured to prevent movement of the first germicidal UV light source beyond an approximately perpendicular position with respect to the position of the second germicidal UV light source.
22. The UV device according to item 18, wherein the second anchoring post is configured to function as a carrying handle.
23. The UV device according to item 8, wherein the first upper frame end and the second upper frame end each comprise at least one opening configured to receive a UV lamp socket and wherein the first germicidal UV light source is operatively attached to the UV lamp socket.
24. The UV device according to item 8, wherein the first lower frame end and the second lower frame end each comprise at least one opening configured to receive a UV lamp socket and wherein the second germicidal UV light source is operatively attached to the UV lamp socket.
25. The UV device according to item 8, wherein the first upper frame end and the second upper frame end are operatively connected by a plurality of rods.
26. The UV device according to item 25, wherein the upper frame further comprises at least one cross connector and wherein the plurality of rods penetrates the at least one cross connector.
27. The UV device according to item 1, further comprising:
   (vi) a UV sensor.
28. The UV device according to item 1, wherein the first germicidal UV light source is a member of a plurality of first germicidal UV light sources selected from the group consisting of two first germicidal UV light sources, three first germicidal UV light sources, four first germicidal UV light sources, five first germicidal UV light sources, six first germicidal UV light sources, seven first germicidal UV light sources, eight first germicidal UV light sources, nine first germicidal UV light sources, and ten first germicidal UV light sources, and wherein members of the plurality of first germicidal UV light sources are the same or different germicidal UV light sources.
29. The UV device according to item 8, wherein the second germicidal UV light source is a member of a plurality of second germicidal UV light sources selected from the group consisting of two second germicidal UV light sources, three second germicidal UV light sources, four second germicidal UV light sources, five second germicidal UV light sources, six second germicidal UV light sources, seven second germicidal UV light sources, eight second germicidal UV light sources, nine second germicidal UV light sources, and ten second germicidal UV light sources, and wherein members of the plurality of second germicidal UV light sources can be the same or different germicidal UV light sources.
30. The UV device according to item 8, wherein the UV device comprises two first germicidal UV light sources operatively connected to the upper frame and two second germicidal UV light sources operatively connected to the lower frame and wherein the two first germicidal UV light sources and the two second germicidal UV light sources are the same or different germicidal UV light sources.
31. The UV device according to item 1, wherein the first germicidal UV light source comprises a lamp selected from the group consisting of a low pressure mercury lamp, a medium pressure mercury lamp, a high pressure mercury lamp, an ultra-high pressure mercury lamp, a low pressure short arc xenon lamp, a medium pressure short arc xenon lamp, a high pressure short arc xenon lamp, an ultra-high pressure short arc xenon lamp, a low pressure long arc xenon lamp, a medium pressure long arc xenon lamp, a high pressure long arc xenon lamp, an ultra-high pressure long arc xenon lamp, a low pressure metal halide lamp, a medium pressure metal halide lamp, a high pressure metal halide lamp, an ultra-high pressure metal halide lamp, a tungsten halogen lamp, a quartz halogen lamp, a quartz iodine lamp, a sodium lamp, and an incandescent lamp.
32. The UV device according to item 1, wherein when the first germicidal UV light source is inserted into the container, room, space or defined environment, the control box resides outside of the container, room, space or defined environment.
33. The UV device according to item 1, wherein the touchscreen is configured to provide an input for a functionality selected from the group consisting of:
   (A) activating the UV device;
   (B) deactivating the UV device;
   (C) providing time input for completing a UV sterilization of a container, room, space or defined environment;
   (D) providing time elapsed for UV sterilization of the container, room, space or defined environment;
   (E) setting a desired UV intensity level;
   (F) adjusting a UV intensity level; and
   (G) logging in a code for a user.
34. The UV device according to item 1, wherein the algorithm determines the sterilization cycle based on at least two parameters selected from the group consisting of (i) species of microorganism present on the surface of the container, room, space or defined environment to be sterilized, (ii) size of the container, room, space or defined environment, (iii) shape of the container, room, space or defined environment,(iv) the material of the surface of the container, room, space or defined environment to be sterilized, (v) extinction depth of the first germicidal UV light source at 254 nm, (vi) distance of the first germicidal UV light source from the surface of the container, room, space or defined environment to be sterilized (vii) distribution and positioning of the first germicidal UV light source, (viii) configuration of the first germicidal UV light source, and (ix) intensity of the first germicidal UV light source.
35. The UV device according to item 1, wherein when the first germicidal UV light source is inserted into the container, room, space or defined environment, the first germicidal UV light source can be moved from a first position to a second position within the container, room, space or defined environment.
36. A system comprising:
   (i) the UV device according to item 1; and
   (ii) a container, a room, a space or a defined environment.
37. The system according to item 36 wherein the container is selected from the group consisting of:
   (A) a container for fermenting an alcoholic beverage;
   (B) a container for storing or transporting a dairy product, a liquid dairy, a liquid dairy composition or a dry dairy composition;
   (C) a container for water, milk, coffee, tea, juice, or a carbonated beverage; and
   (D) a container for a biological fluid.
38. The system according to item 36, wherein the room, space or defined environment is selected from the group consisting of a commercial kitchen, a medical facility, an acute care area, an operating room, a medical equipment storage cabinet, a clean room, a bathroom, a waiting room, a food production area, a food processing area, a nursery home, a car, a bus, a trailer, a rail car, an airplane, a ship, a boat, a grocery store display case, a deli counter, a fish display case, a poultry display case, a floral display case, a refrigerated display case, a non-refrigerated display case, and a conveyor belt.
39. The system according to item 36, wherein the container, room, space or defined environment comprises an interior surface comprising wood, plastic, concrete, a polymer, etched aluminum, foil aluminum, polished aluminum, chromium, glass, nickel, silver, stainless steel, tri-plated steel, water paint, white cotton, white oil paint, white paper, white porcelain, white wall plaster or a fabric.
40. A system comprising:
   (i) the UV device according to item 1; and
   (ii) a case, wherein, the UV device, when not in use, resides within the case.
41. The system according to item 40, wherein the case is attached to the control box.
42. The system according to item 40, further comprising:
   (iii) a transportation rack configured to accommodate the control box and the case for transportation.
43. A method for UV sterilization of an interior surface of a container, room, space or defined environment, the method comprising the steps of:
   (a) movably and inwardly inserting the first UV light source of the UV device of item 1 into a container, room, space or defined environment; and
   (b) activating the first germicidal UV light source;
   whereby the interior surface of the container, room, space or defined environment is sterilized.
44. The method according to item 43, wherein the container is selected from the group consisting of:
   (A) a container for fermenting an alcoholic beverage;
   (B) a container for storing or transporting a dairy product, a liquid dairy, a liquid dairy composition or a dry dairy composition;
   (C) a container for water, milk, coffee, tea, juice, or a carbonated beverage; and
   (D) a container for a biological fluid.
45. The method according to item 43, wherein the room, space or defined environment is selected from the group consisting of a commercial kitchen, a medical facility, an acute care area, an operating room, a medical equipment storage cabinet, a clean room, a bathroom, a waiting room, a food production area, a food processing area, a nursery home, a car, a bus, a trailer, a rail car, an airplane, a ship, a boat, a grocery store display case, a deli counter, a fish display case, a poultry display case, a floral display case, a refrigerated display case, a non-refrigerated display case, and a conveyor belt.
46. A method for manufacturing the UV device of item 1, the method comprising the steps of:
   (a) attaching the first germicidal UV light source to the frame;
   (b) operatively connecting the cable to the control box and to the first germicidal UV light source; and
   (c) attaching the means for operatively attaching the UV device to a container, room, space or defined environment.
47. A method for manufacturing the UV device of item 8, the method comprising the steps of:
   (a) attaching the first germicidal UV light source to the upper frame;
   (b) attaching the second germicidal UV light source to the lower frame; and
   (c) attaching the first hinge to the lower frame and to the upper frame thereby operatively connecting the lower frame to the upper frame so that the upper frame can be moved into a position ranging from about 0 degree to about 90 degrees with respect to the position of the lower frame.
48. The method according to item 47, further comprising the step of:
   (d) attaching a means for controlling or facilitating movement of the upper frame into a position ranging from about 0 degree to about 90 degrees with respect to the position of the lower frame.

## Claims

1. A portable ultraviolet (UV) device for applying a sterilization cycle to a surface within a container, a room, a space, or a defined environment, the UV device comprising:
(i) a first germicidal UV light source and a second germicidal UV light source;
(ii) a frame comprising:
(a) a lower frame comprising a first lower frame end and a second lower frame end;
(b) an upper frame comprising a first upper frame end and a second upper frame end;
(c) a first hinge movably and operatively connecting the lower frame to the upper frame and configured to move the upper frame into an angular position with respect to the position of the lower frame;
wherein the first germicidal UV light source is operatively attached to the upper frame; wherein the second germicidal UV light source is operatively attached to the lower frame; and
wherein, when not in use, the upper frame is positioned on top of the lower frame;
(iii) a control box for controlling a sterilization cycle;
(iv) a cable operatively connecting the control box and the first germicidal UV light source; and
(v) a means for operatively attaching the UV device to the container, room, space or defined environment,
wherein the UV device is configured to movably and inwardly insert the first germicidal UV light source through an opening of the container, room, space or defined environment into the container, room, space or defined environment when the UV device is operatively attached to the container, room, space or defined environment;
wherein the UV device uses an algorithm for determining the sterilization cycle;
wherein the control box comprises a circuit board and at least three components selected from the group consisting of:
(1) a power supply;
(2) electronics for activating/deactivating the first germicidal UV light source;
(3) a timer for controlling the duration of the sterilization cycle;
(4) a memory for storing a pre-determined sterilization cycle;
(5) a safety switch;
(6) an on/off/reset button;
(7) a status indicator light;
(8) an alarm light; and
(9) a user interface selected from the group consisting of a touchscreen and a keyboard; and
wherein the circuit board comprises at least three functionalities selected from the group consisting of:
(A) comprising a radiofrequency identifier reader;
(B) communicating with a radiofrequency identifier;
(C) controlling a movement of the first germicidal UV light source within the container, room, space, or defined environment;
(D) controlling a rate of descent or ascent of the first germicidal UV light source within the container, room, space, or defined environment;
(E) controlling a positioning of first germicidal UV light source within the container, room, space, or defined environment;
(F) controlling an on/off status of a motor, wherein the motor controls the positioning of the first germicidal UV light source within the container, room, space, or defined environment;
(G) controlling an on/off status of the first germicidal UV light source based on measuring whether a pre-determined UV intensity has been attained;
(H) controlling extension of the first germicidal UV light source from a housing;
(I) controlling retraction of the first germicidal UV light source into the housing;
(J) responding to a jammed position status of the first germicidal UV light source;
(K) controlling an optical sensor which initiates a timer upon placing the first germicidal UV source into the container, room space, or defined environment;
(L) controlling a speaker emitting an audible signal at the beginning or completion of the sterilization cycle;
(M) controlling a plurality of LED lights indicting a status of the sterilization cycle;
(N) relaying UV light intensity via a UV sensor to the container, room, space, or defined environment;
(O) uploading and relaying information from the radiofrequency identifier;
(P) generating a report on time of the sterilization cycle;
(Q) generating a report on duration of the sterilization cycle;
(R) generating a report on UV light intensity attained during the sterilization cycle;
(S) emailing, phoning or texting the report on time of the sterilization cycle;
(T) emailing, phoning or texting the report on duration of the sterilization cycle;
(U) emailing, phoning or texting the report on UV light intensity attained during the sterilization cycle;
(V) emailing, phoning or texting an alert to an individual that the sterilization cycle is initiated, interrupted or complete;
(W) emailing, phoning or texting an alert that a UV light source requires replacement;
(X) logging date, time or individual who used the UV device; and
(Y) logging information of the container, room, space, or defined environment in which the UV device will be or has been used.

2. The UV device according to claim 1, further comprising: (vii) a second hinge movably and operatively connecting the lower frame to (v).

3. The UV device according to claim 1, further comprising:
(vii) a means for controlling or facilitating movement of the upper frame to an angular position with respect to the position of the lower frame, wherein, preferably, (vii) permits the first germicidal UV light source be positioned at an angle ranging from about 0 degree to about 90 degrees with respect to the position of the second germicidal UV light source.

4. The UV device according to claim 3, wherein
(vii) is selected from the group consisting of a pneumatic cylinder, a motor, a winch, an upper frame fixture clip, an extension spring, a rope and a cable,
wherein, preferably,
1) (vii) is a rope or a cable and wherein the rope or cable is operatively connected to a first rope or cable anchoring point at the upper frame and operatively connected to a second rope or cable anchoring point located on either the lower frame or located on a mounting bracket movably attached to the lower frame and wherein, upon release of the rope or cable from the second rope or cable anchoring point, the upper frame moves from a horizontal position to an angular position with respect to the position of the lower frame, wherein, optionally, the second rope or cable anchoring point is a first rope post or a second rope post attached to the mounting bracket, or
2) (vii) is an upper frame fixture clip, wherein the upper frame fixture clip is configured to restrict movement of the upper frame, and wherein, upon release from the upper frame fixture clip, the upper frame moves from a horizontal position to an angular position with respect to the position of the lower frame, or
3) (vii) is an extension spring comprising a first hook attached to a first anchoring post and a second hook attached to a second anchoring post, wherein, preferably, the second anchoring post is configured to function as a carrying handle, or
4) (vii) is a motor, or
5) (vii) is a winch.

5. The UV device according to claim 3, further comprising (viii) at least one stop post;
wherein the at least one stop post is configured to prevent movement of the first germicidal UV light source beyond an approximately perpendicular position with respect to the position of the second germicidal UV light source.

6. The UV device according to claim 1, wherein
1) the first upper frame end and the second upper frame end each comprise at least one opening configured to receive a UV lamp socket and wherein the first germicidal UV light source is operatively attached to the UV lamp socket, or
2) the first lower frame end and the second lower frame end each comprise at least one opening configured to receive a UV lamp socket and wherein the second germicidal UV light source is operatively attached to the UV lamp socket, or
3) the first upper frame end and the second upper frame end are operatively connected by a plurality of rods, wherein, preferably, the upper frame further comprises at least one cross connector and wherein the plurality of rods penetrates the at least one cross connector.

7. The UV device according to claim 1, wherein
1) the second germicidal UV light source is a member of a plurality of second germicidal UV light sources selected from the group consisting of two second germicidal UV light sources, three second germicidal UV light sources, four second germicidal UV light sources, five second germicidal UV light sources, six second germicidal UV light sources, seven second germicidal UV light sources, eight second germicidal UV light sources, nine second germicidal UV light sources, and ten second germicidal UV light sources, and wherein members of the plurality of second germicidal UV light sources can be the same or different germicidal UV light sources, or
2) the UV device comprises two first germicidal UV light sources operatively connected to the upper frame and two second germicidal UV light sources operatively connected to the lower frame and wherein the two first germicidal UV light sources and the two second germicidal UV light sources are the same or different germicidal UV light sources.

8. The UV device according to claim 1, wherein
1) the UV device comprises a motorized unit to move the UV light source within the container, room, space or defined environment to a horizontal position, to a vertical position or a combination of both, wherein the motorized unit comprises the motor of feature (F), if this functionality is comprised by the circuit board, or
2) the UV device comprises an optical component, preferably selected from the group consisting of a reflector, a shutter, a lens a splitter, and a mirror.

9. The UV device according to claim 1, wherein
1) the UV device is a portable UV device, or
2) the first germicidal UV light source resides in a housing, wherein, preferably,
2.1) the housing permits UV light to pass through or
2.2) the housing is made of UV fused silica, CaF₂, MgF₂, BaF₂, quartz, sapphire, teflon, polydimethylsiloxane, TPX® or polymethylpentene (PMP), or
3) the UV device further comprises
(vi) a rope, a cable or a rigid rod for moving the first UV light source to a position within the container, room, space or defined environment, or
4) the frame comprises a plurality of openings, or
5) (v) is configured to operatively attach the portable UV device to an opening of the container, to a fixture in or at the container, room, space or defined environment and to permit the moving the first germicidal UV light source to a position within the container, room, space or defined environment, or
6) (v) is a mounting bracket, or
7) the UV device further comprises
(vi) a UV sensor, or
8) the first germicidal UV light source is a member of a plurality of first germicidal UV light sources selected from the group consisting of two first germicidal UV light sources, three first germicidal UV light sources, four first germicidal UV light sources, five first germicidal UV light sources, six first germicidal UV light sources, seven first germicidal UV light sources, eight first germicidal UV light sources, nine first germicidal UV light sources, and ten first germicidal UV light sources, and wherein members of the plurality of first germicidal UV light sources are the same or different germicidal UV light sources, or
9) the first germicidal UV light source comprises a lamp selected from the group consisting of a low pressure mercury lamp, a medium pressure mercury lamp, a high pressure mercury lamp, an ultra-high pressure mercury lamp, a low pressure short arc xenon lamp, a medium pressure short arc xenon lamp, a high pressure short arc xenon lamp, an ultra-high pressure short arc xenon lamp, a low pressure long arc xenon lamp, a medium pressure long arc xenon lamp, a high pressure long arc xenon lamp, an ultra-high pressure long arc xenon lamp, a low pressure metal halide lamp, a medium pressure metal halide lamp, a high pressure metal halide lamp, an ultra-high pressure metal halide lamp, a tungsten halogen lamp, a quartz halogen lamp, a quartz iodine lamp, a sodium lamp, and an incandescent lamp, or
10) when the first germicidal UV light source is inserted into the container, room, space or defined environment, the control box resides outside of the container, room, space or defined environment, or
11) the touchscreen is configured to provide an input for a functionality selected from the group consisting of:
(A) activating the UV device;
(B) deactivating the UV device;
(C) providing time input for completing a UV sterilization of a container, room, space or defined environment;
(D) providing time elapsed for UV sterilization of the container, room, space or defined environment;
(E) setting a desired UV intensity level;
(F) adjusting a UV intensity level; and
(G) logging in a code for a user, or
12) the algorithm determines the sterilization cycle based on at least two parameters selected from the group consisting of (i) species of microorganism present on the surface of the container, room, space or defined environment to be sterilized, (ii) size of the container, room, space or defined environment, (iii) shape of the container, room, space or defined environment,(iv) the material of the surface of the container, room, space or defined environment to be sterilized, (v) extinction depth of the first germicidal UV light source at 254 nm, (vi) distance of the first germicidal UV light source from the surface of the container, room, space or defined environment to be sterilized (vii) distribution and positioning of the first germicidal UV light source, (viii) configuration of the first germicidal UV light source, and (ix) intensity of the first germicidal UV light source, or 13) when the first germicidal UV light source is inserted into the container, room, space or defined environment, the first germicidal UV light source can be moved from a first position to a second position within the container, room, space or defined environment.

10. A system comprising:
(i) the UV device according to any one of claims 1 to 9; and
(ii) a container, a room, a space or a defined environment, preferably wherein
1) the container is selected from the group consisting of: a container for fermenting an alcoholic beverage; a container for storing or transporting a dairy product, a liquid dairy, a liquid dairy composition or a dry dairy composition; a container for water, milk, coffee, tea, juice, or a carbonated beverage; a container for a biological fluid; a container for blood; a container for a blood product; a container for a cell culture product; a container for a biotechnology product; a vat; a silo; a tub; a basket; a case; a box; a barrel; a storage bin; a beverage container; an aquarium; and a container comprising an interior surface on which a microorganism is present, or
2) the room, space or defined environment is selected from the group consisting of a commercial kitchen, a medical facility, an acute care area, an operating room, a medical equipment storage cabinet, a clean room, a bathroom, a waiting room, a food production area, a food processing area, a nursery home, a car, a bus, a trailer, a rail car, an airplane, a ship, a boat, a grocery store display case, a deli counter, a fish display case, a poultry display case, a floral display case, a refrigerated display case, a non-refrigerated display case, and a conveyor belt, or
3) the container, room, space or defined environment comprises an interior surface comprising wood, plastic, concrete, a polymer, etched aluminum, foil aluminum, polished aluminum, chromium, glass, nickel, silver, stainless steel, tri-plated steel, water paint, white cotton, white oil paint, white paper, white porcelain, white wall plaster or a fabric.

11. A system comprising:
(i) the UV device according to any one of claims 1 to 9; and
(ii) a case, wherein, the UV device, when not in use, resides within the case, preferably wherein
1) the case is attached to the control box, or
2) the system further comprises
(iii) a transportation rack configured to accommodate the control box and the case for transportation.

12. A method for UV sterilization of an interior surface of a container, room, space or defined environment, the method comprising the steps of:
(a) movably and inwardly inserting the first UV light source of the UV device of any one of claims 1 to 9 into a container, room, space or defined environment; and
(b) activating the first germicidal UV light source;
whereby the interior surface of the container, room, space or defined environment is sterilized.

13. The method according to claim 12, wherein
1) the container is selected from the group consisting of: a container for fermenting an alcoholic beverage; a container for storing or transporting a dairy product, a liquid dairy, a liquid dairy composition or a dry dairy composition; a container for water, milk, coffee, tea, juice, or a carbonated beverage; a container for a biological fluid; a container for blood; a container for a blood product; a container for a cell culture product; a container for a biotechnology product; a vat; a silo; a tub; a basket; a case; a box; a barrel; a storage bin; a beverage container; an aquarium; and a container comprising an interior surface on which a microorganism is present, or
2) the room, space or defined environment is selected from the group consisting of a commercial kitchen, a medical facility, an acute care area, an operating room, a medical equipment storage cabinet, a clean room, a bathroom, a waiting room, a food production area, a food processing area, a nursery home, a car, a bus, a trailer, a rail car, an airplane, a ship, a boat, a grocery store display case, a deli counter, a fish display case, a poultry display case, a floral display case, a refrigerated display case, a non-refrigerated display case, and a conveyor belt, or
3) the container, room, space or defined environment comprises an interior surface comprising wood, plastic, concrete, a polymer, etched aluminum, foil aluminum, polished aluminum, chromium, glass, nickel, silver, stainless steel, tri-plated steel, water paint, white cotton, white oil paint, white paper, white porcelain, white wall plaster or a fabric.

14. A method for manufacturing the UV device of any one of claims 1 to 9, the method comprising the steps of:
(a) attaching the first germicidal UV light source to the frame;
(b) operatively connecting the cable to the control box and to the first germicidal UV light source; and
(c) attaching the means for operatively attaching the UV device to a container, room, space or defined environment.

15. A method for manufacturing the UV device of any one of claims 1 to 9, the method comprising the steps of:
(a) attaching the first germicidal UV light source to the upper frame;
(b) attaching the second germicidal UV light source to the lower frame; and
(c) attaching the first hinge to the lower frame and to the upper frame thereby operatively connecting the lower frame to the upper frame so that the upper frame can be moved into a position ranging from about 0 degree to about 90 degrees with respect to the position of the lower frame, wherein the method preferably further comprises the step of:
(d) attaching a means for controlling or facilitating movement of the upper frame into a position ranging from about 0 degree to about 90 degrees with respect to the position of the lower frame.
